# EUROPEAN PATENT APPLICATION

(11) **EP 4 484 429 A1**
(43) Date of publication of application: **01.01.2025**
(21) Application number: 23755905.9
(22) Date of filing: 20.02.2023
(51) Int. Cl.: C07D 487/04, A61K 47/36, A61K 47/61, A61P 29/00, A61P 35/00, A61P 1/04

(54) **COMPOUND AND USE THEREOF**

(30) Priority: 21.02.2022 CN 202210157227
(71) Applicant: OnQuality Pharmaceuticals China Ltd., Shanghai 201112 (CN)
(72) Inventor: LIU, Shilan, Shanghai 201112 (CN); ZENG, Wenqin, Shanghai 201112 (CN); LI, Wenxi, Shanghai 201112 (CN); ZHANG, Shiyi, Shanghai 201112 (CN)
(74) Representative: Weickmann & Weickmann PartmbB
(86) International application number: PCT/CN2023/077089
(87) International publication number: WO 2023/155905

(57) **Abstract**

The present application relates to a compound of formula (I) and use thereof, and also relates to a method for preparing the compound.

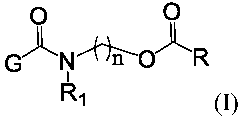

## Description

### TECHNICAL FIELD

The present application relates to the field of medicine, specifically relating to a prodrug of a Janus kinase (JAK) inhibitor and the medical use thereof.

### BACKGROUND

Janus kinase (JAK) is an intracellular non-receptor tyrosine kinase that mediates signaling and activation of various cytokines. The JAK kinase family is divided into four subtypes: JAK1, JAK2, JAK3 and TYK2. Each subtype mediates a different type of cytokine signaling pathway respectively. JAK-1, JAK-2, and TYK-2 are expressed in cells of various tissues throughout the human body, while JAK-3 is primarily expressed in hematopoietic tissue cells. The common feature of cytokine receptors is that the receptors themselves do not show kinase activity, but the intracellular segments of the receptors have binding sites for JAK of tyrosine kinase. When the cytokine receptor binds to its ligand, receptor-coupled JAK is activated, resulting in phosphorylation of the receptor. The phosphorylated tyrosine site can bind to STAT protein containing SH2 domain, so that STAT is recruited into the receptor and phosphorylated by JAK. Subsequently, the phosphorylated tyrosine mediates STAT dimerization. The activated STAT dimer then translocates to the cell nucleus and activates the transcription of its target genes, thereby regulating the growth, activation and differentiation of various cells.

JAK/STAT signaling pathway mediates the signaling of most intracellular cytokines and plays a key role in biological processes such as immune regulation and immune cell proliferation. The JAK/STAT signaling pathway is involved in many important biological processes such as cell proliferation, differentiation, apoptosis and immune regulation. It is closely related to various inflammatory diseases such as rheumatoid arthritis, dermatitis, psoriasis and inflammatory bowel disease (ulcerative colitis and Crohn's disease). Additionally, JAK/STAT signaling pathway is closely related to tumor diseases such as myelofibrosis, polycythemia vera and essential thrombocytosis. Mutations of JAK molecule itself can also lead to acute myelocytic leukemia (AML), acute lymphoblastic leukemia (ALL), ductal breast cancer, non-small cell lung cancer (NSCLC) and other tumor diseases.

The currently marketed and investigational JAK inhibitors primarily function by competing with the kinase domain for binding with ADP. As a result, JAK inhibitors generally suffer from low selectivity defect. Even inhibitors that are selective for a particular JAK subtype still face significant target-related side effects.

Given the favorable efficacy of JAK inhibitors and the serious side effects associated with multiple targets, it is urgent to develop a safer JAK inhibitor drug. In addition, developing a prodrug to enhance local exposure of the drug based on different clinical needs has become an effective strategy for improving safety.

### SUMMARY OF THE INVENTION

In one aspect, the present application provides a compound of formula (I), a pharmaceutically acceptable salt, bioactive metabolite, solvate, hydrate, prodrug, racemate, enantiomer or stereoisomer thereof, 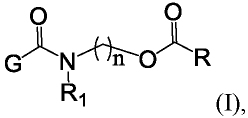 wherein:
G is a JAK inhibitor, R₁ and R are independently selected from: hydrogen, deuterium, tritium, optionally substituted alkyl, optionally substituted deuterated alkyl, optionally substituted halogenated alkyl, optionally substituted alkoxy, optionally substituted halogenated alkoxy, halogen and cyclic optionally substituted group; and n is 1 - 5. In some embodiments, the n is 2 - 4. In some embodiments, the n is 1. In some embodiments, the n is 2. In some embodiments, the n is 3. In some embodiments, the n is 4. In some embodiments, the n is 5.

In some embodiments, the R₁ and R are independently selected from: -(CH₂)ₙ₁Ra, -(CH₂)ₙ₁ORa, - (CH₂)ₙ₁SRa, -(CH₂)ₙ₁C(O)Ra, -(CH₂)ₙ₁C(O)ORa, -(CH₂)ₙ₁S(O)ₘ₁Ra, -(CH₂)ₙ₁NRaRb, - (CH₂)ₙ₁C(O)NRaRb, -(CH₂)ₙ₁NRaC(O)Rb and -(CH₂)ₙ₁NRaS(O)ₘ₁Rb; Wherein:
Ra and Rb can be the same or different, and each is independently selected from the group: hydrogen, deuterium, tritium, alkyl, deuterated alkyl, halogenated alkyl, alkoxy, hydroxyalkyl, halogenated alkoxy, halogen, cyano, nitro, hydroxy, amino, alkenyl, alkynyl, cycloalkyl, heterocyclyl, aryl, and heteroaryl; wherein alkyl, deuterated alkyl, halogenated alkyl, alkoxy, hydroxyalkyl, halogenated alkoxy, alkenyl, alkynyl, cycloalkyl, heterocyclyl, aryl, and heteroaryl are optionally further substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;

Alternatively, the Ra is connected with Rb to form a cycloalkyl, heterocyclyl, aryl or heteroaryl group, wherein cycloalkyl, heterocyclyl, aryl and heteroaryl groups are optionally substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
n1 is 0 - 5, for example, n1 may be 0, 1, 2, 3, 4 or 5; and
m1 is 0, 1 or 2.

In some embodiments, the R₁ is a high molecular compound (for example, a high molecular compound with multiple repeating units, or a high molecular compound obtained by crosslinking). In some embodiments, the R is a high molecular compound (for example, a high molecular compound with multiple repeating units, or a high molecular compound obtained by crosslinking). In some embodiments, the cyclic group is selected from: cycloalkyl, heterocyclyl, oxoheterocyclyl, thioheterocyclyl, aryl and heteroaryl. In some embodiments, wherein cycloalkyl, heterocyclyl, oxoheterocyclyl, thioheterocyclyl, aryl and heteroaryl are optionally substituted with one or more groups selected from the following group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl.

In some embodiments, the R₁ is hydrogen, optionally substituted alkyl or optionally substituted deuterated alkyl.

In some embodiments, the R₁ is hydrogen, optionally substituted C₁-C₆ alkyl or optionally substituted C₁-C₆ deuterated alkyl.

In some embodiments, the R₁ is hydrogen, optionally substituted methyl or optionally substituted deuterated methyl.

In some embodiments, the R is selected from: optionally substituted alkyl, optionally substituted deuterated alkyl, optionally substituted cycloalkyl, optionally substituted heterocycloalkyl, optionally substituted aryl and optionally substituted heteroaryl.

In some embodiments, the R is selected from: optionally substituted C₁-C₂₀ alkyl, optionally substituted C₁-C₂₀ deuterated alkyl, optionally substituted C₃-C₂₀ cycloalkyl, optionally substituted C₁-C₂₀ heterocycloalkyl, optionally substituted C₆-C₂₀ aryl and optionally substituted C₁-C₂₀ heteroaryl.

In some embodiments, the n is 2.

In some embodiments, the R₁ is hydrogen, optionally substituted methyl or optionally substituted deuterated methyl, the n is 2, and the R is optionally substituted C₃-C₂₀ cycloalkyl or optionally substituted C₁-C₂₀ heterocycloalkyl.

In some embodiments, the G is a JAK inhibitor selected from the group: Ruxolitinib, Tofacitinib, Oclacitinib, Fedratinib, Peficitinib, Upadacitinib, Barictinib, Fligotinib, Decernotinib, Cerdulatinib, Lestaurtinib, Pacritinib, Momelotinib, Gandotinib, Abrocitinib, Solcitinib, SHR-0203, Itacitinib, PF-06651600, BMS-986165, Abrocitinib, Cucurbitacin I, CHZ868, TD-1473, Zotiraciclib, Alkotinib, Jaktinib, AZD-4205, DTRMHS-07, KL130008, WXSH-0150, TQ05105, WXFL10203614, GLPG0634, CEP-33779, R-348, Itacitinib, Ritlecitinib, Brepocitinib, Tasocitinib, Deucravacitinib, INCB-039110, Izencitinib, Entrectinib, Ivarmacitinib, Deuruxolitinib, Adelatinib, NDI-034858, Nezulcitinib, ATI-01777, TD-8236, INCB-054707, Ropsacitinib, AGA-201, ATI50001, Gusacitinib, Cerdulatinib, Roniciclib, AT-9283, FMX-114, OST-122, TT-00420, Repotrectinib, INCB-052793, CT-340, BMS-911543, Ilginatinib, BGB-23339, ICP-332, ESK-001, SYHX-1901, VTX-958, TLL-018, CEE-321, CJ-15314, TD-5202, ABBV-712, GLPG-3667, CPL-116, AZD-4604, TAS-8274, MAX-40279, TD-3504, KN-002, AZD-0449, R-548, AC-410, Spebrutinib, ONX-0805, AEG-41174, XL-019, CR-4, WP-1066, GDC-0214, INCB-047986, PF-06263276, R-333, AZD-1480, Tozasertib, CS-12192 and AC-1101. In some embodiments, the G is a JAK inhibitor selected from the group: Tofacitinib, Ruxolitinib, Baricitinib, Peficitinib, Pacritinib, Delgocitinib, Pf-04965842, Upadacitinib, Filgotinib, Itacitinib, Fedratinib, Decernotinib, SHR-0302, ASN-002, Cerdulatinib, BMS-986165, PF-06700841, INCB-52793, ATI-502, PF-06651600, AZD-4205, Deuterium Modified Ruxolitinib Analog, ATI-501, R-348, NS-018, Jaktinib Hydrochloride and KL-130008.

In some embodiments, the compound is selected from the group:

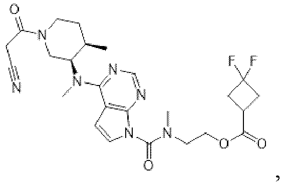

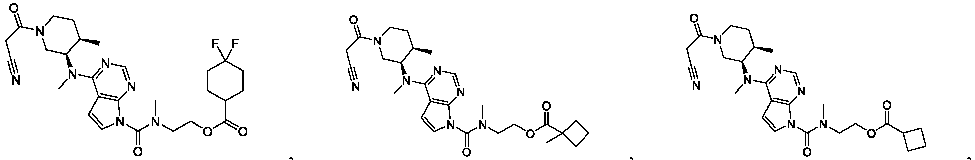

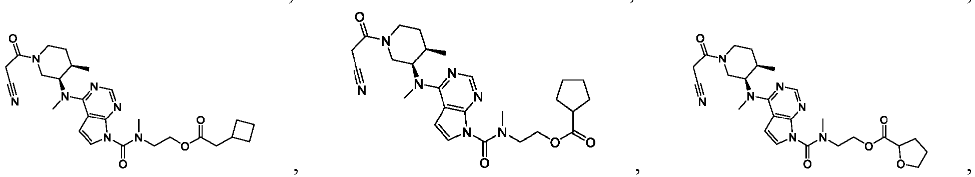

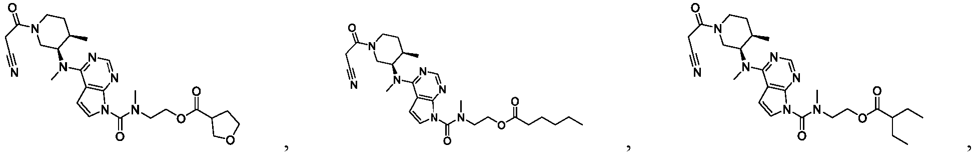

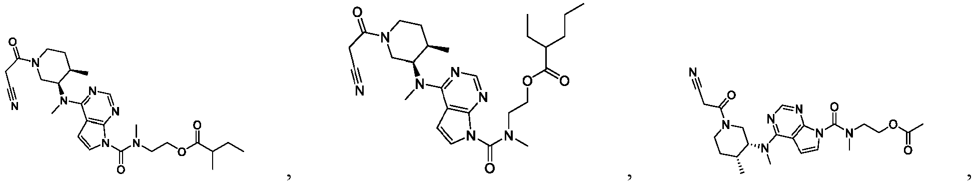

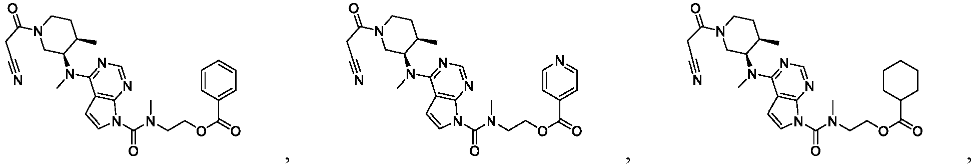

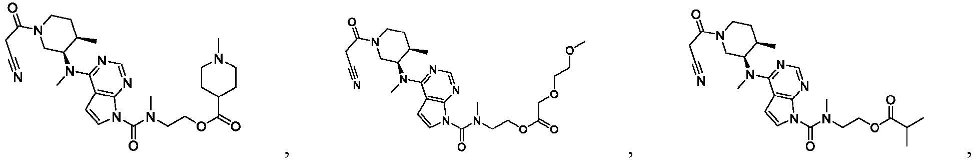

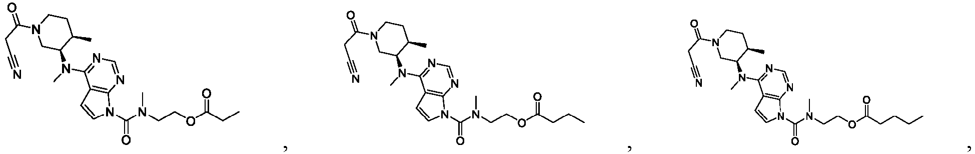

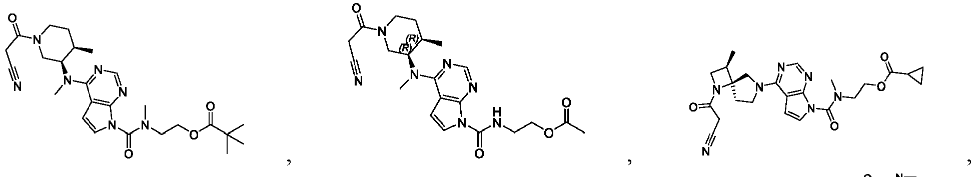

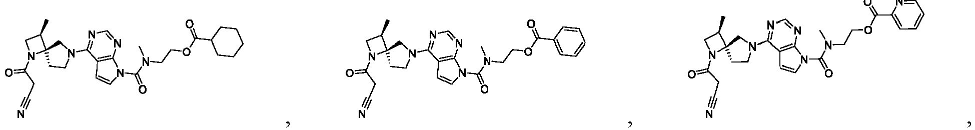

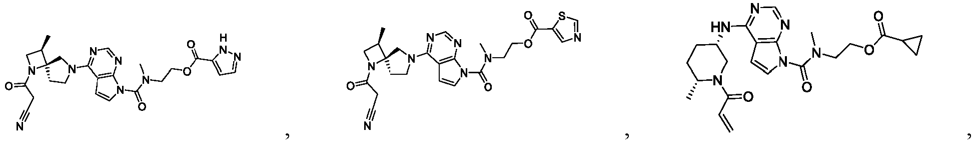

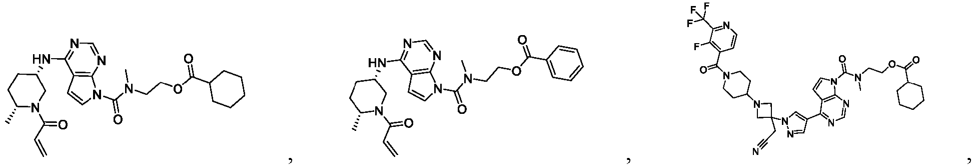

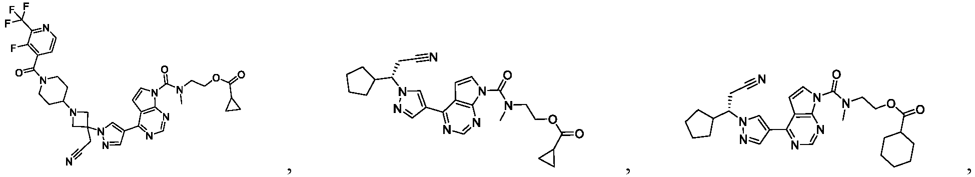

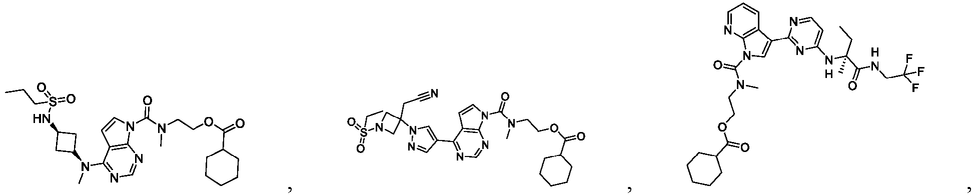

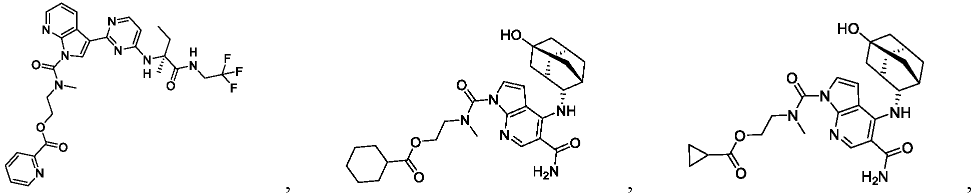

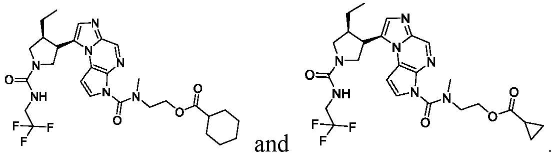

In another aspect, the present application provides methods for preparing the compound described herein, the pharmaceutically acceptable salt, bioactive metabolite, solvate, hydrate, prodrug, racemate, enantiomer or stereoisomer thereof.

In another aspect, the present application provides a composition comprising the compound described herein, the pharmaceutically acceptable salt, bioactive metabolite, solvate, hydrate, prodrug, racemate, enantiomer or stereoisomer thereof.

In some embodiments, the composition is a pharmaceutical composition comprising a pharmaceutically acceptable carrier.

In some embodiments, the concentration of the compound in the composition, the pharmaceutically acceptable salt, bioactive metabolite, solvate, hydrate, prodrug, racemate, enantiomer or stereoisomer thereof is from about 0.001% w/w to about 40% w/w.

In some embodiments, the composition is a formulation suitable for topical administration. In some embodiments, the composition is an ointment.

In another aspect, the present application provides the compound, the pharmaceutically acceptable salt, bioactive metabolite, solvate, hydrate, prodrug, racemate, enantiomer or stereoisomer thereof; or the composition of the present application may be used for preventing, alleviating and/or treating a disease or disorder associated with the administration of an antitumor agent in a subject. In another aspect, the present application provides the compound, the pharmaceutically acceptable salt, bioactive metabolite, solvate, hydrate, prodrug, racemate, enantiomer or stereoisomer thereof; or the use of the composition of the invention for preparing a medicament for preventing, alleviating and/or treating a disease or disorder associated with the administration of an antitumor agent in a subject.

In another aspect, the compound described in the present application, the pharmaceutically acceptable salt, bioactive metabolite, solvate, hydrate, prodrug, racemate, enantiomer or stereoisomer thereof; or the composition of the invention can be used to prepare JAK inhibitor. In another aspect, the present application provides a method for preventing, alleviating and/or treating a disease or disorder associated with the administration of an antitumor agent in a subject, with the method comprising administering the compound described herein, the pharmaceutically acceptable salt, bioactive metabolite, solvate, hydrate, prodrug, racemate, enantiomer or stereoisomer thereof, or the composition described herein to a subject in need.

In another aspect, the present application provides a method for preventing, treating and/or alleviating a JAK-mediated disease or disorder in a subject in need thereof, the method comprising: administering to the subject an effective amount of the compound described in the present application, the pharmaceutically acceptable salt, bioactive metabolite, solvate, hydrate, prodrug, racemate, enantiomer or stereoisomer thereof; or a composition described in the present application. The effective dose may be a dose capable of effectively preventing, treating and/or alleviating the JAK-mediated disease or disorder.

In some embodiments, the method further comprises administering an additional therapeutic agent. The additional therapeutic agent may be an agent effective in preventing, treating and/or alleviating a JAK-mediated disease or disorder. In some embodiments, the additional therapeutic agent is selected from: Ruxolitinib, Tofacitinib, Oclacitinib, Fedratinib, Peficitinib, Upadacitinib, Barictinib, Fligotinib, Decernotinib, Cerdulatinib, Lestaurtinib, Pacritinib, Momelotinib, Gandotinib, Abrocitinib, Solcitinib, SHR-0203, Itacitinib, PF-06651600, BMS-986165, Abrocitinib, Cucurbitacin I, CHZ868, TD-1473, Zotiraciclib, Alkotinib, Jaktinib, AZD-4205, DTRMHS-07, KL130008, WXSH-0150, TQ05105, WXFL10203614, GLPG0634, CEP-33779, R-348, Itacitinib, Ritlecitinib, Brepocitinib, Tasocitinib, Deucravacitinib, INCB-039110, Izencitinib, Entrectinib, Ivarmacitinib, Deuruxolitinib, Adelatinib, NDI-034858, Nezulcitinib, ATI-01777, TD-8236, INCB-054707, Ropsacitinib, AGA-201, ATI50001, Gusacitinib, Cerdulatinib, Roniciclib, AT-9283, FMX-114, OST-122, TT-00420, Repotrectinib, INCB-052793, CT-340, BMS-911543, Ilginatinib, BGB-23339, ICP-332, ESK-001, SYHX-1901, VTX-958, TLL-018, CEE-321, CJ-15314, TD-5202, ABBV-712, GLPG-3667, CPL-116, AZD-4604, TAS-8274, MAX-40279, TD-3504, KN-002, AZD-0449, R-548, AC-410, Spebrutinib, ONX-0805, AEG-41174, XL-019, CR-4, WP-1066, GDC-0214, INCB-047986, PF-06263276, R-333, AZD-1480, Tozasertib, CS-12192 and AC-1101.

In another aspect, the present application provides a compound described herein, a pharmaceutically acceptable salt, bioactive metabolite, solvate, hydrate, prodrug, racemate, enantiomer or stereoisomer thereof, or use of the composition described in the present application for preparing a medicament for preventing, treating and/or alleviating a JAK-mediated disease or disorder in a subject in need.

In some embodiments, the JAK-mediated disease or disorders is selected from: autoimmune disorders or autoimmune responses, extensive activation of immune response, bacterial infections, viral infections, inflammation, chronic and/or acute inflammatory disorders or conditions, and/or autoinflammatory disorders, fibrotic disorders, metabolic disorders, neoplasm, or cardiovascular or cerebrovascular disorders, skin disorders, pruritus, hair loss disorders, cancer or malignancy, autoimmune connective tissue diseases and autoimmune conditions; Still's disease, adult-onset Still's disease, Th17-associated inflammation, polychondritis (for example, relapsed polychondritis); myositis, polymyositis, autoimmune myositis, dermatomyositis, juvenile dermatomyositis; myasthenia gravis; arthritis (for example, rheumatoid arthritis, juvenile rheumatoid arthritis, systemic-onset juvenile rheumatoid arthritis, osteoarthritis, infectious arthritis, inflammatory arthritis, inflammatory bowel disease-associated arthritis, idiopathic arthritis, juvenile idiopathic arthritis, systemic juvenile idiopathic arthritis, psoriatic arthritis), spondylitis/spondyloarthritis/spondyloarthropathy (ankylosing spondylitis), gout, scleroderma (systemic scleroderma, juvenile scleroderma), Reiter's syndrome/reactive arthritis, Lyme disease, lupus/systemic lupus erythematosus (SLE) (lupus erythematosus, juvenile systemic lupus erythematosus, cutaneous lupus (subacute cutaneous lupus, chronic cutaneous lupus/discoid lupus, chilblain lupus erythematosus)), polymyalgia rheumatica, enthesitis, mixed connective tissue disease, enthesopathy, carditis, myocarditis, angiogenesis disorder, myelodysplastic syndrome, atherosclerosis, atherosclerotic restenosis (atherosclerotic coronary restenosis), acute coronary syndrome, myocardial infarction, cardiac allograft vasculopathy, transplanted arterial disease; vasculitis (large vessel vasculitis, small vessel vasculitis, giant cell arteritis, polyarteritis nodosa, and vasculitic syndromes, which comprise aortitis, Wegener's granulomatosis and behcet disease), Stimulator of Interferon Genes (STING)-associated vasculopathy with onset in infancy (SAVI); gastrointestinal disorders, enterocolitis, colitis, inflammatory bowel disease (ulcerative colitis and Crohn's disease), irritable bowel syndrome, enteritis syndrome/spastic colon, celiac sprue; acute and chronic pancreatitis; primary biliary cirrhosis, primary sclerotic cholangitis, jaundice, cirrhosis (for example, primary biliary cirrhosis, or cirrhosis attributable to fatty liver diseases (for example, alcoholic fatty liver disease and non-alcoholic fatty liver disease); esophagitis, gastritis, gastric and duodenal ulcers, peritonitis; nephropathy, immune-mediated glomerulonephrosis, autoimmune nephropathy, membranous glomerulopathy, chronic progressive nephropathy, diabetic kidney disease/diabetic nephropathy, renal fibrosis, renal ischemia/reperfusion injury, HIV-associated nephropathy, ureteral obstructive nephropathy, glomerulosclerosis, proteinuria, nephrotic syndrome, polycystic kidney disease, autosomal dominant polycystic kidney disease, immune-mediated nephropathy, autoimmune nephropathy, chronic progressive nephropathy, diabetic nephropathy, renal fibrosis, ischemic/reperfusion injury-associated nephropathy, HIV-associated nephropathy, ureteral obstructive nephropathy, glomerulonephritis, chronic kidney disease (for example, diabetic nephropathy), hypertension-induced nephropathy, glomerulosclerosis, proteinuria, nephrotic syndrome, polycystic kidney disease, autosomal dominant polycystic kidney disease, diabetic kidney disease, lupus nephritis; interstitial cystitis; periodontitis, gingivitis; pulmonary inflammation, sinusitis, pneumonia, bronchitis, asthma, bronchial asthma, atopic asthma, non-atopic asthma, allergic bronchopulmonary mycosis, aspirin-induced asthma, adult-onset asthma, fixed airflow obstruction asthma, exercise-induced asthma, cough variant asthma, work related asthma, nocturnal asthma, asthma complicated with obesity, eosinophilic asthma, hormone resistant asthma/severe asthma, exogenous asthma, endogenous asthma/cryptogenic asthma, Churg-Strauss syndrome, bronchiolitis, obliterative bronchiolitis, chronic obstructive pulmonary disease (COPD), interstitial pulmonary disease (pulmonary fibrosis, idiopathic pulmonary fibrosis), acute lung injury, pulmonary fibrosis (for example, idiopathic pulmonary fibrosis or cystic fibrosis), chronic obstructive pulmonary disease, adult respiratory distress syndrome, acute lung injury, drug-induced lung injury; Meniere's disease; eye disorders (including ocular inflammation, uveitis, dry eye/keratoconjunctivitis sicca, scleritis, episcleritis, keratitis/keratopathy, choroiditis, retinal vasculitis, optic neuritis, retinopathy (diabetic retinopathy, immune-mediated retinopathy, macular degeneration, wet macular degeneration and dry (senile) macular degeneration)); mastocytosis, iron-deficiency anemia, uremia, hypereosinophilic syndrome (HES), systemic mastocyte disease (SMCD), myelodysplastic syndrome, idiopathic thrombocytopenic purpura; bone resorption diseases; neurodegenerative disorders, neurological/neuromuscular disorders (for example, multiple sclerosis, Parkinson's disease, Huntington's disease, amyotrophic lateral sclerosis (ALS) (including familial ALS and sporadic ALS), Alzheimer's disease, myasthenia gravis, Lambert-Eaton myasthenic syndrome (LEMS), Guillain-Barre syndrome, meningitis, encephalitis, traumatic brain injury; nervous system damage, delusional parasitosis, dysregulation of neuronal processes and sensory perception, stroke/neuronal ischemia, spinal cord injury, peripheral neuropathy, pseudaphia, spinal cord injury, mental disease; paresthesia pain (acute pain, chronic pain, neuropathic pain, or fibromyalgia), nerve stimulation, peripheral neuropathy; pruritus/scrapie (atopic pruritus, dry pruritus, psoriasis-associated pruritus/psoriatic scrapie/psoriasis-associated scrapie), acute pruritus, chronic pruritus, idiopathic pruritus, chronic idiopathic pruritus, biliary scrapie, hepatobiliary-associated scrapie, renal-associated scrapie/renal scrapie, uremic scrapie, cholestasis, intrahepatic cholestasis of pregnancy, lichen simplex chronicus-associated pruritus, lymphoma-associated scrapie, leukemia-associated scrapie, prurigo nodularis, atopic dermatitis-associated scrapie, atopic scrapie/atopic pruritus, bullous scrapie, brachioradialis pruritus, neurogenic scrapie, neurotic scrapie, notalgia paresthetica, HIV-associated pruritic papules, psychogenic scrapie, swimmer scrapie, itch or uremic scrapie, urticaria scrapie; skin diseases (for example, dermatological drug reaction/drug eruption, dermatoxerasia/dry skin, rash, skin sensitization, skin irritation, sunburn, infection caused by shaving, body lice, head lice/phthiriasis, crab louse, cutaneous larva migrans, scabies, parasitic infections, insect infections, urticaria, papular urticaria, insect bites, insect stings, dandruff, foreign bodies or devices on the skin, fungal infections, herpes, varicella, eosinophilic folliculitis, dermatosis of pregnancy/pruritic urticaria papula and plaque (PUPP), inflammatory dermatosis, neutrophilic dermatosis, histiocytoid neutrophilic dermatosis, intestinal bypass syndrome dermatosis, psoriasis/psoriasis vulgaris, lichen planus, lichen sclerosus et atrophicus, acne (acne vulgaris, comedonal acne, inflammatory acne, nodular cystic acne, keloidalis acne, acne keloidalis nuchae), atopy (allergic contact sensitization, allergic dermatitis), dermatitis (atopic dermatitis/eczema, contact dermatitis, photodermatitis, seborrheic dermatitis, stasis dermatitis), acute febrile neutrophil dermatosis (Sweet syndrome), chronic atypical neutrophilic dermatosis with lipodystrophy and elevated temperature (CANDLE syndrome), hidradenitis suppurativa, urticaria, pyoderma gangraenosum, alopecia (eyebrow loss, hair loss intranasal, alopecia cicatrisata (for example, alopecia scarring, alopecia scarring centralis, lichen planopilaris, frontal fibrosing alopecia, folliculitis decalvans), non-cicatricial alopecia (alopecia areata (AA) (patchy AA, alopecia totalis (AT), alopecia universalis (AU), alopecia serpentina, alopecia horseshoe)), androgenic/androgenic alopecia (AGA)/AGA in males and females ), telogen effluvium, tinea capitis, hypotrichosis (simple hereditary hypotrichosis), lichen planopilaris (frontal fibrosing alopecia), punctate palmoplantar keratodermasis, erythema elevatum diutinum (EED), neutrophilic eccrine hidradenitis, palisaded neutrophilic granulomatous dermatitis, neutrophilic urticaria dermatosis, vitiligo (for example, segmental vitiligo (including single segmental vitiligo, double segmental vitiligo and multi segmental vitiligo), non-segmental vitiligo (for example, acrotype vitiligo, facial vitiligo, acrofacial vitiligo, central facial vitiligo, mucosal vitiligo, confetti macules, trichrome vitiligo, borderline inflammatory vitiligo, quadrichrome vitiligo, blue vitiligo, Koebner phenomenon, vitiligo vulgaris, generalized vitiligo, common vitiligo), mixed vitiligo/ non-segmental vitiligo accompanied by segmental vitiligo, localized vitiligo, solitary mucous vitiligo and vitiligo with or without gray hair (body hair involved); bullous diseases, immune bullous disease (for example, bullous pemphigus, scarring pemphigus, pemphigus vulgaris, linear IgA disease), pemphigoid gestationis, xeroderma pigmentosum; fibrosis and scarring disorders; fibroma, liver fibrosis, pulmonary fibrosis, idiopathic pulmonary fibrosis (for example, scleroderma, increased fibrosis, keloid, low-grade scarring of postoperative scars); wound healing, surgical scarring and radiation-induced fibrosis (for example, head and neck, gastrointestinal tract, or lungs), central nervous system scarring, digestive or gastrointestinal tract fibrosis, renal fibrosis, liver or bile duct fibrosis, liver fibrosis (for example, non-alcoholic steatohepatitis, hepatitis C, or liver cell carcinoma), cardiac fibrosis (for example, endomyocardial fibrosis or atrial fibrosis), ocular scarring, fibrosclerosis, scar growth, wound or scab healing, keloid, mediastinal fibrosis, myelofibrosis, retroperitoneal fibrosis/Ormond disease, progressive mass fibrosis, renal systemic fibrosis; Sjogren syndrome, sarcoidosis, familial Mediterranean fever, cryopyrin-associated periodic syndrome (Muckle-Wells syndrome, familial cold autoinflammatory syndrome/familial cold urticaria /TNF receptor-associated periodic syndrome, neonatal-onset multisystem inflammatory disease), oxygen-induced inflammation, reperfusion injury, postoperative trauma, tissue injury, hyperthermia syndrome; diabetes (Type I and II diabetes)/diabetes, Hashimoto thyroiditis, Graves' disease, Addison's disease, Castleman's disease, hyperparathyroidism, menopause, obesity, steroid resistance, glucose intolerance, metabolic syndrome, thyroid disease, pituitaritis; systemic immunosenescence; autoimmune atrophic gastritis, autoimmune atrophic gastritis with pernicious anemia, autoimmune encephalomyelitis, autoimmune orchitis, Goodpasture syndrome, Sjogren syndrome, autoimmune thrombocytopenia, sympathetic ophthalmia; secondary hematological manifestations of autoimmune diseases (for example, anemia), autoimmune hemolytic syndrome (autoimmune hemolytic anemia), autoimmune hepatitis and inflammatory hepatitis, autoimmune ovarian failure, autoimmune thrombocytopenia, silicone implantation-associated autoimmune diseases, drug-induced autoimmune, HIV-associated autoimmune syndrome, metal-induced autoimmune, autoimmune deafness, and autoimmune thyroid disorders; anaphylaxis and allergy (including hypersensitivity, for example, Type I hypersensitivity (for example, allergies), Type II hypersensitivity (for example, Goodpasture syndrome and autoimmune hemolytic anemia), Type III hypersensitivity (for example, Arthus reaction, serum disease) and Type IV hypersensitivity (for example, contact dermatitis and allograft rejection); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); rejection (for example, graft-versus-host reaction/graft-versus-host disease, allograft rejection (for example, acute/chronic allograft rejection), early allograft rejection; malignancies, cancers, lymphoma, leukemia, multiple myeloma, solid tumors, teratoma, metastatic disorders and bone disorders, internal cancer, bone cancer, oral/pharyngeal cancer, esophageal cancer, larynx cancer, gastric cancer, intestinal cancer, colon cancer, rectum cancer, lung cancer (for example, non-small cell lung cancer or small cell lung cancer), hepatic cancer (liver cancer), pancreatic cancer, nerve cancer, brain cancer (for example, glioma, spongioblastoma multiforme, astrocytoma, neuroblastoma, and neurinoma), head and neck cancer, throat cancer, ovarian cancer, uterine cancer, prostate cancer, testicular cancer, bladder cancer, renal cancer (kidney cancer), breast cancer, gallbladder cancer, cervical cancer, thyroid cancer, prostate cancer, eye cancer (eye malignancies), and skin cancer (melanoma and keratoacanthoma); and fibrotic cancers, fibromas, fibroadenomas, fibrosarcomas, myeloproliferative disorders, neoplasms (hematopoietic tissue neoplasms, spinal cord neoplasms, lymphoid tissue neoplasms (myelofibrosis, primary myelofibrosis, polycythemia vera, essential thrombocytosis)), leukemias (acute lymphoblastic leukemia, acute myelocytic leukemia, and chronic myelocytic leukaemia, chronic lymphoblastic leukemia, acute lymphoblastic leukemia, chronic myelomonocytic leukemia (CMML), or promyelocytic leukemia), multiple myeloma and other myeloid malignancies (myelofibrosis with myeloid metaplasia (MMM), primary myelofibrosis (PMF), idiopathic myelofibrosis (IMF)), lymphoma (Hodgkin's disease, cutaneous lymphoma (cutaneous T-cell lymphoma, mycosis fungoides), lymphoma (for example, B-cell lymphoma, T-cell lymphoma, mantle cell lymphoma, hairy cell lymphoma, Burkitt's lymphoma, mastocytoma, Hodgkin's disease, or non-Hodgkin's disease); Kaposi's sarcoma, rhabdomyosarcoma, seminoma, teratoma, osteosarcoma, follicular thyroid carcinoma; increased accumulation of exogenous or synthetic opioids, notalgia paresthetica, obsessive-compulsive disorder, obsessive-compulsive disorder- associated nostopathy and combinations thereof.

Those skilled in the art can easily perceive other aspects and advantages of the present application from the following detailed description. In the following detailed description, only exemplary embodiments of the present application are shown and described. As those skilled in the art will appreciate, the content of the present application enables those skilled in the art to make changes to the detailed description of embodiments which are disclosed without departing from the definition and scope of the invention to which the present application relates. Correspondingly, the drawings and descriptions of the present application are only exemplary rather than restrictive.

### BRIEF DESCRIPTION OF DRAWINGS

The particular features of the invention to which the present application relates are set forth in the appended claims. The features and advantages of the invention to which the present application relates can be better understood with reference to the exemplary embodiments described in detail hereinafter and the appended drawings. A brief description of the appended drawings is as follows:
FIG. 1 shows a mouse rash model after administration of antitumor agents.
FIG. 2 shows photographs of the left side, back and right side of typical rats in the control group and the administration group.
FIG. 3 shows the rash grades of the administration group and the control group at the end of the experiment.
FIG. 4 shows the rash grades of the administration group and the control group at the end of the experiment.
FIG. 5 shows photographs of the left side, back and right side of typical rats in the control group and the administration group.
FIG. 6 shows the rash grades of the administration group and the control group at the end of the experiment.
FIG. 7 shows the rash grades of the administration group and the control group at the end of the experiment.

### DETAILED DESCRIPTION

The embodiments of the present application are described hereinafter by means of specific examples, and those skilled in the art can readily understand the other advantages and effects of the invention as described herein from the disclosure of the description.

### DEFINITION OF TERMS

In the present application, the term "prodrug" is also referred to as "prodrug", which generally refers to an agent or compound converted into a parent drug or active drug molecule *in vivo.* For example, the "prodrug" may be a precursor of another pharmaceutically active molecule that, after administration to a subject, through chemical or physiological processes such as solvolysis or enzymatic cleavage, or under physiological conditions, produces the other pharmaceutically active molecule *in vivo.*

As used herein, the term "optionally substituted alkyl" comprises straight-chain or branched-chain alkyl groups without any substituents, and straight-chain or branched-chain alkyl groups containing one or more non-hydrogen substituents. For example, the alkyl generally comprises a saturated aliphatic hydrocarbon group with the specified number of carbon atoms, which may be branched- or straight-chain. The term "alkyl" also refers to non-aromatic cycloalkyl groups. For example, the alkyl can have 1 - 20 carbons (i.e., C₁ - C₂₀). For example, C₁ - C₁₀ as in "C₁ - C₁₀ alkyl" is defined as a group comprising 1, 2, 3, 4, 5, 6, 7, 8, 9, or 10 carbons having a linear, branched, or cyclic arrangement (i.e., cycloalkyl). The term "cycloalkyl" refers to a monocyclic saturated aliphatic hydrocarbon group with the specified number of carbon atoms. For example, "alkyl" specifically comprises methyl, ethyl, n-propyl, isopropyl, n-butyl, tert-butyl, isobutyl, amyl, hexyl, heptyl, octyl, nonyl, decyl and more, as well as cycloalkyl including cyclopropyl, methylcyclopropyl, 2,2-dimethyl-cyclobutyl, 2-ethyl-cyclopentyl, cyclohexyl and more.

In the present application, the term "bioactive metabolite" generally refers to a metabolite produced by a compound (such as the compound of the present application) processed *in vivo* through metabolism in a human or animal body or cell. For example, the "bioactive metabolite" may comprise derivatives of any formula produced in an individual upon administration of the parent compound. These derivatives can be produced from the parent compound by various biochemical transformations in the body of an individual such as oxidation, reduction, hydrolysis or conjugation, and comprise, for example, oxide and demethylated derivatives. The metabolites of the compound of the invention can be identified using conventional techniques known in the prior art. See for example, Bertolini, G. et al., J. Med. Chem. 40: 2011-2016 (1997); Shan, D. et al., J. Pharm. Sci. 86(7): 765-767; Bagshawe K., Drug Dev. Res. 34: 220-230 (1995); Bodor, N., Advances in Drug Res. 13: 224-331 (1984); Bundgaard, H., Design of Prodrugs (Elsevier Press 1985); and Larsen, I.K., Design and Application of Prodrugs, Drug Design and Development (Krogsgaard-Larsen et al., Editors, Harwood Academic Publishers, 1991). It is clear that chemical compounds that are metabolites of the compound of formula (I) or the tautomers, prodrugs and stereoisomers thereof, as well as the pharmaceutically acceptable salts, esters, and prodrugs of any of them, are comprised in the present application.

In the present application, the term "JAK inhibitor" generally refers to Janus tyrosine kinase inhibitors, which refer to compounds that target, reduce or inhibit Janus tyrosine kinases, may directly or indirectly target one or more JAKs, may inhibit the JAKs at the transcriptional level, translational level and/or post-translational modification level of proteins, may inhibit the quantity or expression of the JAKs, and may inhibit the activity of the JAKs.

In the present application, the term "pharmaceutically acceptable salt" generally refers to a salt that retains the biological effectiveness and properties of the free base or free acid, and is biologically or otherwise desirable. The salt may be a salt formed with an inorganic acid or a salt formed with an organic acid. Furthermore, the pharmaceutically acceptable salt can be prepared by adding an inorganic base or an organic base to the free acid.

In the present application, the term "pharmaceutically acceptable" generally means that the compounds, materials, compositions and/or dosage forms are suitable for use in contact with human and animal tissues without undue toxicity, irritation, allergic reaction or other problems or complications within the scope of reasonable medical judgment, and have a reasonable benefit/risk ratio. In some embodiments, a pharmaceutically acceptable compound, material, composition, and/or dosage form is one approved by a regulatory agency (such as the U.S. Food and Drug Administration, China Food and Drug Administration, or European Medicines Agency) or listed on a generally recognized pharmacopoeia (such as the US Pharmacopoeia, the Chinese Pharmacopoeia or the European Pharmacopoeia) for animals (more particularly for humans).

### DETAILED DESCRIPTION OF THE INVENTION

In one aspect, the present application provides a compound of formula (I), and also provides a pharmaceutically acceptable salt, bioactive metabolite, solvate, hydrate, prodrug, racemate, enantiomer or stereoisomer, 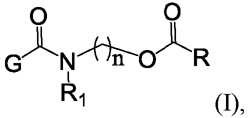 wherein:
G is a JAK inhibitor,
R₁ and R are independently selected from: hydrogen, deuterium, tritium, optionally substituted alkyl, optionally substituted deuterated alkyl, optionally substituted halogenated alkyl, optionally substituted alkoxy, optionally substituted halogenated alkoxy, halogen and optionally substituted cyclic base; and n is 1, 2, 3, 4 or 5.

In another aspect, the present application provides methods for preparing the compound described herein, the pharmaceutically acceptable salt, bioactive metabolite, solvate, hydrate, prodrug, racemate, enantiomer or stereoisomer thereof.

In another aspect, the present application provides a composition comprising the compound described herein, the pharmaceutically acceptable salt, bioactive metabolite, solvate, hydrate, prodrug, racemate, enantiomer or stereoisomer thereof.

In some embodiments, the composition of the present application is a pharmaceutical composition. For example, it may contain one or more pharmaceutically acceptable carriers. For example, the pharmaceutically acceptable carrier may be selected from the group: fillers, binders, disintegrants, buffers, preservatives, lubricants, flavoring agents, thickeners, colorants and emulsifiers.

In some embodiments, the pharmaceutical composition or the compound of the present application is formulated for topical administration. For example, the medicament or the compound may be formulated for transdermal administration. In some embodiments, the medicament or the compound is formulated for topical skin application. In some embodiments, the medicament is formulated as a cream, lotion, gel, ointment, salve, spray, liposomal formulation, liniment and/or aerosol. In some embodiments, the medicament or the compound may be formulated as an ointment.

In some embodiments, the pharmaceutical composition or the compound is an oral formulation. In some embodiments, the pharmaceutical composition or the compound may be an injectable preparation. In some embodiments, the pharmaceutical composition or the compound may be formulated for topical administration within the oral cavity.

In the pharmaceutical composition, the concentration of the compound, the pharmaceutically acceptable salt, bioactive metabolite, solvate, hydrate, prodrug, racemate, enantiomer or stereoisomer may be about 0.0001% to about 50%. For example, the concentration of the compound can be from about 0.0001% (w/w) to about 40% (w/w), for example, can be from about 0.0001% (w/w) to about 30% (w/w), about 0.0001% (w/w) to about 20% (w/w), about 0.0001% (w/w) to about 15% (w/w), about 0.0001% (w/w) to about 10% (w/w), about 0.0001% (w/w) to about 9.5% (w/w), about 0.0001% (w/w) to about 9% (w/w), about 0.0001% (w/w) to about 8.5% (w/w), about 0.0001% (w/w) to about 8% (w/w), about 0.0001% (w/w) to about 7.5% (w/w), about 0.0001% (w/w) to about 7% (w/w), about 0.0001% (w/w) to about 6.5% (w/w), about 0.0001% (w/w) to about 6% (w/w), about 0.0001% (w/w) to about 5.5% (w/w), about 0.0001% (w/w) to about 5% (w/w), about 0.0001% (w/w) to about 4.5% (w/w) , about 0.0001% (w/w) to about 4% (w/w), about 0.0001% (w/w) to about 3.5% (w/w), about 0.0001% (w/w) to about 3% ( w/w), about 0.0001% (w/w) to about 2.5% (w/w), about 0.0001% (w/w) to about 2% (w/w), about 0.0001% (w/w) to about 1.5% (w/w), about 0.0001% (w/w) to about 1% (w/w), about 0.0001% (w/w) to about 0.5% (w/w), about 0.0001% (w /w) to about 0.01% (w/w) or less. In the composition of the present application, the concentration of the compound and the pharmaceutically acceptable salt, bioactive metabolite, solvate, hydrate, prodrug, racemate, enantiomer or stereoisomer thereof can be from about 0.0001% (w/w) to about 5% (w/w), about 0.0001% (w/w) to about 4% (w/w), about 0.0001% (w/w) to about 3% (w/w), about 0.0001% (w/w) to about 2.5% (w/w), about 0.0001% (w/w) to about 2% (w/w), about 0.0001% (w/w) to about 1.5% (w/w), about 0.0001% (w/w) to about 1% (w/w), about 0.0001% (w/w) to about 0.9% (w/w), about 0.0001% (w/w) to about 0.8% (w/w), about 0.0001% (w/w) to about 0.7% (w/w), about 0.0001% (w/w) to about 0.6% (w/w), about 0.05% (w/w) to about 0.5% (w/w), about 0.05% (w /w) to about 0.4% (w/w), about 0.05% (w/w) to about 0.3% (w/w), about 0.05% (w/w) to about 0.2% (w/w), about 0.1% (w/w) to about 0.2% (w/w) or less. For example, the concentration of the compound may be from about 0.05% (w/w) to about 10% (w/w). In some embodiments, the concentration of the compound is about 5% (w/w). In some embodiments, the concentration of the compound is about 2.5% (w/w). In some embodiments, the concentration of the compound is about 1% (w/w).

In the present application, one or more other active ingredients may also be comprised in the composition. For example, the active ingredient may refer to a monomeric compound having medical utility or physiological activity. For example, the other active ingredients may be selected from the group: anti-inflammatory agents, analgesics, local anesthetics, antibiotics, antihistamines, antiseptics, immunosuppressants and anti-hemorrhagic agents.

In another aspect, the present application provides the compound, the pharmaceutically acceptable salt, bioactive metabolite, solvate, hydrate, prodrug, racemate, enantiomer or stereoisomer thereof; or the composition (for example, pharmaceutical composition) of the present application may be used for preventing, alleviating and/or treating a disease or disorder associated with the administration of an antitumor agent in a subject.

For example, the compound described herein, the pharmaceutically acceptable salt, bioactive metabolite, solvate, hydrate, prodrug, racemate, enantiomer or stereoisomer thereof, or the composition described herein (for example, a pharmaceutical composition) can be used for preparing a medicament (for example, a packaged medicament) for preventing, alleviating and/or treating a disease or disorder associated with the administration of an antitumor agent in a subject. In another aspect, the compound described herein, the pharmaceutically acceptable salt, bioactive metabolite, solvate, hydrate, prodrug, racemate, enantiomer or stereoisomer thereof, or the composition described herein (for example, a pharmaceutical composition) can be used for preparing the JAK inhibitor, or a composition (for example, a pharmaceutical composition) comprising the JAK inhibitor.

In another aspect, the present application also provides a method for preventing, alleviating and/or treating a disease or disorder associated with the administration of an antitumor agent in a subject, with the method comprising administering the compound described herein, the pharmaceutically acceptable salt, bioactive metabolite, solvate, hydrate, prodrug, racemate, enantiomer or stereoisomer thereof, or the composition described herein (for example, a pharmaceutical composition) to a subject in need.

In another aspect, the present application provides a method for preventing, treating and/or alleviating a JAK-mediated disease or disorder in a subject in need thereof, the method comprising: administering to the subject an effective amount of the compound described in the present application, the pharmaceutically acceptable salt, bioactive metabolite, solvate, hydrate, prodrug, racemate, enantiomer or stereoisomer thereof; or a composition described in the present application. The effective dose may be a dose capable of effectively preventing, treating and/or alleviating the JAK-mediated disease or disorder.

In some embodiments, the method further comprises administering an additional therapeutic agent. The additional therapeutic agent may be an agent effective in preventing, treating and/or alleviating a JAK-mediated disease or disorder. In some embodiments, the additional therapeutic agent is selected from: Ruxolitinib, Tofacitinib, Oclacitinib, Fedratinib, Peficitinib, Upadacitinib, Barictinib, Fligotinib, Decernotinib, Cerdulatinib, Lestaurtinib, Pacritinib, Momelotinib, Gandotinib, Abrocitinib, Solcitinib, SHR-0203, Itacitinib, PF-06651600, BMS-986165, Abrocitinib, Cucurbitacin I, CHZ868, TD-1473, Zotiraciclib, Alkotinib, Jaktinib, AZD-4205, DTRMHS-07, KL130008, WXSH-0150, TQ05105, WXFL10203614, GLPG0634, CEP-33779, R-348, Itacitinib, Ritlecitinib, Brepocitinib, Tasocitinib, Deucravacitinib, INCB-039110, Izencitinib, Entrectinib, Ivarmacitinib, Deuruxolitinib, Adelatinib, NDI-034858, Nezulcitinib, ATI-01777, TD-8236, INCB-054707, Ropsacitinib, AGA-201, ATI50001, Gusacitinib, Cerdulatinib, Roniciclib, AT-9283, FMX-114, OST-122, TT-00420, Repotrectinib, INCB-052793, CT-340, BMS-911543, Ilginatinib, BGB-23339, ICP-332, ESK-001, SYHX-1901, VTX-958, TLL-018, CEE-321, CJ-15314, TD-5202, ABBV-712, GLPG-3667, CPL-116, AZD-4604, TAS-8274, MAX-40279, TD-3504, KN-002, AZD-0449, R-548, AC-410, Spebrutinib, ONX-0805, AEG-41174, XL-019, CR-4, WP-1066, GDC-0214, INCB-047986, PF-06263276, R-333, AZD-1480, Tozasertib, CS-12192 and AC-1101.

In another aspect, the present application provides a compound described herein, a pharmaceutically acceptable salt, bioactive metabolite, solvate, hydrate, prodrug, racemate, enantiomer or stereoisomer thereof, or use of the composition described in the present application for preparing a medicament for preventing, treating and/or alleviating a JAK-mediated disease or disorder in a subject in need.

### COMPOUND

In the compound of the present application, the R₁ can be hydrogen, optionally substituted alkyl or optionally substituted deuterated alkyl. For example, the R₁ can be hydrogen, optionally substituted C₁ - C₆ alkyl or optionally substituted C₁ - C₆ deuterated alkyl. For example, the R₁ can be hydrogen, optionally substituted C₁ - C₃ alkyl or optionally substituted C₁ - C₃ deuterated alkyl. In some embodiments, the R₁ is hydrogen, optionally substituted methyl or optionally substituted deuterated methyl.

For example, the R₁ can be unsubstituted alkyl (for example, unsubstituted C₁ - C₆ alkyl, unsubstituted C₁ - C₃ alkyl, or unsubstituted methyl).

In some embodiments, the R₁ can also be substituted alkyl (for example, substituted C₁ - C₆ alkyl, substituted C₁ - C₃ alkyl, or substituted methyl). When the R₁ is a substituted alkyl, it may be substituted by any other group, for example, it may be substituted by one or more groups selected from the group: hydrogen, alkyl, alkoxy, cycloalkyl, heterocycloalkyl, aryl, heteroaryl, alkylcarbonyl, alkoxycarbonyl, cycloalkylcarbonyl, heterocycloalkylcarbonyl, arylcarbonyl, heteroarylcarbonyl, hydroxy, halogen, carboxyl, amino, cyano and/or sulfonyl.

In some embodiments, the R₁ is selected from: -(CH₂)ₙ₁Ra, -(CH₂)ₙ₁ORa, -(CH₂)ₙ₁SRa, - (CH₂)ₙ₁C(O)Ra, -(CH₂)ₙ₁C(O)ORa, -(CH₂)ₙ₁S(O)ₘ₁Ra, -(CH₂)ₙ₁NRaRb, -(CH₂)ₙ₁C(O)NRaRb, - (CH₂)ₙ₁NRaC(O)Rb and -(CH₂)ₙ₁NRaS(O)ₘ₁Rb; wherein: the Ra and Rb can be the same or different, and each independently selected from hydrogen, deuterium, tritium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl, and heteroaryl, wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl; or, the Ra and Rb are connected to form a cycloalkyl, heterocyclyl, aryl or heteroaryl, wherein the cycloalkyl, heterocyclyl, aryl and heteroaryl are optionally substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl; n1 is 0 - 5, for example, the n1 can be 0, 1, 2, 3, 4 or 5; and m1 is 0, 1 or 2.

In some embodiments, the R₁ is a high molecular compound (for example, a high molecular compound with multiple repeating units, or a high molecular compound obtained by crosslinking). In the compound of the present application, the R may be selected from: optionally substituted alkyl, optionally substituted deuterated alkyl, optionally substituted cycloalkyl, optionally substituted heterocycloalkyl, optionally substituted aryl and optionally substituted heteroaryl.

For example, the R can be selected from: optionally substituted C₁ - C₂₀ alkyl, optionally substituted C₁ - C₂₀ deuterated alkyl, optionally substituted C₃ - C₂₀ cycloalkyl, optionally substituted C₁ - C₂₀ heterocycloalkyl, optionally substituted C₆ - C₂₀ aryl and optionally substituted C₁ - C₂₀ heteroaryl. For example, the R can be selected from: optionally substituted C₁ - C₁₀ alkyl, optionally substituted C₁ - C₁₀ deuterated alkyl, optionally substituted C₃ - C₁₀ cycloalkyl, optionally substituted C₁ - C₁₀ heterocycloalkyl, optionally substituted C₆ - C₁₀ aryl and optionally substituted C₁ - C₁₀ heteroaryl.

For example, the R can be unsubstituted alkyl, deuterated alkyl, cycloalkyl, heterocycloalkyl, aryl or heteroaryl. In some embodiments, the R is unsubstituted C₁ - C₂₀ alkyl, C₁ - C₂₀ deuterated alkyl, C₃ -C₂₀ cycloalkyl, C₁ - C₂₀ heterocycloalkyl, C₆ - C₂₀ aryl or C₁ - C₂₀ heteroaryl. In some embodiments, the R is unsubstituted C₁ - C₁₀ alkyl, C₁ - C₁₀ deuterated alkyl, C₃ -C₁₀ cycloalkyl, C₁ - C₁₀ heterocycloalkyl, C₆ - C₁₀ aryl or C₁ - C₁₀ heteroaryl.

In some embodiments, the R can also be substituted alkyl, substituted deuterated alkyl, substituted cycloalkyl, substituted heterocycloalkyl, substituted aryl or substituted heteroaryl. In some embodiments, the R is substituted C₁ - C₂₀ alkyl, substituted C₁ - C₂₀ deuterated alkyl, substituted C₃ - C₂₀ cycloalkyl, substituted C₁ - C₂₀ heterocycloalkyl, substituted C₆ - C₂₀ aryl or substituted C₁ - C₂₀ heteroaryl. In some embodiments, the R is substituted C₁ - C₁₀ alkyl, substituted C₁ - C₁₀ deuterated alkyl, substituted C₃ - C₁₀ cycloalkyl, substituted C₁ - C₁₀ heterocycloalkyl, substituted C₆ - C₁₀ aryl or substituted C₁ - C₁₀ heteroaryl. When the R is substituted alkyl, substituted deuterated alkyl, substituted cycloalkyl, substituted heterocycloalkyl, substituted aryl or substituted heteroaryl, it can be substituted by any other group, for example it may be substituted by one or more groups selected from the group: hydrogen, alkyl, alkoxy, cycloalkyl, heterocycloalkyl, aryl, heteroaryl, alkylcarbonyl, alkoxycarbonyl, cycloalkylcarbonyl, heterocycloalkylcarbonyl, arylcarbonyl, heteroarylcarbonyl, hydroxy, halogen, carboxyl, amino, cyano and/or sulfonyl.

In some embodiments, the R is selected from: -(CH₂)ₙ₁Ra, -(CH₂)ₙ₁ORa, -(CH₂)ₙ₁SRa, - (CH₂)n1C(O)Ra, -(CH₂)ₙ₁C(O)ORa, -(CH₂)n1S(O)m1Ra, -(CH₂)ₙ₁NRaRb, -(CH₂)ₙ₁C(O)NRaRb, -(CH₂)ₙ₁NRaC(O)Rb and -(CH₂)ₙ₁NRaS(O)ₘ₁Rb; wherein: the Ra and Rb can be the same or different, and each independently selected from hydrogen, deuterium, tritium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl, and heteroaryl, wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl; or, the Ra and Rb are connected to form a cycloalkyl, heterocyclyl, aryl or heteroaryl, wherein the cycloalkyl, heterocyclyl, aryl and heteroaryl are optionally substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl; n1 is 0 - 5, for example, the n1 can be 0, 1, 2, 3, 4 or 5; and m1 is 0, 1 or 2.

In some embodiments, the R is a high molecular compound (for example, a high molecular compound with multiple repeating units, or a high molecular compound obtained by crosslinking). In the present application, the alkyl may comprise: methyl, ethyl, propyl, isopropyl, butyl, isobutyl, tert-butyl, pentyl, isopentyl, tert-pentyl, hexyl, isohexyl, methylpentyl, dimethylbutyl, heptyl, octyl, nonyl or decyl. The substituted alkyl may be substituted with a group selected from the group: hydroxyl, halogen, ester, nitrile and carbonyl.

In some embodiments of the present application, the cyclic group (for example, a saturated cyclic group, a partially saturated cyclic group, or an unsaturated cyclic group, with or without heteroatoms) can be a three-membered ring, four-membered ring, five-membered ring, six-membered ring, bridged ring, and/or fused ring. In some embodiments, the substituted cyclic group (for example, cycloalkyl, heterocycloalkyl, aryl, or heteroaryl) can be substituted with a group selected from the group: hydroxyl, halogen, ester, nitrile and carbonyl.

When the R is optionally substituted heterocycloalkyl (for example, optionally substituted C₁ - C₂₀ heterocycloalkyl, or optionally substituted C₁ - C₁₀ heterocycloalkyl), or optionally substituted heteroaryl (for example, optionally substituted C₁ - C₂₀ heteroaryl, or optionally substituted C₁ - C₁₀ heteroaryl), it may comprise heteroatoms (for example, monovalent groups of the following heteroatoms) selected from the group: fluorine, oxygen, sulfur, and nitrogen.

In some embodiments, the heterocyclyl (for example, heterocycloalkyl or heteroaryl) may be an optionally substituted six-membered nitrogen-containing heterocyclyl. In some embodiments, the heterocyclyl (for example, heterocycloalkyl or heteroaryl) may be selected from: furan, thiophene, pyrrole, thiazole, imidazole, pyridine, pyrazine, pyrimidine, pyridazine, indole, quinoline, pteridine and acridine.

In some embodiments, the cyclic group is selected from: cycloalkyl, heterocyclyl, oxoheterocyclyl, thioheterocyclyl, aryl and heteroaryl. In some embodiments, wherein cycloalkyl, heterocyclyl, oxoheterocyclyl, thioheterocyclyl, aryl and heteroaryl are optionally substituted with one or more groups selected from the following group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl.

In some embodiments, the optionally substituted aryl is an optionally substituted phenyl. For example, the aryl (for example, phenyl) may be substituted with a group selected from the group: hydroxy, halogen, ester, nitrile and carbonyl.

In some embodiments, the n is 1 - 5. In some embodiments, the n is 2 - 4. In some embodiments, the n is 1, 2 or 3. In some embodiments, the n is 1. In some embodiments, the n is 2. In some embodiments, the n is 3. In some embodiments, the n is 4. In some embodiments, the n is 5.

In some embodiments of the compound of the present application, the R₁ is hydrogen, optionally substituted methyl or optionally substituted deuterated methyl, the n is 2, and the R is optionally substituted C₃-C₂₀ cycloalkyl or optionally substituted C₁-C₂₀ heterocycloalkyl.

In some embodiments of the compound of the present application, the R₁ is hydrogen, optionally substituted methyl or optionally substituted deuterated methyl, the n is 2, and the R is optionally substituted C₃-C₁₀ cycloalkyl or optionally substituted C₁-C₁₀ heterocycloalkyl.

In some embodiments of the compound of the present application, the G is a JAK inhibitor selected from the group: Ruxolitinib, Tofacitinib, Oclacitinib, Fedratinib, Peficitinib, Upadacitinib, Barictinib, Fligotinib, Decernotinib, Cerdulatinib, Lestaurtinib, Pacritinib, Momelotinib, Gandotinib, Abrocitinib, Solcitinib, SHR-0203, Itacitinib, PF-06651600, BMS-986165, Abrocitinib, Cucurbitacin I, CHZ868, TD-1473, Zotiraciclib, Alkotinib, Jaktinib, AZD-4205, DTRMHS-07, KL130008, WXSH-0150, TQ05105, WXFL10203614, GLPG0634, CEP-33779, R-348, Itacitinib, Ritlecitinib, Brepocitinib, Tasocitinib, Deucravacitinib, INCB-039110, Izencitinib, Entrectinib, Ivarmacitinib, Deuruxolitinib, Adelatinib, NDI-034858, Nezulcitinib, ATI-01777, TD-8236, INCB-054707, Ropsacitinib, AGA-201, ATI50001, Gusacitinib, Cerdulatinib, Roniciclib, AT-9283, FMX-114, OST-122, TT-00420, Repotrectinib, INCB-052793, CT-340, BMS-911543, Ilginatinib, BGB-23339, ICP-332, ESK-001, SYHX-1901, VTX-958, TLL-018, CEE-321, CJ-15314, TD-5202, ABBV-712, GLPG-3667, CPL-116, AZD-4604, TAS-8274, MAX-40279, TD-3504, KN-002, AZD-0449, R-548, AC-410, Spebrutinib, ONX-0805, AEG-41174, XL-019, CR-4, WP-1066, GDC-0214, INCB-047986, PF-06263276, R-333, AZD-1480, Tozasertib, CS-12192 and AC-1101.

In some embodiments of the compounds herein, the G is a JAK inhibitor selected from the group: Tofacitinib, Ruxolitinib, Baricitinib, Peficitinib, Pacritinib, Delgocitinib, Pf-04965842, Upadacitinib, Filgotinib, Itacitinib, Fedratinib, Decernotinib, SHR-0302, ASN-002, Cerdulatinib, BMS-986165, PF-06700841, INCB-52793, ATI-502, PF-06651600, AZD-4205, Deuterium Modified Ruxolitinib Analog, ATI-501, R-348, NS-018, Jaktinib Hydrochloride and KL-130008. For example, the G may comprise (or have) a structure selected from the group: 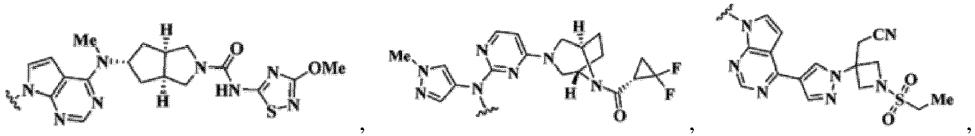 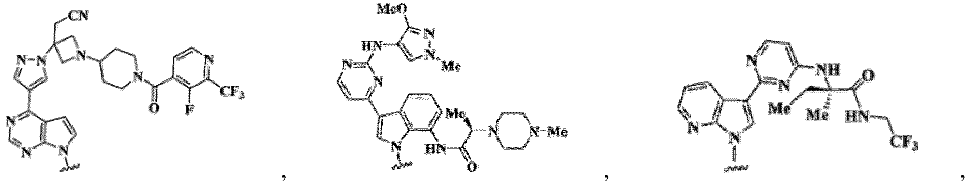 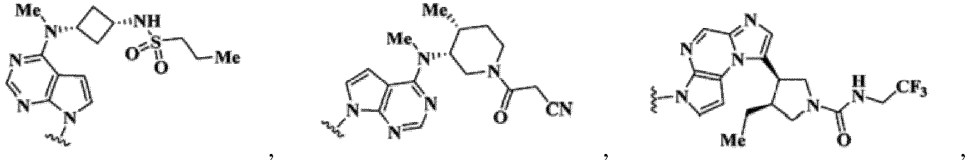 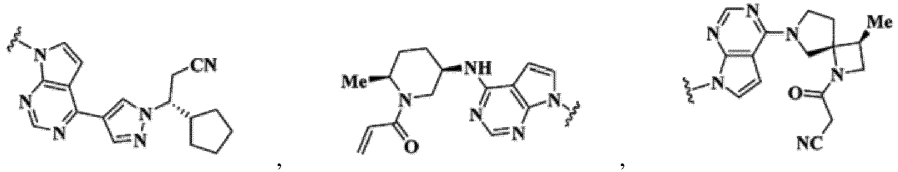 and 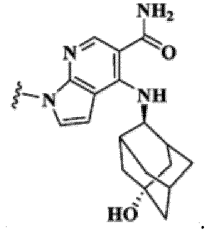 For example, the structure of Tofacitinib is

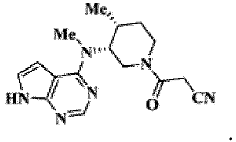

In some embodiments, the compounds of the present application are selected from the group:

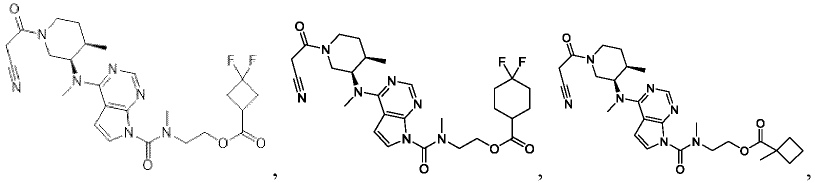

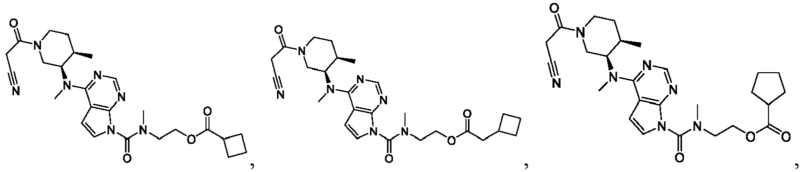

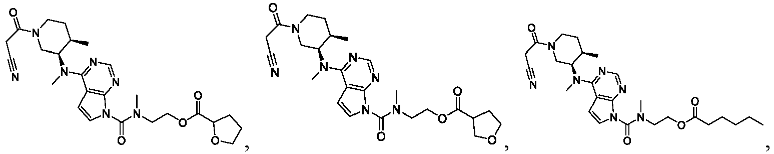

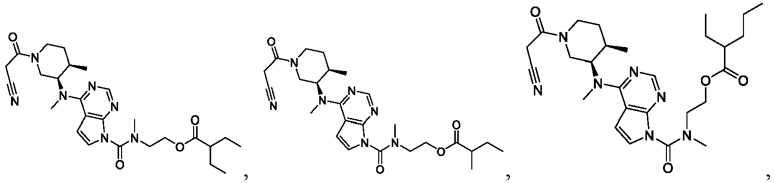

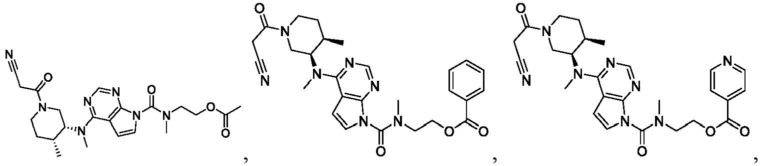

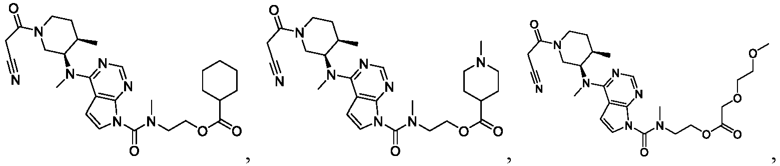

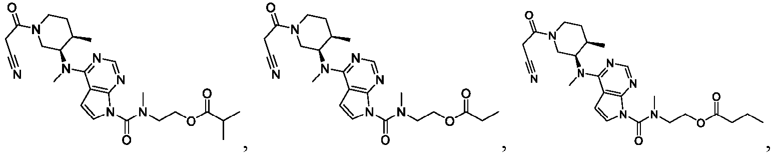

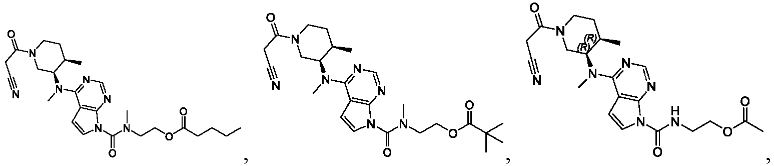

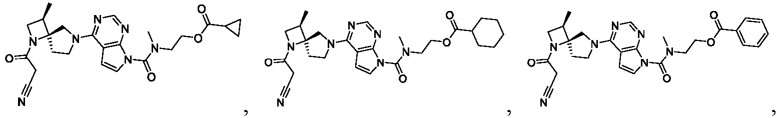

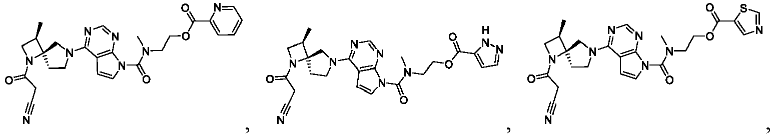

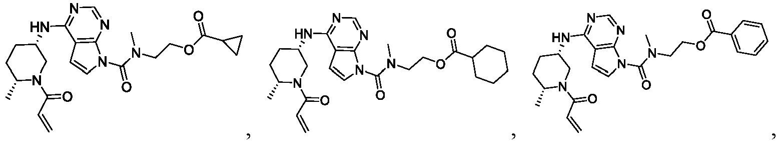

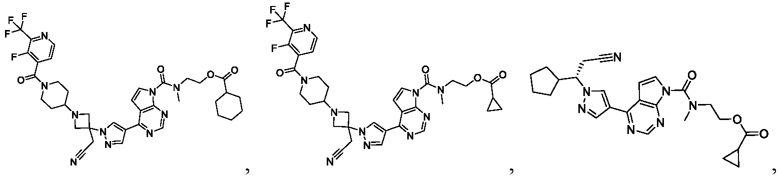

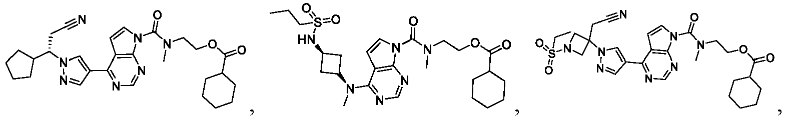

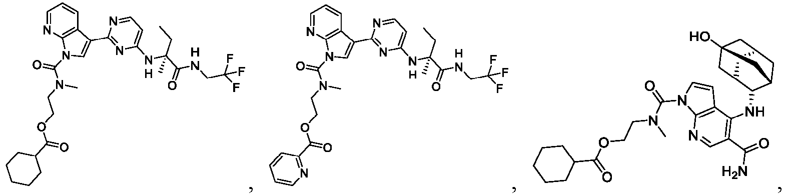

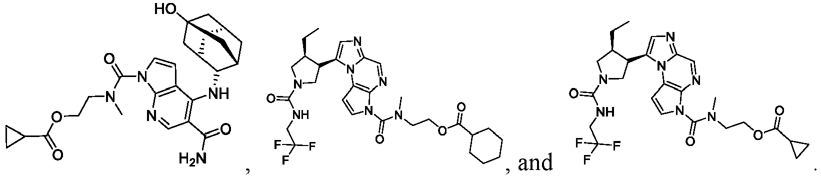

### Antitumor Agent

According to any aspect of the present application, the antitumor agent may comprise a small molecule compound, a small molecule conjugate, a protein and/or a polynucleotide.

For example, the antitumor agent may comprise a chemotherapeutic agent, a targeted therapeutic agent and/or an immunotherapeutic agent.

In some embodiments, the antitumor agent is a targeted therapeutic agent. The targeted therapeutic agent may comprise a small molecule compound and/or an antibody or an antigen-binding fragment thereof. The antibody may comprise a monoclonal antibody, a multispecific antibody, a chimeric antibody, a humanized antibody, a fully human antibody and/or an antibody-drug conjugate. The antigen-binding fragment may comprise Fab, Fab', F(ab)₂, a Fv fragment, F(ab')₂, scFv, di-scFv and/or dAb.

In some embodiments, the targeted therapeutic agent targets molecules inside tumor cells, on the surface of tumor cells and/or in the tumor microenvironment. For example, the targeted therapeutic agent may target proteins and/or nucleic acid molecules. For example, the targeted therapeutic agent may target a tumor-associated antigen.

In some embodiments, the targeted therapeutic agent targets one or more targets selected from the group: VEGF, EGFR, EGFR1, EGFR2, EGFR3, EGFR4, HER2, HER3, HER4, VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic agent can inhibit activity of one or more targets selected from the group: VEGF, EGFR, EGFR1, EGFR2, EGFR3, EGFR4, HER2, HER3, HER4, VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic agent can reduce expression of one or more targets selected from the group: VEGF, EGFR, EGFR1, EGFR2, EGFR3, EGFR4, HER2, HER3, HER4, VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic agent may comprise a hormone therapy, a signal transduction inhibitor, a gene expression modulator, an apoptosis inducer, an angiogenesis inhibitor and/or a toxin delivery molecule.

In some embodiments, the targeted therapeutic agent is a tyrosine kinase inhibitor.

In some embodiments embodiments, the targeted therapeutic agent is a VEGFR inhibitor and/or a VEGF inhibitor. For example, the VEGFR inhibitor may inhibit VEGFR1, VEGFR2 and/or VEGFR3. In some embodiments, the targeted therapeutic agent is an EGFR inhibitor. In some embodiments, the targeted therapeutic agent is a BRAF inhibitor. In some embodiments, the targeted therapeutic agent is a PDGFR inhibitor. In some embodiments, the targeted therapeutic agent is an FGFR inhibitor. In some embodiments, the targeted therapeutic agent is an mTOR inhibitor. In some embodiments, the targeted therapeutic agent is an HER2 inhibitor.

For example, in embodiments of any aspect of the present application, the EGFR (for example, HER2) inhibitor may be selected from the following compounds and pharmaceutically acceptable salts thereof: Afatinib, Olmutinib, Lapatinib, Gefitinib, and Dacomitinib.

For example, in embodiments of any aspect of the present application, the VEGFR inhibitor and/or VEGF inhibitor may be selected from the following compounds and pharmaceutically acceptable salts thereof: Ramucirumab, Bevacizumab, Anlotinib, Regorafenib, Cabozantinib, Lenvatinib, Sorafenib, Fruquintinib, Famitinib, Apatinib, Axitinib and Nintedanib.

For example, in embodiments of any aspect of the present application, the BRAF inhibitor may be selected from the following compounds and pharmaceutically acceptable salts thereof: Vemurafenib, Encorafenib, Selumetinib, and Dabrafenib.

For example, in embodiments of any aspect of the present application, the PDGFR inhibitor may be selected from the following compounds and pharmaceutically acceptable salts thereof: Sunitinib and Nintedanib.

For example, in embodiments of any aspect of the present application, the FGFR inhibitor may be selected from the following compounds and pharmaceutically acceptable salts thereof: Erdafitinib and Infigratinib.

For example, in embodiments of any aspect of the present application, the mTOR inhibitor may be selected from the following compounds and pharmaceutically acceptable salts thereof: Everolimus.

For example, the targeted therapeutic agent described herein may be selected from one or more of the following group: Afatinib, Dacomitinib, Osimertinib, EAI045, Gefitinib, Almonertinib, Pyrotinib, Brigatinib, Neratinib, Olmutinib, Bosutinib, Icotinib, Vandetanib, Lapatinib, Alflutinib, BPI-7711, Mobocertinib, Dovitinib, Zorifertinib, Varlitinib, Orelabrutinib, Acalabrutinib, Brutinib, Ibrutinib, Dasatinib, Pirtobrutinib, Tolebrutinib, Rilzabrutinib, Fenebrutinib, Evobrutinib, Selumetinib, Tivozanib, Dovitinib, Surufatinib, Binimetinib, Cobimetinib, Trametinib, Regorafenib, GSK-1120212, Alpelisib, Duvelisib, Copanlisib, Idelalisib, Nortriptyline, Inavolisib, Dactolisib, Apitolisib, Parsaclisib, Buparlisib, Rigosertib, Enzastaurin, Paxalisib, Leniolisib, Ipatasertib, Zotarolimus, Sirolimus, Everolimus, Temsirolimus, Sorafenib, Apatinib, Lenvatinib, Sunitinib, Cabozantinib, Axitinib, Nintedanib, Brivanib, Vatalanib, Fruquintinib, Dabrafenib, Vemurafenib, Encorafenib, Pazopanib, Crizotinib, Panobinostat, Erlotinib, Rituximab, Panitumumab, Cetuximab, Erfonrilimab, Efactinib, Cadonilimab, Ramucirumab, Bevacizumab, Anlotinib, Ponatinib, Famitinib, Erdafitinib, AZD4547, Infigratinib, BCD-217, Amivantamab, MCLA-129, EMB-01, LY3164530, JNJ-61186372, anti-EGFR and cMet bispecific antibodies, GB263, pharmaceutically acceptable salts thereof, and any composition thereof. Wherein the EGFR and cMet bispecific antibodies may be as described in WO2010115551A1, WO2014081954A1 or WO2015016559A1.

According to any aspect of the present application, the targeted therapeutic agent may be administered in combination with one or more other therapies.

In some embodiments, the antitumor agent is a chemotherapeutic agent. For example, the chemotherapeutic agent may comprise a pyrimidine nucleoside analog and/or a prodrug thereof. In some embodiments, the chemotherapeutic agent comprises one or more selected from the group: Capecitabine, Cytarabine, Docetaxel, Adriamycin, Fluorouracil (5-FU), Fluorouridine, Tegafur, Idarubicin, Paclitaxel, Epirubicin, Acelarin (NUC-1031), Doxorubicin, Folinate, Cisplatin, Paclitaxel, Cyclophosphamide, Vincristine and 5-FU prodrug.

In some embodiments, the chemotherapeutic agent comprises one or more selected from the group: tegafur, 5'-deoxyfluorouridine, fluorouridine, 2'-deoxy-fluorouridine, prodrug derivative of fluorouridine, prodrug derivative of 2'-deoxy-fluorouridine, trifluoro-methyl-2'-deoxyuridine, 6-azauridine and 3-deazauridine.

For example, the chemotherapeutic agent may be administered in combination with one or more other therapies. In some embodiments, the one or more other therapies comprise one or more other antitumor therapies described herein (for example, antitumor agents).

For example, the antitumor agents (for example cytotoxic anti-cancer agents) may comprise alkylating agent such as, Nitrogen Mustard, Nitrogen Mustard N-oxide Hydrochloride, Chlorambucil, Cyclophosphamide, Ifosfamide, Thiotepa, Isothiocyanate, Busulfan, Nimustine Hydrochloride, Mitropium bromide, Melphalan, Dacarbazine, Ranimustine, Sodium Phopofol Phosphate, Ethylene Triamine, Carmustine, Lomustine, Streptozotocin, Pipobroman, Ethoglucid, Carboplatin, *cis*-platin, Miriplatin, Nedaplatin, Tinidamine, Omustine, Dichloropyridine, Fupisitan, Prednipixitine, Pumitepa, Ribomustin Hydrochloride, Temozolomide, Diclofenac, Trovafloxacin, Zinostatin, Simvastatin, Penem, Cystemustine and Bizelesin; Antimetabolites such as Mercaptopurine, 6-mercaptopurine nucleoside, Thioinosine, Methotrexate, Pemetrexed, Entectine, Cytarabine, Oxaliplatin, Tesabatin Hydrochloride, 5-FU and the derivatives thereof (for example, Fluorouracil, Tegafur, UFT, Dosiholu, Carmofur, Capecitabine, etc.), Aminopterin, Nazothioamine, Calcium Leucovorin, Microphyllum, Calcium Folinate, Calcium Levofate, Cladribine, Emitoful, Fludarabine, Gemcitabine, Hydroxylurea, Pentostatin, Piritrexim, Iodouridine, Nitoguanone, Tiazolylfuran, Vernal stat, and Bendamustine; Antitumor antibiotics such as Dactinomycin D, Dactinomycin C, Mitomycin C, Chromomycin A3, Bleomycin Hydrochloride, Bleomycin Sulfate, Cetimycin Hydrochloride, Doxorubicin Hydrochloride, Mitoxantrone Hydrochloride and Idarubicin Hydrochloride; and/or plant-derived cytotoxic antitumor agents such as Etoposide, Etoposide Phosphate, Vinblastine Sulfate, Vincristine Sulfate, Teniposide, Paclitaxel, Docetaxel, and Vinorelbine; VEGF inhibitors such as Bevacizumab, and those disclosed in PCT patent applications WO 2005/012359, WO 2005/044853, WO 98/45332, WO 96/30046, WO 94/10202, US Patents US 7,060,269, US 6,582,959, US 6,703,020, US 6,054,297, US Patent Applications US 2006/009360, US 2005/0186208, US 2003/0206899, US 2003/0190317, US 2003/0203409 and US 2005/0112126.

In some embodiments, the antitumor agent may be an immunotherapeutic antitumor agent that may comprise, for example: Bropirimine, Krestin, Etofuran, Lentinan, Ubenimex, Interferon, Interleukin, Macrophage Colony Stimulating Factor, Granulocyte Colony Stimulating Factor, Erythropoietin, Lymphotoxin, BCG Vaccine, *Corynebacterium parvum,* Everolimus, Levamisole, Polysaccharide K, Procodazole and/ or immune checkpoint inhibitors (eg, CTLA4 inhibitors, TIM-3 inhibitors, PD-1 inhibitors (eg, Nivolumab, Pembrolizumab, Pidilizumab, AMP514 (Amplimmune), AMP-224, and other PD-1 inhibitors disclosed in PCT patent applications WO2006/121168, WO2009/114335, WO2009/101611, US patent US 8609089, US patent applications US2010/028330 and US2012/0114649), PD-L1 inhibitors (for example, YW243.55.S70, MPDL3280A, MEDI-4736, MSB-0010718C, MDX-1105, and other PD-L1 inhibitors disclosed in PCT patent application WO2010/077634 and U.S. Pat. No. 7,943,743)).

In some embodiments, the antitumor agent may comprise a hormone therapy antitumor agent. For example, the hormone therapy antitumor agent may comprise Fusitatine, Stilboestrol, Chlorinated Costen, Medroxyprogesterone Acetate, Megestrol Acetate, Cyproterone Acetate, Cyproterone Acetate, Danazol, Allylestrenol, Progesterone, Mepartricin, Raloxifene or Meloxifene, Levofloxacin, Anti-estrogens (e.g., Tamoxifen Citrate, Toremifene Citrate, etc.), contraceptives, Prostacyclin, Testolactone, Aminosuccinimide, LH-RH agonists (e.g., Goserelin Acetate, Buserelin, Leuprorelin, etc.), Droloxifene, Epiandrostanol, Ethinylestradiol Sulfonate, Fubenzole Hydrochloride, Anastrozole, Letrozole, Exemestane, Vorozole, Anti-androgens (e.g., Flutamide, Bicalutamide, Nilutamide, etc.), 5α-Reductase Inhibitors (e.g., Finasteride, Epristeride), Corticosteroids (e.g., Dexamethasone, Prednisolone, Betamethasone, Triamcinolone Acetonide, etc.) and/or androgen synthesis inhibitors (e.g., Abiraterone, etc.).

### Disease or Disorder Associated with an Antitumor Agent

According to any aspect of the present application, a disease or disorder associated with administration of the antitumor agent may be caused by the antitumor agent alone or by multiple treatment regimens including the antitumor agent.

In some embodiments, the disease or disorder is caused by administration of the antitumor agent. For example, the disease or disorder may onset or progression after administration of the antitumor agent.

In some embodiments, the subject has not suffered the disease or disorder prior to administration of the antitumor agent.

For example, the disease or disorder may comprise an epithelial disease or disorder. The epithelial disease or disorder may comprise a disease or disorder associated with an endothelial cell lesion, and/or a disease or disorder associated with an epithelial cell lesion. For example, the epithelial cells may comprise skin epithelial cells, oral epithelial cells, nasal epithelial cells, gastric epithelial cells, and/or intestinal epithelial cells.

In some embodiments, the endothelial cells comprise vascular endothelial cells. The lesion of vascular endothelial cells may comprise endothelial dysfunction. For example, the lesion of vascular endothelia cell may comprise degenerative vascular disease (e.g., atherosclerosis, Mönckeberg arteriosclerosis and arteriolosclerosis (e.g., hyline degenerating arteriolosclerosis and proliferative arteriolosclerosis)), inflammatory vascular disease (e.g., infectious arteritis, syphilitic arteritis, giant cell arteritis, thromboangitis obliterans and rheumatic arteritis), functional vascular disease (e.g., Raynaud's disease, acrocyanosis and erythromelalgia) and congenital vascular disease (e.g., congenital arteriovenous fistula) and more.

In the present application, the epithelial cells may comprise skin epithelial cells, oral epithelial cells, nasal cavity epithelial cells, gastric epithelial cells and/or intestinal epithelial cells. For example, the lesion of epithelial cell may comprise lesion of cutaneous epithelial cell (e.g., rash, acne, rosacea, atopic dermatitis, contact dermatitis, seborrheic dermatitis, lupus, scleroderma, pemphigus, pigmentation, melasma, vitiligo, urticaria, tinea corporis, pruritus, alopecia, hair change, erythema, paronychia and schizonychia, xerosis cutis, hypersensitivity and psoriasis), lesion of oral epithelial cell (fe.g., pemphigus, herpes labialis, herpetic stomatitis, granulomatous cheilitis, oral ulcer, pemphigoid, Sjögren's syndrome, Bechet's disease and oral sarcoidosis, etc.), lesion of nasal epithelial cell (epistaxis, sinusitis, nasal furuncle and nasal polyps, etc.), lesion of gastric epithelial cell (e.g., gastritis, intestinal metaplasia, gastric perforation, gastric fistula, gastric ulcer and gastrointestinal polyp) and/or lesion of small intestinal epithelial cell (e.g., enteritis, Crohn's disease, enterobrosis, intestinal fistula, enterelcosis, ulcerative colitis and NSAIDs enteropathy) and the like.

The inventors of the present application have found that an antitumor agent causes damage to endothelial cells, endothelial tissues, thereby causing diseases or disorders of skin tissues, oral cavity tissues, nasal cavity tissues and/or gastrointestinal tract tissues. In the onset and progression of these diseases or disorders, the disease process typically begins with damage/lesion of endothelial cells and endothelial tissues, and epithelial cells may also exhibit pathological changes, ultimately manifesting in the patient as endothelial cell lesions associated with the administration of anti-tumor agents and/or epithelial cell lesions associated with the administration of anti-tumor agents.

For example, the disease or disorder may comprise a skin disease or disorder, a sensory disease or disorder, and/or a gastrointestinal disease or disorder.

In some embodiments, the skin disease or disorder comprises alopecia, body odor, bullous dermatitis, dry skin, eczema, erythema multiforme, erythroderma, lipoatrophy, hair color changes, abnormal hair texture, hirsutism, hyperhidrosis, hyperkeratosis, hypertrichosis, hypohidrosis, lipohypertrophy, nail changes, nail discoloration, nail loss, nail ridges, skin pain, hand-foot syndrome, photosensitivity, pruritus, purpura, acneiform rash, maculopapular rash, scalp pain, skin atrophy, skin hyperpigmentation, skin hypopigmentation, skin induration, skin ulcers, Stevens - Johnson syndrome, subcutaneous emphysema, telangiectasia, toxic epidermal necrosis, rash and/or urticaria.

In some embodiments, the skin disease or disorder is hand-foot syndrome.

In some embodiments, the present application relates to the use of a compound of the present application (for example, a compound of Formula (I) herein) for preventing, alleviating and/or treating a disease or disorder (for example, rash) associated with administration of the antitumor agent (for example, an EGFR inhibitor) in a subject.

In some embodiments, the severity of the skin disease or disorder is Grade 1 or above, Grade 2 or above, Grade 3 or above, Grade 4 or above, or Grade 5 according to NCI-CTCAE.

In some embodiments, the subject comprises a cancer patient. In some embodiments, the affected site of the skin disease or disorder is different from that of cancer.

In the present application, the disease or disorder associated with administration of the antitumor agent may be statistically significantly correlated to the antitumor agent. In some embodiments, the disease or disorder associated with administration of the antitumor agent may be caused by the antitumor agent. For example, the disease or disorder associated with the administration of the anti-tumor agent may comprise a skin disease or disorder, a sensory disease or disorder, and/or a gastrointestinal disease or disorder associated with the administration of the anti-tumor agent. For example, the skin disease or disorder, the sensory disease or disorder, and/or the gastrointestinal disease or disorder associated with the administration of the anti-tumor agent may comprise epithelial tissue diseases or disorders associated with the administration of the anti-tumor agent in the skin tissue, sensory features, and/or gastrointestinal tract. In some embodiments, the disease or disorder associated with the administration of the anti-tumor agent may comprise side effects or adverse reactions caused by the administration of the anti-tumor agent.

In the present application, the disease or disorder associated with administration of the antitumor agent may be a new indication different from any other disease or disorder in the past. For example, the disease or disorder associated with administration of the antitumor agent may be unique in its diagnosis, treatment and/or symptoms. For example, erythromycin ointment is capable of treating rash, while has no effect on treating the rash associated with administration of the antitumor agent. In some embodiments, the disease or disorder associated with administration of the antitumor agent may comprise a rash associated with administration of the antitumor agent, hand-foot syndrome associated with administration of the antitumor agent, pruritus associated with administration of the antitumor agent, erythema associated with administration of the antitumor agent, xerosis cutis associated with administration of the antitumor agent, alopecia associated with administration of the antitumor agent, paronychia associated with administration of the antitumor agent, pigmentation disorder associated with administration of the antitumor agent, oral unclers associated with administration of the antitumor agent, xerostomia associated with administration of the antitumor agent, epistaxis associated with administration of the antitumor agent, nasopharyngitis associated with administration of the antitumor agent, cheilitis associated with administration of the antitumor agent, esophagitis associated with administration of the antitumor agent, esogastritis associated with administration of the antitumor agent, gastric ulcer associated with administration of the antitumor agent, diarrhea associated with administration of the antitumor agent, vomiting associated with administration of the antitumor agent, nausea associated with administration of the antitumor agent, anorexia associated with administration of the antitumor agent, constipation associated with administration of the antitumor agent, and/or abdominal pain associated with administration of the antitumor agent. For example, the disease or disorder associated with administration of the antitumor agent comprises hand-foot syndrome associated with administration of the antitumor agent. For example, the severity of the disease or disorder associated with administration of the antitumor agent is Grade 1 or above, Grade 2 or above, Grade 3 or above, Grade 4 or above, and/or Grade 5 according to NCI-CTCAE V5.0.

In some embodiments, the disease or disorder may comprise rash, hand-foot syndrome, pruritus, erythema, xerosis cutis, alopecia, paronychia, pigmentation disorder, oral unclers, xerostomia, epistaxis, nasopharyngitis, cheilitis, esophagitis, esogastritis, gastric ulcer, diarrhea, vomiting, nausea, anorexia, constipation and/or abdominal pain. For example, the disease or disorder comprises hand-foot syndrome.

In some embodiments, the disease or disorder associated with the antitumor agent is substantially incapable of being treated or alleviated by administration of an agent selected from the group: 1% sildenafil, urea cream, vaseline ointment, urea ointment, brimonidine ointment, vitamin B6 ointment, nicotine ointment, dexamethasone ointment, hydrocortisone ointment, vitamin K1 ointment (0.1%), erythromycin ointment and triamcinolone acetonide ointment.

In the present application, the severity of the disease or disorder may increase after the antitumor agent is administered. For example, the severity of the disease or disorder may increase by about 5% or above, about 10% or above, about 15% or above, about 20% or above, about 25% or above, about 30% or above, about 35% or above, about 40% or above, about 45% or above, about 50% or above, about 60% or above, about 70% or above, about 80% or above, about 90% or above, about 100% or above, about 200% or above or more.

In the present application, the subject may not have suffered from the disease or disorder before the antitumor agent is administered. In the present application, the term "the subject does not have the disease or disorder" generally means that a subject has no past medical history involving the disease or disorder associated with the administration of the antitumor agent. For example, the subject does not have a disease or disorder associated with the administration of the antitumor agent more than 1 day, 1 week, 1 month, 1 year, or 10 years before the administration of the antitumor agent, or since the birth of the subject.

### JAK-mediated Disease or Disorder

According to any aspect of the present application, the JAK-mediated disease or disorders may be selected from: autoimmune disorders or autoimmune responses, extensive activation of immune response, bacterial infections, viral infections, inflammation, chronic and/or acute inflammatory disorders or conditions, and/or autoinflammatory disorders, fibrotic disorders, metabolic disorders, neoplasm, or cardiovascular or cerebrovascular disorders, skin disorders, pruritus, hair loss disorders, cancer or malignancy, autoimmune connective tissue diseases and autoimmune conditions; Still's disease, adult-onset Still's disease, Th17-associated inflammation, polychondritis (for example, relapsed polychondritis); myositis, polymyositis, autoimmune myositis, dermatomyositis, juvenile dermatomyositis; myasthenia gravis; arthritis (for example, rheumatoid arthritis, juvenile rheumatoid arthritis, systemic-onset juvenile rheumatoid arthritis, osteoarthritis, infectious arthritis, inflammatory arthritis, inflammatory bowel disease-associated arthritis, idiopathic arthritis, juvenile idiopathic arthritis, systemic juvenile idiopathic arthritis, psoriatic arthritis), spondylitis/spondyloarthritis/spondyloarthropathy (ankylosing spondylitis), gout, scleroderma (systemic scleroderma, juvenile scleroderma), Reiter's syndrome/reactive arthritis, Lyme disease, lupus/systemic lupus erythematosus (SLE) (lupus erythematosus, juvenile systemic lupus erythematosus, cutaneous lupus (subacute cutaneous lupus, chronic cutaneous lupus/discoid lupus, chilblain lupus erythematosus)), polymyalgia rheumatica, enthesitis, mixed connective tissue disease, enthesopathy, carditis, myocarditis, angiogenesis disorder, myelodysplastic syndrome, atherosclerosis, atherosclerotic restenosis (atherosclerotic coronary restenosis), acute coronary syndrome, myocardial infarction, cardiac allograft vasculopathy, transplanted arterial disease; vasculitis (large vessel vasculitis, small vessel vasculitis, giant cell arteritis, polyarteritis nodosa, and vasculitic syndromes, which comprise aortitis, Wegener's granulomatosis and behcet disease), Stimulator of Interferon Genes (STING)-associated vasculopathy with onset in infancy (SAVI); gastrointestinal disorders, enterocolitis, colitis, inflammatory bowel disease (ulcerative colitis and Crohn's disease), irritable bowel syndrome, enteritis syndrome/spastic colon, celiac sprue; acute and chronic pancreatitis; primary biliary cirrhosis, primary sclerotic cholangitis, jaundice, cirrhosis (for example, primary biliary cirrhosis, or cirrhosis attributable to fatty liver diseases (for example, alcoholic fatty liver disease and non-alcoholic fatty liver disease); esophagitis, gastritis, gastric and duodenal ulcers, peritonitis; nephropathy, immune-mediated glomerulonephrosis, autoimmune nephropathy, membranous glomerulopathy, chronic progressive nephropathy, diabetic kidney disease/diabetic nephropathy, renal fibrosis, renal ischemia/reperfusion injury, HIV-associated nephropathy, ureteral obstructive nephropathy, glomerulosclerosis, proteinuria, nephrotic syndrome, polycystic kidney disease, autosomal dominant polycystic kidney disease, immune-mediated nephropathy, autoimmune nephropathy, chronic progressive nephropathy, diabetic nephropathy, renal fibrosis, ischemic/reperfusion injury-associated nephropathy, HIV-associated nephropathy, ureteral obstructive nephropathy, glomerulonephritis, chronic kidney disease (for example, diabetic nephropathy), hypertension-induced nephropathy, glomerulosclerosis, proteinuria, nephrotic syndrome, polycystic kidney disease, autosomal dominant polycystic kidney disease, diabetic kidney disease, lupus nephritis; interstitial cystitis; periodontitis, gingivitis; pulmonary inflammation, sinusitis, pneumonia, bronchitis, asthma, bronchial asthma, atopic asthma, non-atopic asthma, allergic bronchopulmonary mycosis, aspirin-induced asthma, adult-onset asthma, fixed airflow obstruction asthma, exercise-induced asthma, cough variant asthma, work related asthma, nocturnal asthma, asthma complicated with obesity, eosinophilic asthma, hormone resistant asthma/severe asthma, exogenous asthma, endogenous asthma/cryptogenic asthma, Churg-Strauss syndrome, bronchiolitis, obliterative bronchiolitis, chronic obstructive pulmonary disease (COPD), interstitial pulmonary disease (pulmonary fibrosis, idiopathic pulmonary fibrosis), acute lung injury, pulmonary fibrosis (for example, idiopathic pulmonary fibrosis or cystic fibrosis), chronic obstructive pulmonary disease, adult respiratory distress syndrome, acute lung injury, drug-induced lung injury; Meniere's disease; eye disorders (including ocular inflammation, uveitis, dry eye/keratoconjunctivitis sicca, scleritis, episcleritis, keratitis/keratopathy, choroiditis, retinal vasculitis, optic neuritis, retinopathy (diabetic retinopathy, immune-mediated retinopathy, macular degeneration, wet macular degeneration and dry (senile) macular degeneration)); mastocytosis, iron-deficiency anemia, uremia, hypereosinophilic syndrome (HES), systemic mastocyte disease (SMCD), myelodysplastic syndrome, idiopathic thrombocytopenic purpura; bone resorption diseases; neurodegenerative disorders, neurological/neuromuscular disorders (for example, multiple sclerosis, Parkinson's disease, Huntington's disease, amyotrophic lateral sclerosis (ALS) (including familial ALS and sporadic ALS), Alzheimer's disease, myasthenia gravis, Lambert-Eaton myasthenic syndrome (LEMS), Guillain-Barre syndrome, meningitis, encephalitis, traumatic brain injury; nervous system damage, delusional parasitosis, dysregulation of neuronal processes and sensory perception, stroke/neuronal ischemia, spinal cord injury, peripheral neuropathy, pseudaphia, spinal cord injury, mental disease; paresthesia pain (acute pain, chronic pain, neuropathic pain, or fibromyalgia), nerve stimulation, peripheral neuropathy; pruritus/scrapie (atopic pruritus, dry pruritus, psoriasis-associated pruritus/psoriatic scrapie/psoriasis-associated scrapie), acute pruritus, chronic pruritus, idiopathic pruritus, chronic idiopathic pruritus, biliary scrapie, hepatobiliary-associated scrapie, renal-associated scrapie/renal scrapie, uremic scrapie, cholestasis, intrahepatic cholestasis of pregnancy, lichen simplex chronicus-associated pruritus, lymphoma-associated scrapie, leukemia-associated scrapie, prurigo nodularis, atopic dermatitis-associated scrapie, atopic scrapie/atopic pruritus, bullous scrapie, brachioradialis pruritus, neurogenic scrapie, neurotic scrapie, notalgia paresthetica, HIV-associated pruritic papules, psychogenic scrapie, swimmer scrapie, itch or uremic scrapie, urticaria scrapie; skin diseases (for example, dermatological drug reaction/drug eruption, dermatoxerasia/dry skin, rash, skin sensitization, skin irritation, sunburn, infection caused by shaving, body lice, head lice/phthiriasis, crab louse, cutaneous larva migrans, scabies, parasitic infections, insect infections, urticaria, papular urticaria, insect bites, insect stings, dandruff, foreign bodies or devices on the skin, fungal infections, herpes, varicella, eosinophilic folliculitis, dermatosis of pregnancy/pruritic urticaria papula and plaque (PUPP), inflammatory dermatosis, neutrophilic dermatosis, histiocytoid neutrophilic dermatosis, intestinal bypass syndrome dermatosis, psoriasis/psoriasis vulgaris, lichen planus, lichen sclerosus et atrophicus, acne (acne vulgaris, comedonal acne, inflammatory acne, nodular cystic acne, keloidalis acne, acne keloidalis nuchae), atopy (allergic contact sensitization, allergic dermatitis), dermatitis (atopic dermatitis/eczema, contact dermatitis, photodermatitis, seborrheic dermatitis, stasis dermatitis), acute febrile neutrophil dermatosis (Sweet syndrome), chronic atypical neutrophilic dermatosis with lipodystrophy and elevated temperature (CANDLE syndrome), hidradenitis suppurativa, urticaria, pyoderma gangraenosum, alopecia (eyebrow loss, hair loss intranasal, alopecia cicatrisata (for example, alopecia scarring, alopecia scarring centralis, lichen planopilaris, frontal fibrosing alopecia, folliculitis decalvans), non-cicatricial alopecia (alopecia areata (AA) (patchy AA, alopecia totalis (AT), alopecia universalis (AU), alopecia serpentina, alopecia horseshoe)), androgenic/androgenic alopecia (AGA)/AGA in males and females ), telogen effluvium, tinea capitis, hypotrichosis (simple hereditary hypotrichosis), lichen planopilaris (frontal fibrosing alopecia), punctate palmoplantar keratodermasis, erythema elevatum diutinum (EED), neutrophilic eccrine hidradenitis, palisaded neutrophilic granulomatous dermatitis, neutrophilic urticaria dermatosis, vitiligo (for example, segmental vitiligo (including single segmental vitiligo, double segmental vitiligo and multi segmental vitiligo), non-segmental vitiligo (for example, acrotype vitiligo, facial vitiligo, acrofacial vitiligo, central facial vitiligo, mucosal vitiligo, confetti macules, trichrome vitiligo, borderline inflammatory vitiligo, quadrichrome vitiligo, blue vitiligo, Koebner phenomenon, vitiligo vulgaris, generalized vitiligo, common vitiligo), mixed vitiligo/ non-segmental vitiligo accompanied by segmental vitiligo, localized vitiligo, solitary mucous vitiligo and vitiligo with or without gray hair (body hair involved); bullous diseases, immune bullous disease (for example, bullous pemphigus, scarring pemphigus, pemphigus vulgaris, linear IgA disease), pemphigoid gestationis, xeroderma pigmentosum; fibrosis and scarring disorders; fibroma, liver fibrosis, pulmonary fibrosis, idiopathic pulmonary fibrosis (for example, scleroderma, increased fibrosis, keloid, low-grade scarring of postoperative scars); wound healing, surgical scarring and radiation-induced fibrosis (for example, head and neck, gastrointestinal tract, or lungs), central nervous system scarring, digestive or gastrointestinal tract fibrosis, renal fibrosis, liver or bile duct fibrosis, liver fibrosis (for example, non-alcoholic steatohepatitis, hepatitis C, or liver cell carcinoma), cardiac fibrosis (for example, endomyocardial fibrosis or atrial fibrosis), ocular scarring, fibrosclerosis, scar growth, wound or scab healing, keloid, mediastinal fibrosis, myelofibrosis, retroperitoneal fibrosis/Ormond disease, progressive mass fibrosis, renal systemic fibrosis; Sjogren syndrome, sarcoidosis, familial Mediterranean fever, cryopyrin-associated periodic syndrome (Muckle-Wells syndrome, familial cold autoinflammatory syndrome/familial cold urticaria /TNF receptor-associated periodic syndrome, neonatal-onset multisystem inflammatory disease), oxygen-induced inflammation, reperfusion injury, postoperative trauma, tissue injury, hyperthermia syndrome; diabetes (Type I and II diabetes)/diabetes, Hashimoto thyroiditis, Graves' disease, Addison's disease, Castleman's disease, hyperparathyroidism, menopause, obesity, steroid resistance, glucose intolerance, metabolic syndrome, thyroid disease, pituitaritis; systemic immunosenescence; autoimmune atrophic gastritis, autoimmune atrophic gastritis with pernicious anemia, autoimmune encephalomyelitis, autoimmune orchitis, Goodpasture syndrome, Sjogren syndrome, autoimmune thrombocytopenia, sympathetic ophthalmia; secondary hematological manifestations of autoimmune diseases (for example, anemia), autoimmune hemolytic syndrome (autoimmune hemolytic anemia), autoimmune hepatitis and inflammatory hepatitis, autoimmune ovarian failure, autoimmune thrombocytopenia, silicone implantation-associated autoimmune diseases, drug-induced autoimmune, HIV-associated autoimmune syndrome, metal-induced autoimmune, autoimmune deafness, and autoimmune thyroid disorders; anaphylaxis and allergy (including hypersensitivity, for example, Type I hypersensitivity (for example, allergies), Type II hypersensitivity (for example, Goodpasture syndrome and autoimmune hemolytic anemia), Type III hypersensitivity (for example, Arthus reaction, serum disease) and Type IV hypersensitivity (for example, contact dermatitis and allograft rejection); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); rejection (for example, graft-versus-host reaction/graft-versus-host disease, allograft rejection (for example, acute/chronic allograft rejection), early allograft rejection; malignancies, cancers, lymphoma, leukemia, multiple myeloma, solid tumors, teratoma, metastatic disorders and bone disorders, internal cancer, bone cancer, oral/pharyngeal cancer, esophageal cancer, larynx cancer, gastric cancer, intestinal cancer, colon cancer, rectum cancer, lung cancer (for example, non-small cell lung cancer or small cell lung cancer), hepatic cancer (liver cancer), pancreatic cancer, neurve cancer, brain cancer (for example, glioma, spongioblastoma multiforme, astrocytoma, neuroblastoma, and neurinoma), head and neck cancer, throat cancer, ovarian cancer, uterine cancer, prostate cancer, testicular cancer, bladder cancer, renal cancer (kidney cancer), breast cancer, gallbladder cancer, cervical cancer, thyroid cancer, prostate cancer, eye cancer (eye malignancies), and skin cancer (melanoma and keratoacanthoma); and fibrotic cancers, fibromas, fibroadenomas, fibrosarcomas, myeloproliferative disorders, neoplasms (hematopoietic tissue neoplasms, spinal cord neoplasms, lymphoid tissue neoplasms (myelofibrosis, primary myelofibrosis, polycythemia vera, essential thrombocytosis)), leukemias (acute lymphoblastic leukemia, acute myelocytic leukemia, and chronic myelocytic leukaemia, chronic lymphoblastic leukemia, acute lymphoblastic leukemia, chronic myelomonocytic leukemia (CMML), or promyelocytic leukemia), multiple myeloma and other myeloid malignancies (myelofibrosis with myeloid metaplasia (MMM), primary myelofibrosis (PMF), idiopathic myelofibrosis (IMF)), lymphoma (Hodgkin's disease, cutaneous lymphoma (cutaneous T-cell lymphoma, mycosis fungoides), lymphoma (for example, B-cell lymphoma, T-cell lymphoma, mantle cell lymphoma, hairy cell lymphoma, Burkitt's lymphoma, mastocytoma, Hodgkin's disease, or non-Hodgkin's disease); Kaposi's sarcoma, rhabdomyosarcoma, seminoma, teratoma, osteosarcoma, follicular thyroid carcinoma; increased accumulation of exogenous or synthetic opioids, notalgia paresthetica, obsessive-compulsive disorder, obsessive-compulsive disorder- associated nostopathy and combinations thereof.

### Pharmaceutical Combination and Pharmaceutical Composition

In another aspect, the present application provides a pharmaceutical combination, comprising: 1) an antitumor agent; and 2) a compound of formula (I). In some embodiments, the antitumor agent and the compound of the present application (for example, a compound of formula (I) herein) are not mixed with each other in the pharmaceutical combination. In some embodiments, the antitumor agent and the compound of the present application (for example, a compound of formula (I) herein) are each stored in separate containers in the pharmaceutical combination. In some embodiments, the compound of the present application (for example, a compound of formula (I) herein) is prepared for transdermal administration in the pharmaceutical combination. In some embodiments, the compound of the present application (for example, a compound of formula (I) herein) is prepared as an ointment in the pharmaceutical combination. In some embodiments, the concentration of the compound of the present application (for example, a compound of Formula (I) herein) in the pharmaceutical combination is from about 0.005% w/w to about 40% w/w. In some embodiments, the concentration of the compound of the present application (for example, a compound of Formula (I) herein) in the pharmaceutical combination is from about 0.05% w/w to about 10% w/w.

According to any aspect of the present application, the composition or compound does not substantially affect the therapeutic effect of the antitumor agent.

In the present application, the "substantially does not affect" may refer to the therapeutic effect of using the compound of the present application (for example, a compound of formula (I) herein) combined with the antitumor agent is comparable to or does not show significant inferiorities as compared with that of the antitumor agent alone. For example, for any subject, the reduction in tumor volume caused by using a compound of the present application (for example, a compound of formula (I) of the present application) combined with the antitumor agent is consistent with that of using the antitumor agent alone, or the reduction is not less than about 5%, not less than about 4%, not less than about 3%, not less than about 2%, not less than about 1%, not less than about 0.5%, not less than about 0.1%, not less than about 0.01%, not less than about 0.001% or less.

In some embodiments, the administration site of the drug is different from that of the antitumor agent. In some embodiments, the drug is administered at a site other than the site of cancer or a potential site of cancer metastasis.

In some embodiments, the administration mode of the drug is different from the antitumor agent. In the pharmaceutical combination and/or kit of the present application, in some embodiments, the antitumor agent and the compound of the present application (for example, a compound of formula (I) herein) are not mixed with each other.

In some embodiments, the antitumor agent and the compound of the present application (for example, a compound of formula (I) herein) are each stored in separate containers. For example, two or more drugs packaged separately from each other may be comprised in the pharmaceutical combination, wherein at least one of the drugs comprises the antitumor agent described herein and at least one additional drug comprises a compound of formula (I) described herein.

In some embodiments and in the pharmaceutical combination, wherein the compound of the present application in 2) (for example, a compound of formula (I) herein) can prevent, alleviate and/or treat a disease or disorder associated with the antitumor agent in 1).

In some embodiments, the compound of the present application in 2) (for example, a compound of formula (I) herein) does not substantially affect the therapeutic effect of the antitumor agent in 1).

In some embodiments, the compound of the present application (for example, a compound of formula (I) herein) in 2) is administered before, concurrently with or after the administration of the antitumor agent in 1) in the pharmaceutical combination herein.

### Prevention and/or Treatment Methods

In the present application, the subject may comprise a human or a non-human animal. For example, a non-human animal may be selected from the group: monkey, chicken, goose, cat, dog, mouse and rat. In addition, non-human animals may also comprise any animal species other than humans, such as livestock animals, rodents, primates, domestic animals, or poultry animals. Humans may be Caucasian, African, Asian, Semitic, or of other races, or a mixture of races. As another example, the human may be an elderly, an adult, a teenager, a child, or an infant.

The effective amount in humans can be estimated from that in laboratory animals. For example, Freireich et al. described the correlation of doses in animals and humans (based on mg/m² (BSA)) (Freireich et al., Cancer Chemother. Rep. 50, 219 (1966)). The body surface area (BSA) can be approximately determined from a patient's height and weight. For example, as shown in Scientific Tables, Geigy Pharmaceuticals, Ardsley, N.Y, 537 (1970).

According to the method of the present application, the antitumor agent may be used due to administering the antitumor agent to the subject.

For example, a compound of the present application (for example, a compound of formula (I) herein) may be administered before, concurrently with or after administering the antitumor agent to the subject. When the antitumor agent described herein is administered concurrently with the compound of the present application (for example, the compound of formula (I) hereof), the compound of the present application (for example, the compound of formula (I) hereof) is administered at about 0.0001-10% relative to the total dose (for example, about 0.005-10%, about 0.01-10%, about 0.05-10%, about 0.1-10%, about 0.2-10%, about 0.3-10%, about 0.4-10%, about 0.5-10%, about 0.6-10%, about 0.7-10%, about 0.8-10%, about 0.9-10%, about 0.95-10%, about 1-10%, about 2-10%, about 3-10%, about 5-10%, about 6-10%, about 8-10% or a smaller range). In the embodiments, wherein the compound of the present application (for example, a compound of formula (I) herein) is administered at intervals with the antitumor agent, the compound of the present application (for example, a compound of formula (I) herein) may be administered at intervals before or after administering the antitumor agent. The intervals can be 1 minute, 2 minutes, 5 minutes, 10 minutes, 20 minutes, 30 minutes, 45 minutes, 1 hour, 2 hours, 3 hours, 4 hours, 5 hours, 6 hours, 12 hours, 18 hours, 1 day, 2 days, 3 days, 1 week, 2 weeks, 3 weeks, 1 month, 2 months, 3 months or longer.

The present application also provides a method comprising administering to a subject a compound of formula (I), wherein the subject was, is and/or will be given the antitumor agent and suffers from or is susceptible to a disease or disorder associated with administration of the antitumor agent. The present application also provides a method for preventing or treating a disease or disorder, comprising administering a compound of formula (I) to a subject susceptible to or suffering from the disease or disorder, wherein the subject was, is and/or will be given the antitumor agent.

In the present application, the subject may have suffered from a disease or disorder associated with administering the antitumor agent, or the subject may have a high probability of suffering from a disease or disorder associated with administration of the antitumor agent.

The present application also provides a method comprising the steps of: 1) monitoring one or more side effects, such as skin tissue, sensory organs and/or gastrointestinal characteristics, in a subject administered with the antitumor agent; 2) administering a compound of formula (I) to the subject when the monitoring shows that the subject experiences side effects associated with administering the antitumor agent, such as a disease or disorder involving the skin, sensory organs and/or gastrointestinal tract.

In the present application, the method may also comprise continuing to monitor a disease or disorder involving the skin, sensory organs and/or gastrointestinal tract, and optionally reducing or discontinuing the antitumor agent. For example, continued monitoring refers to monitoring for approximately at least 1 day, at least 1 week, at least 10 days, at least 2 weeks, at least 3 weeks, at least 1 month, at least 3 months or longer after administration of the antitumor agent. For example, the reduction or discontinuation means that a dose of the antitumor agent administered to the subject is reduced by about at least 5%, at least 10%, at least 20%, at least 30%, at least 40%, at least 50%, at least 60%, at least 70%, at least 80%, at least 90%, at least 95%, at least 99% or 100% as compared with the dose of the antitumor agent in step 1) of the method.

In the present application, the severity of the disease or disorder associated with the administration of an antitumor agent may increase after administering the antitumor agent. For example, the severity may be an increase of at least 5%, at least 10%, at least 20%, at least 30%, at least 40%, at least 50%, at least 60%, at least 70%, at least 80%, at least 90% or more.

In the present application, the subject may not have suffered from the disease or disorder before the antitumor agent is administered.

In the present application, the compound of the present application (for example, a compound of formula (I) herein) may be topically administered to the subject. For example, the compound of the present application (for example, a compound of formula (I) herein) may be topically administered to a site in the subject substantially free of cancer cells. As another example, the compound of the present application (for example, a compound of Formula (I) herein) may be administered to a non-cancer site in the subject.

Without intending to be limited by any theory, the following embodiments are only offered to illustrate the compounds, preparation methods and uses in the present application, but not to limit the scope of the present application.

### EXAMPLES

### Preparation of Compound

### Preparation of Compound A1: 2-(4-((3R,4R)-1-(2-cyanoacetyl)-4-methylpiperidin-3-yl)(methyl)amino)-N-methyl-7H-pyrrole [2,3-d] pyridine -7-formylamino)ethyl 3,3-difluorocyclobutane-1-carboxylate

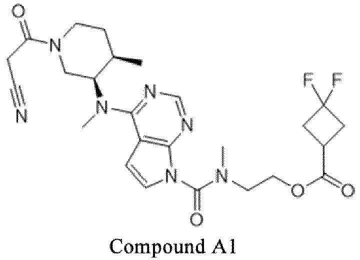

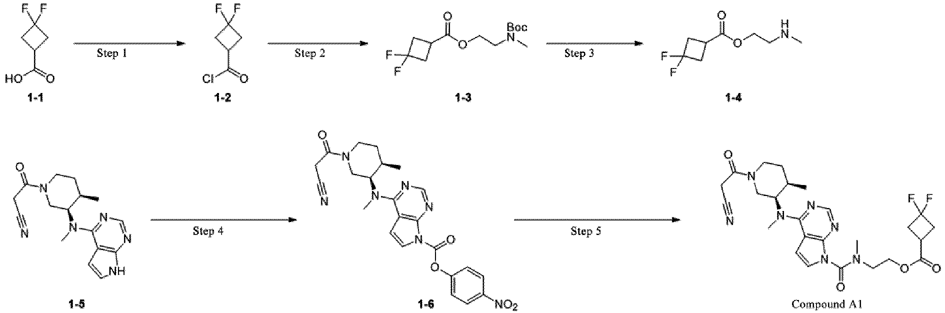

### Step 1

### 3,3-difluorocyclobutane formyl chloride (1-2)

To a mixture of 3,3-difluorocyclobutanecarboxylic acid (3.0 g, 22.0 mmol, 1.0 eq) and N,N-dimethylformamide (161.1 mg, 2.2 mmol, 169.6 uL, 0.1 eq) in dichloromethane (20 mL), add oxalyl chloride (4.2 g, 33.1 mmol, 2.9 mL, 1.5 eq) at 0 °C, and keep stirring at room temperature for 2 hours. Remove the solvent by distillation under reduced pressure and obtain the crude product of 3,3-difluorocyclobutylformyl chloride (3.4 g), which was used in the next step without purification.

### Step 2

### 2-[t-butyloxycarboryl (methyl) amino]ethyl-3,3-difluorocyclobutane formate ester (1-3)

To a mixture of N-BOC-N-methylaminoethanol (2.5 g, 14.3 mmol, 1.0 eq) and triethylamine (14.4 g, 142.7 mmol, 19.9 mL, 10.0 eq) in dichloromethane (20 mL), add 3,3-difluorocyclobutylformyl chloride (3.3 g, 21.4 mmol, 1.5 eq). Keep stirring at 25°C for 3 hours. Remove the solvent by distillation under reduced pressure and obtain the crude product of 2-[t-butyloxycarboryl (methyl) amino] ethyl-3,3-difluorocyclobutane formate ester (4.0 g), which was used in the next step without purification.

### Step 3

### 2-(methylamino) ethyl-3,3-difluorocyclobutyl ester (1-4)

To a mixture of 2-[t-butyloxycarboryl (methyl) amino] ethyl-3,3-difluorocyclobutane formate ester (1.8 g, 6.1 mmol, 1.0 eq) in dichloromethane (9 mL), add trifluoroacetic acid (4.6 g, 40.5 mmol, 3.0 mL). Keep stirring at 0 °C for 2 hours and distill under reduced pressure to obtain a yellow solid (1.1 g), i.e., the crude product of 2-(methylamino)ethyl-3,3-difluorocyclobutyl ester, which was used in the next step without purification.

### Step 4

### (4-nitrophenyl) 4-[(3R,4R)-1-(2-cyanoacetyl)-4-methyl-3-piperidyl]- methylamino] pyrrole [2,3-d] pyridine-7-carboxylate (1-6)

To a mixture of 3-[(3R,4R)-4-methyl-3-[methyl(7H-pyrrole [2,3-d] pyridine-4-yl)amino]-1-piperidyl]-3-oxopropionitrile (10.0 g, 32.0 mmol, 1.0 eq) in dichloromethane (50 mL), add triethylamine (6.5 g, 64.0 mmol, 8.9 mL, 2.0 eq) and 4-nitrophenyl chloroformate (9.7 g, 48.0 mmol, 1.5 eq). Keep stirring at 20°C for 4 hours, filter and evaporate the filtrate rotatablely to obtain a yellow oil, i.e., the crude product of (4-nitrophenyl) 4-[(3R,4R)-1-(2-cyanoacetyl)-4-methyl-3-piperidyl]-methylamino] pyrrole-[2,3-d] pyridine-7-carboxylate (15.1 g), which was used in the next step without purification.

### Step 5

### 2-[[4-[[(3R,4R)-1-(2-cyanoacetyl)-4-methyl-3-piperidyl]-methylamino] pyrrole- [2,3-d] pyridine -7-carbonyl]-methylamino]ethyl-3,3-difluorocyclobutyl ester (Example A1)

To a mixture of 2-[t-butyloxycarboryl (methyl) amino] ethyl-3,3-difluorocyclobutane formate ester (2.7 g, 5.7 mmol, 1.0 *eq*) and 2-(methylamino)ethyl-3,3-difluorocyclobutyl ester (1.1 g, 5.7 mmol, 1.0 *eq*) in dichloromethane (20 mL), add triethylamine (2.9 g, 28.5 mmol, 3.9 mL, 5.0 *eq*) and stir at 25°C for 6 hours. Remove the solvent by distillation under reduced pressure and perform reversed-phase HPLC (column: Xtimate C18 150*40 mm*10 µm; mobile phase: [water(FA)-ACN]; B%: 25%-60%, 10 min) to obtain a white solid, i.e., 2-[[4-[[(3R,4R)-1-(2-cyanoacetyl)-4-methyl-3-piperidyl]-methylamino] pyrrole-[2,3-d] pyridine-7-carbonyl]-methylamino]ethyl-3,3-difluorocyclobutyl ester (2.1 g, 3.9 mmol, 68% yield, 98% purity). LC-MS: (M+H)⁺, 532.3. ¹H NMR(400 MHz, DMSO-d6): δ (ppm) 8.26-8.16 (m, 1H), 7.32 (br s, 1H), 6.82 (br s, 1H), 4.86 (br d, J = 2.0 Hz, 1H), 4.38 (br s, 1H), 4.22-3.96 (m, 3H), 3.86-3.62 (m, 3H), 3.50-3.41 (m, 3H), 3.34 (br s, 1H), 3.28 (s, 3H), 3.20-3.00 (m, 2H), 2.97-2.51 (m, 5H), 2.42-2.34 (m, 1H), 1.96-1.68 (m, 1H), 1.62-1.48 (m, 1H), 1.02 (d, *J* = 7.0 Hz, 3H).

Other compounds in the following table were synthesized using a procedure similar to that of Compound A1 with proper reactants according to the synthesis of the compound of the present invention, and these compounds were characterized.

| No. | Structure | Description | Ms (M+H+) |
|---|---|---|---|
| A2 | 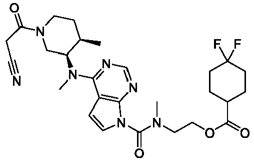 | White solid | 560.0 |
| A3 | 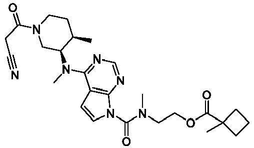 | White solid | 510.3 |
| A4 | 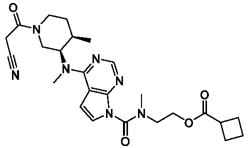 | Yellow solid | 496.3 |
| A5 | 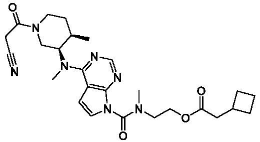 | Yellow solid | 510.3 |
| A6 | 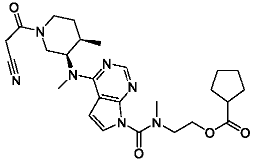 | White solid | 510.9 |
| A7 | 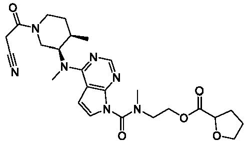 | White solid | 512.2 |
| A8 | 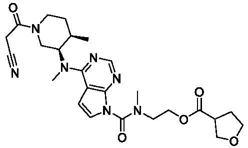 | Yellow solid | 512.5 |
| A9 | 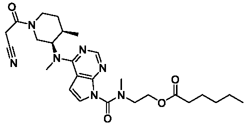 | Yellow solid | 512.3 |
| A10 | 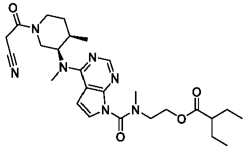 | Yellow oil | 512.4 |
| A11 | 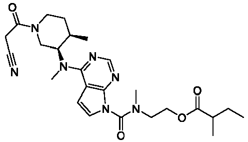 | Yellow solid | 498.5 |
| A12 | 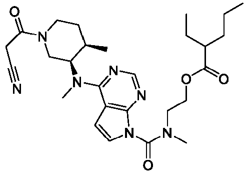 | Yellow solid | 526.1 |
| A13 | 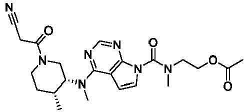 | Yellow solid | 456.4 |
| A14 | 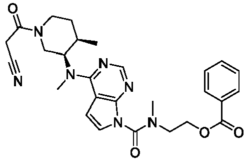 | Yellow solid | 519.2 |
| A15 | 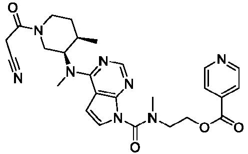 | Yellow solid | 519.3 |
| A16 | 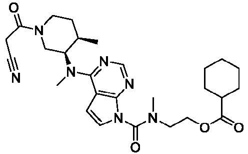 | Yellow solid | 524.5 |
| A17 | 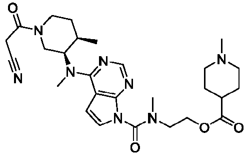 | Yellow solid | 539.2 |
| A18 | 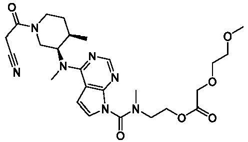 | White solid | 531.3 |
| A19 | 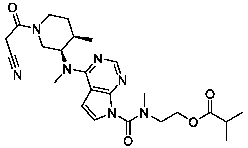 | White solid | 484.3 |
| A20 | 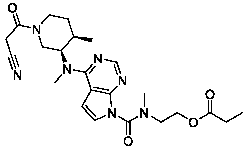 | White solid | 470.2 |
| A21 | 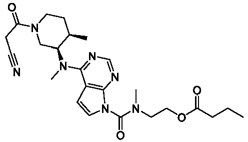 | White solid | 484.3 |
| A22 | 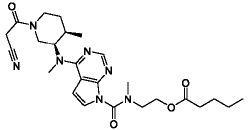 | Green solid | 498.3 |
| A23 | 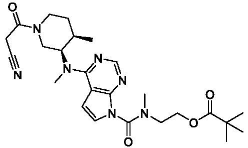 | Green solid | 498.2 |
| A24 | 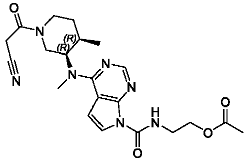 | White solid | 442.3 |
| A27 | 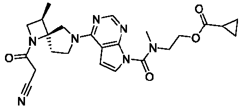 | White solid | 480.2 |
| A28 | 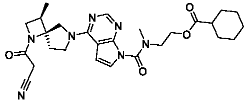 | White solid | 521.1 |
| A29 | 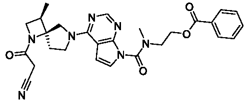 | White solid | 518.0 |
| A30 | 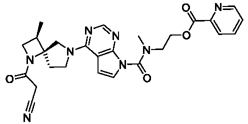 | White solid | 519.0 |
| A31 | 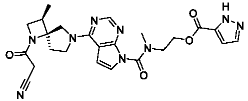 | White solid | 506.4 |
| A32 | 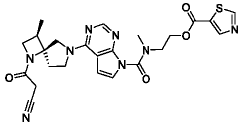 | Yellow solid | 523.3 |
| A33 | 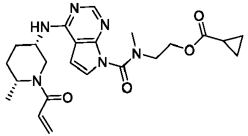 | Yellow solid | 455.3 |
| A34 | 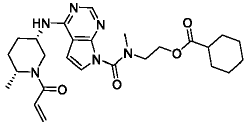 | Yellow solid | 497.2 |
| A35 | 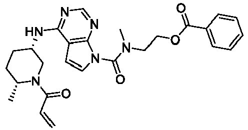 | White solid | 491.2 |
| A36 | 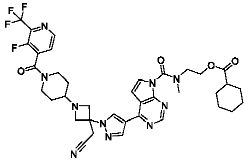 | White solid | 765.5 |
| A37 | 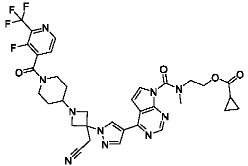 | White solid | 723.3 |
| A38 | 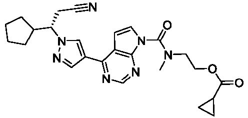 | Yellow solid | 475.1 |
| A39 | 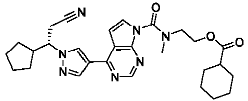 | Yellow solid | 518.4 |
| A40 | 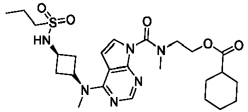 | Yellow solid | 535.2 |
| A41 | 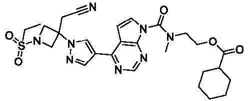 | Yellow solid | 583.3 |
| A42 | 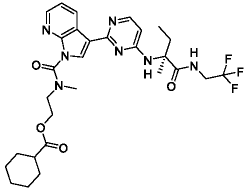 | Yellow solid | 604.2 |
| A43 | 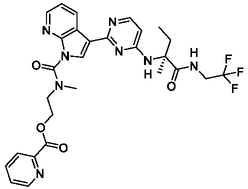 | Yellow solid | 599.3 |
| A44 | 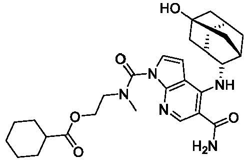 | Yellow solid | 538.2 |
| A45 | 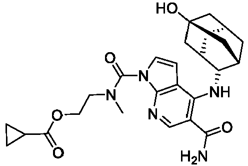 | Yellow solid | 496.1 |
| A46 | 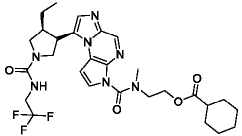 | Yellow solid | 592.2 |
| A47 | 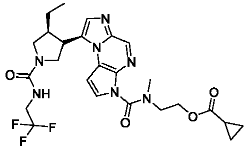 | Yellow solid | 550.3 |

In addition, the solubility and log D of the above compounds were determined. Briefly, the determination method is as follows:

### Thermodynamic Solubility Assay

Weigh not less than 2 mg of the sample powder into a Whatman Mini-UniPrep vial. If it is required to test the thermodynamic solubility of samples in multiple buffers, a separate vial is needed for each test; add 450 µL of buffer to each Whatman Mini-UniPrep vial, respectively; after that, install the piston cap of Whatman Mini-UniPrep and press it above the liquid level, so that the filter is in contact with the buffer during shaking; shake the solubility sample by vortexing for 1 minute. Record the solution phenomenon; shake at 600 rpm at room temperature (about 25°C) for 24 hours; press the cap of Whatman Mini-UniPrep filter vial to the bottom to obtain the filtrate of the sample solubility solution; all sample vials were filtered and the insoluble substances and leakage before and after filtration were recorded; obtain the sample diluent by diluting the buffer by 100 folds. Inject 3 UV standard solutions from low to high concentrations into the HPLC, then inject the diluent and supernatant of the test compound, with two replicates of test sample; and integrate the UV peaks. Simulate a standard curve and calculate the thermodynamic solubility of the sample. Thermodynamic solubility was tested in PBS buffer (pH=6.5), and the thermodynamic stability of isopropyl myristate was tested with isopropyl myristate as the vehicle.

### LogD Measurement

Two replicates of test compounds (DMSO stock solution 10 mM, 2 µL/well) and QCs (DMSO 10 mM, 2 µL/well) were transferred from the storage tube to a 96-well cluster tube. Add buffer-saturated n-octanol (149 µL/well) and n-octanol-saturated PBS (149 µL/well) to each well; vortex mix the cluster tube for 1 minute, mix by shaking on a plate shaker at 880 rpm for one hour, and then centrifuge at 4000 rpm for 10 minutes. Remove the cover pad, accurately measure a certain amount of n-octanol layer samples (4 µL/well) into a 96-well plate containing n-octanol internal standard diluent (796 µL/well); accurately measure a certain amount of buffer salt layer samples (30 µL/well) into a 96-well plate containing buffer internal standard diluent (570 µL/well); the dilution factor of the sample can be adjusted according to different properties of the sample. Samples were analyzed using a triple quadrupole mass spectrometer. The processed sample solution was analyzed by the fast isocratic elution and small column retention method, the concentration correction was carried out by dilution factor and internal standard, and the ratio of the corrected peak area was used to calculate the final result (LogD/LogP value).

The determination results are shown in the following table:

| No. | LogD7.4 (Oct/buff) | Thermodynamic Solubility pH=6.5 (µg/mL) | Thermodynamic Solubility in Isopropyl Myristate (µg/mL) |
|---|---|---|---|
| A1 | 1.45 | 644.96 | 877.79 |
| A2 | 1.86 | 239.96 | 1016.96 |
| A3 | 2.10 | 304.24 | 2336 |
| A4 | 2.35 | 486.15 | 1250.18 |
| A5 | 2.05 | 147.35 | 1200.12 |
| A6 | 1.95 | 299.95 | 1526.04 |
| A7 | 0.63 | 4390.1 | 374.94 |
| A8 | 0.56 | 2943.6 | 208.18 |
| A9 | 1.69 | 234.05 | 1375.42 |
| A10 | 2.07 | 233.17 | 1831.44 |
| A11 | 1.87 | 169.53 | 1591.97 |
| A12 | 2.72 | 15.96 | 339.38 |
| A18 | 0.23 | >4844.81 | 54.66 |
| A20 | 0.97 | 2433.39 | 778.62 |
| A29 | 2.35 | 223.23 | 876.82 |
| A30 | 0.94 | 1235.57 | 286.36 |

In addition, the plasma stability of these compounds was determined. Briefly, the determination method comprises the following steps:
1) Thaw the frozen mixed plasma in a 37°C water bath before testing, and then centrifuge at 4000 rpm for 5 min to remove blood clots;
2) Add 98 µL blank plasma into each well of the 96-well reaction plate;
3) In addition to the blank samples, add 2 µL of 100 µM working solution of test compound into each well;
3) Incubate the reaction plate at 37°C and start timing. Incubation times were 0, 10, 30, 60 and 120 minutes, respectively, with two replicate wells at each time point;
4) Terminate the incubation at the corresponding time point, and add 400 µL of acetonitrile solution containing 200 ng/mL tolbutamide and 200 ng/mL labetalol to precipitate proteins;
5) Shake each plate for 20 minutes, then centrifuge at 4000 rpm for 20 minutes, transfer 50 µL of supernatant to another 96-well plate, and dilute each sample with 100 µL of pure water;
6) Analyze the sample by LC-MS/MS.

The experiment results are as follows, and it can be seen from the test results that the selected compounds are easily metabolized to the target compound in human plasma.

The results are shown in the following table:

| **Compound** | **Time (min)** | **% Retained** | | **T_{1/2} (min)** | | **% Formed** | |
|---|---|---|---|---|---|---|---|
| | | **Mini Pigs** | **Humans** | **Mini Pigs** | **Humans** | **Mini Pigs** | **Humans** |
| **A13** | 0 | 100.0 | 100.0 | >289.1 | 32.5 | 0.9 | 1.1 |
| | 10 | 85.3 | 77.1 | | | 1.0 | 4.2 |
| | 30 | 91.0 | 48.2 | | | 2.4 | 16.1 |
| | 60 | 91.0 | 28.1 | | | 4.9 | 35.2 |
| | 120 | 77.6 | 7.4 | | | 11.9 | 73.3 |
| **A16** | 0 | 100.0 | 100.0 | 2.8 | 2.2 | 23.0 | 25.6 |
| | 10 | 8.3 | 4.5 | | | 59.4 | 58.9 |
| | 30 | 0.1 | 0.0 | | | 83.7 | 56.7 |
| | 60 | 0.0 | 0.0 | | | 76.7 | 83.6 |
| | 120 | 0.0 | 0.0 | | | 86.0 | 84.5 |
| **A17** | 0 | 100.0 | 100.0 | 66.8 | 25.0 | 1.6 | 1.6 |
| | 10 | **100.9** | **83.4** | | | 2.6 | 4.1 |
| | 30 | 76.0 | 50.7 | | | 9.5 | 13.6 |
| | 60 | 52.2 | 20.4 | | | 23.7 | 30.0 |
| | 120 | 30.7 | 3.8 | | | 36.2 | 44.7 |
| **A19** | 0 | 100.0 | 100.0 | 24.3 | 2.6 | 3.6 | 18.7 |
| | 10 | 73.0 | 5.1 | | | 20.9 | 61.3 |
| | 30 | 46.2 | **0.0** | | | 42.7 | 69.9 |
| | 60 | 18.5 | 0.0 | | | 70.2 | 76.3 |
| | 120 | 3.3 | 0.0 | | | 77.6 | 95.7 |
| **A22** | 0 | 100.0 | 100.0 | 6.3 | 3.1 | 12.3 | 2.1 |
| | 10 | 25.5 | 4.5 | | | 57.5 | 48.7 |
| | 30 | 1.8 | 0.1 | | | 70.8 | 81.0 |
| | 60 | 0.1 | **0.1** | | | 77.5 | 110.8 |
| | 120 | **0.1** | **0.0** | | | 98.7 | 118.3 |
| **A23** | 0 | 100.0 | 100.0 | 41.1 | 20.8 | 1.7 | 2.3 |
| | 10 | 75.1 | 60.2 | | | 11.1 | 12.2 |
| | 30 | 51.3 | 40.1 | | | 24.2 | 35.9 |
| | 60 | 39.2 | 18.5 | | | 42.2 | 72.4 |
| | 120 | 12.0 | 1.6 | | | 52.8 | 84.5 |
| **A29** | 0 | 100.0 | 100.0 | 46.4 | 16.4 | 0.4 | 0.4 |
| | 10 | 79.4 | 67.2 | | | 16.2 | 23.8 |
| | 30 | 66.8 | 32.2 | | | 42.7 | 49.4 |
| | 60 | 41.2 | 9.6 | | | 57.8 | 78.4 |
| | 120 | 16.1 | 0.6 | | | 80.9 | 107.6 |
| **A15** | 0 | 100.0 | 100.0 | 71.5 | 4.8 | 2.0 | 1.4 |
| | 10 | 88.3 | 20.6 | | | 5.9 | 38.4 |
| | 30 | 75.9 | 0.9 | | | 14.3 | 54.1 |
| | 60 | 57.6 | 0.0 | | | 26.6 | 61.7 |
| | 120 | 30.8 | 0.0 | | | 37.8 | 72.0 |
| **A18** | 0 | 100.0 | 100.0 | 9.1 | 1.9 | 1.1 | 0.6 |
| | 10 | 57.3 | 2.6 | | | 26.9 | 40.9 |
| | 30 | 13.5 | 0.0 | | | 50.1 | 48.7 |
| | 60 | 1.3 | 0.0 | | | 64.3 | 54.0 |
| | 120 | 0.0 | 0.0 | | | 64.9 | 66.7 |

### An Experiment to Verify the Prevention of Rash Caused by Small Molecule EGFR Inhibitors with Topical Administration of Compounds of the Present Application on a Rat Animal Model

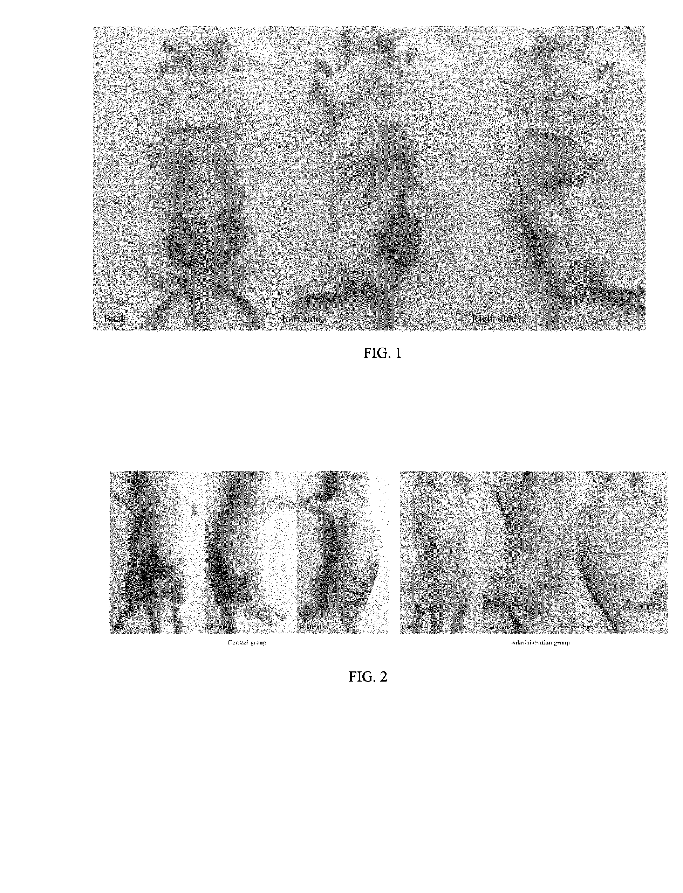

A rat animal model was established. After daily gavage of a small molecule EGFR inhibitor to female SD rats for 6 weeks, the rats developed extensive rashes on their backs several days later (picture shown in FIG. 1). No left-right difference was observed in the location of the rash, with a similar degree of rash on both sides. Similar to humans, rats developed skin rashes on their faces and bodies after oral administration of small molecule EGFR inhibitors. Both of them had the same etiology and very similar symptoms. Therefore, rats are a proper animal model for mimicking EGFR inhibitor-induced rash.

After the SD rats were fed and adapted for one week (about 200 g), the rats were divided into 10 rats per group. The hair on the back of rats was gently removed with an electric razor one day before the experiment, and then an intragastric administration test was carried out. EGFR inhibitors were dissolved in a sterile aqueous solution, and diluted with PBS buffer; the amount of each gavage was not more than 2 mL, and the dosage was shown in Table 1. The experiment comprised the administration group and the control group. After intragastric administration, apply the ointment (type and concentration are shown in Table 1) of the compound used in the present application to the backs (about 3 cm*3 cm) of rats in the administration group; apply blank matrix ointment (about 0.5 g) to the backs (about 3cm*3cm) of rats in the control group; fix the rats with a fixed cylinder for about 4 hours after drug application, release the rats after 4 hours, wipe off the residual drug at the application site with clean water, and put them back into the cage. The frequency of intragastric administration of EGFR inhibitors is shown in Table 1, but the compounds of the present application and blank matrix ointment are applied only once a day. The intragastric administration and application test were repeated daily until an obvious rash appeared in the control group. At this time, the number of rats with normal skin or significantly milder rash than that in the control group was calculated as the number of rats with effective inhibition of rashes.

Table 1 lists the animal experiment combination of various small molecule EGFR inhibitors and the compound ointment of the present application, as well as the corresponding experiment results (wherein, the value in the control rate column = the number of rats whose rash is milder in the administration group than that in the control group/the total number of rats in the administration group × 100%).

**Table 1: Experiment Conditions and Results of Examples 1-24**

| Exampl es | Inhibitor | Type of Inhibitor | Dose | Frequency | Drug Application | Concentration (wt%) | Days | Control Rate |
|---|---|---|---|---|---|---|---|---|
| 1 | Afatinib | Second-generation EGFR Small Molecule Inhibitor | 40 mg/kg | Once a Day | A7 Ointment | 0.1% | 14 | 20% |
| | | | | | | 0.3% | | 50% |
| | | | | | | 1.0% | | 70% |
| | | | | | | 3.0% | | 75% |
| | | | | | | 5.0% | | 80% |
| | | | | | | 10.0% | | 60% |
| 2 | Gefitinib | First-generation EGFR Small Molecule Inhibitor | 80 mg/kg | Twice a Day | A7 Ointment | 0.3% | 15 | 10% |
| | | | | | | 1.0% | | 50% |
| | | | | | | 3.0% | | 60% |
| | | | | | | 5.0% | | 60% |
| 3 | Dacomitinib | Second-generation EGFR Small Molecule Inhibitor | 10 mg/kg | Once a Day | A7 Ointment | 1.0% | 12 | 40% |
| | | | | | | 5.0% | | 80% |
| 4 | Osimertinib | Third-generation EGFR Small Molecule Inhibitor | 60 mg/kg | Twice a Day | A7 Ointment | 0.3% | 10 | 20% |
| | | | | | | 5.0% | | 70% |
| 5 | EAI045 | Fourth-generation EGFR Small Molecule Inhibitor | 80 mg/kg | Once a Day | A7 Ointment | 1.0% | 11 | 30% |
| | | | | | | 10.0% | | 60% |
| 6 | Erlotinib | First-generation EGFR Small Molecule Inhibitor | 70 mg/kg | Once a Day | A12 Ointment | 0.3% | 14 | 20% |
| | | | | | | 3.0% | | 50% |
| 7 | Afatinib | Second-generation EGFR Small Molecule Inhibitor | 40 mg/kg | Once a Day | A12 Ointment | 1.0% | 14 | 60% |
| | | | | | | 3.0% | | 75% |
| | | | | | | 5.0% | | 90% |
| 8 | Osimertinib | Third-generation EGFR Small Molecule Inhibitor | 60 mg/kg | Twice a Day | A12 Ointment | 0.3% | 10 | 50% |
| | | | | | | 5.0% | | 80% |
| 9 | EAI045 | Fourth-generation EGFR Small Molecule Inhibitor | 80 mg/kg | Once a Day | A12 Ointment | 1.0% | 11 | 60% |
| | | | | | | 10.0% | | 70% |
| 10 | Dacomitinib | Second-generation EGFR Small Molecule Inhibitor | 10 mg/kg | Once a Day | A14 Ointment | 1.0% | 12 | 50% |
| | | | | | | 5.0% | | 65% |
| 11 | Osimertinib | Third-generation EGFR Small Molecule Inhibitor | 60 mg/kg | Twice a Day | A14 Ointment | 0.3% | 10 | 30% |
| | | | | | | 10.0% | | 80% |
| 12 | Zorifertinib | EGFR Small Molecule Inhibitor | 60 mg/kg | Once a Day | A14 Ointment | 3%% | 14 | 50% |
| 13 | BPI-7711 | EGFR Small Molecule Inhibitor | 60 mg/kg | Once a Day | A14 Ointment | 5%% | 12 | 70% |
| 14 | Erlotinib | First-generation EGFR Small Molecule Inhibitor | 70 mg/kg | Once a Day | A16 Ointment | 0.3% | 14 | 30% |
| | | | | | | 3.0% | | 50% |
| 15 | Afatinib | Second-generation EGFR Small Molecule Inhibitor | 40 mg/kg | Once a Day | A16 Ointment | 1.0% | 14 | 60% |
| | | | | | | 5.0% | | 80% |
| | | | | | | 10.0% | | 100% |
| 16 | Zorifertinib | EGFR Small Molecule Inhibitor | 60 mg/kg | Once a Day | A16 Ointment | 1.0% | 14 | 30% |
| 17 | BPI-7711 | EGFR Small Molecule Inhibitor | 60 mg/kg | Once a Day | A16 Ointment | 1.0% | 12 | 50% |
| 18 | EAI045 | Fourth-generation EGFR Small Molecule Inhibitor | 80 mg/kg | Once a Day | A18 Ointment | 0.3% | 11 | 30% |
| | | | | | | 3.0% | | 70% |
| | | | | | | 5.0% | | 90% |
| 19 | Zorifertinib | EGFR Small Molecule Inhibitor | 60 mg/kg | Once a Day | A18 Ointment | 3.0% | 12 | 60% |
| 20 | BPI-7711 | EGFR Small Molecule Inhibitor | 60 mg/kg | Once a Day | A18 Ointment | 3.0% | 14 | 70% |
| 21 | Osimertinib | Third-generation EGFR Small Molecule Inhibitor | 60 mg/kg | Twice a Day | A39 Ointment | 0.1% | 10 | 10% |
| | | | | | | 3.0% | | 30% |
| 22 | EAI045 | Fourth-generation EGFR Small Molecule Inhibitor | 80 mg/kg | Once a Day | A39 Ointment | 1.0% | 11 | 20% |
| | | | | | | 5.0% | | 65% |
| 23 | Zorifertinib | EGFR Small Molecule Inhibitor | 60 mg/kg | Once a Day | A39 Ointment | 5.0% | 14 | 40% |
| 24 | BPI-7711 | EGFR Small Molecule Inhibitor | 60 mg/kg | Once a Day | A39 Ointment | 5.0% | 12 | 90% |

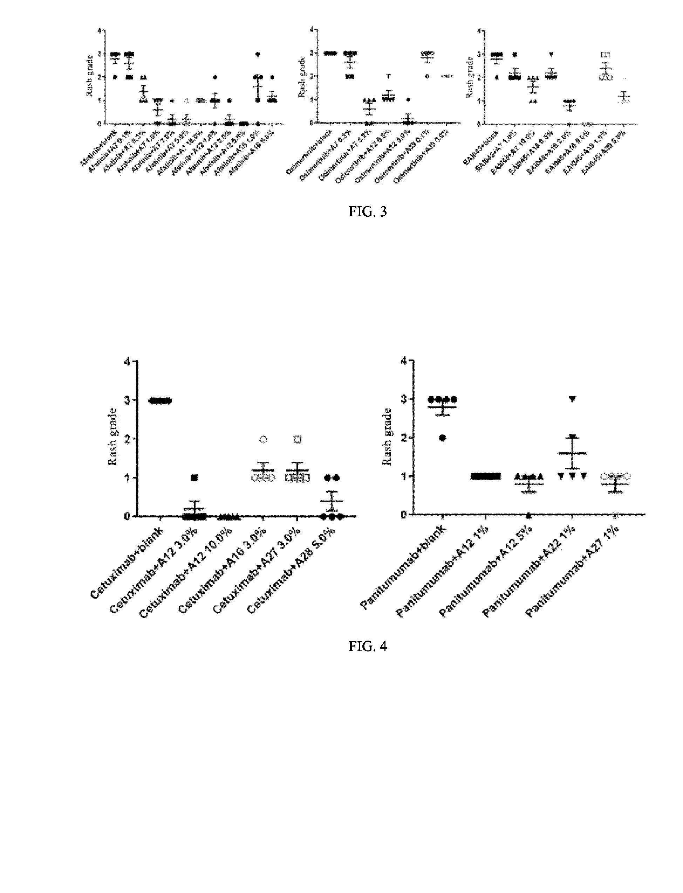

FIG. 2 shows photographs of the left side, back and right side of typical rats in the control group and the administration group in Table 1. FIG. 3 shows the rash grades of the administration group and the control group at the end of the experiment.

The results in Table 1 and FIGs. 2-3 show that the compound ointment of the present application is effective for preventing rashes caused by small molecule EGFR inhibitors.

### An Experiment to Verify the Prevention of Rash Caused by Monoclonal EGFR Inhibitors with Topical Administration of Compounds of the Present Application on a Rat Animal Model

After the SD rats were fed and adapted for one week (about 200 g), the rats were divided into 10 rats per group. The hair on the back of rats was gently removed with an electric razor one day before the experiment, and then an administration test was carried out. The experiments were divided into the the administration group with the compound of the present application and the control group. The EGFR monoclonal antibody solution diluted with normal saline was injected via the tail vein twice a week, and the injection rate and time are shown in Table 2. After injection, the administration group applied the compound ointment of the present application on the backs (about 3 cm*3 cm) of rats every day, and the control group applied blank matrix ointment (about 0.5 g) to the backs (about 3 cm*3 cm) of rats every day. After drug application, the rats were fixed with a fixed cyclinder for 4 h; after that, the rats were released and the residual drug at the application site was wiped off with clear water. Monoclonal antibody EGFR inhibitors were injected via the tail vein twice a week, and the compound of the present application and blank ointment were applied once a day until obvious rashes appeared in the control group. After 10-14 days of drug application, the number of rats with normal skin or significantly milder rash in the administration group than that in the control group was counted as the number of rats with effective inhibition of rashes.

Table 2 lists the animal experiment combinations of various monoclonal antibody EGFR inhibitors and the compound ointment of the present application, as well as the corresponding experiment results (wherein, the value in the control rate column = the number of rats whose rash is milder in the administration group than that in the control group/the total number of rats in the administration group × 100%).

**Table 2: Experiment Conditions and Results of Examples 25-34**

| Examples | EGFR Inhibitor | Dose | Frequency | Drug Application | Concentration (wt%) | Days | Control Rate |
|---|---|---|---|---|---|---|---|
| 25 | Cetuximab | 100 mg/kg, 15 min at 1.3 mL/kg/min per injection | Twice a week via tail vein injection | A12 Ointment | 0.3% | 10 | 20% |
| | | | | | 3.0% | | 70% |
| | | | | | 10.0% | | 85% |
| 26 | Panitumumab | 10mg/kg | Twice a week via tail vein injection | A12 Ointment | 1.0% | 14 | 50% |
| | | | | | 5.0% | | 60% |
| | | | | | 10.0% | | 60% |
| 27 | Cetuximab | 100 mg/kg, 15 min at 1.3 mL/kg/min per injection | Twice a week via tail vein injection | A16 Ointment | 0.3% | 10 | 10% |
| | | | | | 3.0% | | 50% |
| | | | | | 10.0% | | 70% |
| 28 | Panitumumab | 10mg/kg | Twice a week via tail vein injection | A16 Ointment | 1.0% | 14 | 50% |
| | | | | | 5.0% | | 80% |
| | | | | | 10.0% | | 60% |
| 29 | Cetuximab | 100 mg/kg, 15 min at 1.3 mL/kg/min per injection | Twice a week via tail vein injection | A22 Ointment | 0.3% | 10 | 20% |
| | | | | | 5.0% | | 40% |
| 30 | Panitumumab | 10mg/kg | Twice a week via tail vein injection | A22 Ointment | 1.0% | 14 | 30% |
| | | | | | 10.0% | | 65% |
| 31 | Cetuximab | 100 mg/kg, 15 min at 1.3 mL/kg/min per injection | Twice a week via tail vein injection | A27 Ointment | 0.2% | 10 | 20% |
| | | | | | 3.0% | | 40% |
| 32 | Panitumumab | 10mg/kg | Twice a week via tail vein injection | A27 Ointment | 1.0% | 14 | 45% |
| | | | | | 5.0% | | 70% |
| 33 | Cetuximab | 100 mg/kg, 15 min at 1.3 mL/kg/min per injection | Twice a week via tail vein injection | A28 Ointment | 0.2% | 10 | 30% |
| | | | | | 5.0% | | 70% |
| 34 | Panitumumab | 10mg/kg | Twice a week via tail vein injection | A28 Ointment | 0.2% | 14 | 45% |
| | | | | | 5.0% | | 100% |

FIG. 4 shows the rash grades of the administration group and the control group at the end of the experiment (monoclonal EGFR inhibitor).

The results in Table 2 and FIG. 4 show that the compound ointment of the present application is effective in preventing rashes caused by monoclonal antibody EGFR inhibitors.

### An Experiment to Verify the Treatment of Rash Caused by Small Molecule EGFR Inhibitors with Topical Administration of Compounds of the Present Application on a Rat Animal Model

After the SD rats were fed and adapted for one week (about 200 g), the rats were divided into 10 rats per group. The hair on the back of rats was gently removed with an electric razor one day before the experiment, and then an intragastric administration test was carried out. EGFR inhibitors were dissolved in a sterile aqueous solution, and diluted with PBS buffer; the amount of each gavage was not more than 2 mL, and the dosage was shown in Table 3. The gavage continued every day until the rats showed symptoms of skin rash, at which time the treatment experiment began. The experiments were divided into the administration group with the compound of the present application and the control group. During the treatment experiment, EGFR inhibitors were intragastrically administered continuously every day. After the gavage, the administration group applied the compound ointment of the present application on the back (about 3 cm*3 cm) of rats, and the control group applied blank matrix ointment on the back (about 3 cm*3 cm); after application, the rats were fixed with a fixed cyclinder for about 4 hours, and then the rats were released, with the residual drug at the application site wiped off with clean water, and put back into the cage. The frequency of intragastric administration of EGFR inhibitors is shown in Table 3, but the compounds of the present application and blank ointment are applied only once a day. The EGFR inhibitor was repeatedly intragastrically administered daily, and the number of rats whose skin rash in the administration group returned to normal or significantly milder than that in the control group at the endpoint of the experiment was counted as the number of rats with effective treatment of rashes.

Table 3 lists the animal experiment combinations of various small molecule EGFR inhibitors and the compound ointment of the present application, as well as the corresponding experiment results (wherein, the value in the control rate column = the number of rats whose rash is milder in the administration group than that in the control group/the total number of rats in the administration group × 100%).

**Table 3: Experiment Conditions and Results of Examples 35-58**

| Examples | EGFR Inhibitor | Type of Inhibitor | Dose | Frequency | Dosing Modelling Days | Drug Application | Concentration (wt%) | Control Rate |
|---|---|---|---|---|---|---|---|---|
| 35 | Afatinib | Second-generation EGFR Small Molecule Inhibitor | 40mg/ kg | Once a Day | 6 | A31 Ointment | 0.1% | 10% |
| | | | | | | | 1.0% | 60% |
| | | | | | | | 3.0% | 70% |
| | | | | | | | 5.0% | 90% |
| | | | | | | | 10.0% | 90% |
| 36 | Gefitinib | First-generation EGFR Small Molecule Inhibitor | 80mg/ kg | Twice a Day | 10 | A31 Ointment | 0.3% | 20% |
| | | | | | | | 3.0% | 40% |
| | | | | | | | 10.0% | 90% |
| 37 | Osimertin ib | Third-generation EGFR Small Molecule Inhibitor | 60mg/ kg | Twice a Day | 7 | A31 Ointment | 0.5% | 40% |
| | | | | | | | 1.0% | 60% |
| | | | | | | | 5.0% | 70% |
| 38 | EAI045 | Fourth-generation EGFR Small Molecule Inhibitor | 80mg/ kg | Once a Day | 8 | A31 Ointment | 0.1% | 20% |
| | | | | | | | 5.0% | 70% |
| | | | | | | | 10.0% | 90% |
| 39 | Afatinib | Second-generation EGFR Small Molecule Inhibitor | 40mg/ kg | Once a Day | 6 | A36 Ointment | 0.5% | 20% |
| | | | | | | | 5.0% | 60% |
| 40 | Osimertin ib | Third-generation EGFR Small Molecule Inhibitor | 60mg/ kg | Twice a Day | 7 | A36 Ointment | 1.0% | 30% |
| | | | | | | | 10.0% | 50% |
| 41 | EAI045 | Fourth-generation EGFR Small Molecule Inhibitor | 80mg/ kg | Once a Day | 8 | A36 Ointment | 0.5% | 20% |
| | | | | | | | 5.0% | 40% |
| 42 | Larotinib | Fourth-generation EGFR Small Molecule Inhibitor | 20mg/ kg | Once a Day | 6 | A36 Ointment | 5.0% | 60% |
| 43 | BPI-7711 | EGFR Small Molecule Inhibitor | 60mg/ kg | Once a Day | 6 | A36 Ointment | 5.0% | 30% |
| 44 | Dacomiti nib | Second-generation EGFR Small Molecule Inhibitor | 10mg/ kg | Once a Day | 5 | A28 Ointment | 0.2% | 30% |
| | | | | | | | 2.0% | 60% |
| | | | | | | | 5.0% | 80% |
| | | | | | | | 10.0% | 60% |
| 45 | Gefitinib | First-generation EGFR Small Molecule Inhibitor | 80mg/ kg | Twice a Day | 10 | A28 Ointment | 1.0% | 30% |
| | | | | | | | 5.0% | 30% |
| 46 | Osimertin ib | Third-generation EGFR Small Molecule Inhibitor | 60mg/ kg | Twice a Day | 7 | A28 Ointment | 0.5% | 20% |
| | | | | | | | 5.0% | 60% |
| 47 | EAI045 | Fourth-generation EGFR Small Molecule Inhibitor | 80mg/ kg | Once a Day | 8 | A28 Ointment | 1.0% | 40% |
| | | | | | | | 10.0% | 100% |
| 48 | Larotinib | Fourth-generation EGFR Small Molecule Inhibitor | 20mg/ kg | Once a Day | 6 | A28 Ointment | 3.0% | 40% |
| 49 | BPI-7711 | EGFR Small Molecule Inhibitor | 60mg/ kg | Once a Day | 6 | A28 Ointment | 3.0% | 80% |
| 50 | Afatinib | Second-generation EGFR Small Molecule Inhibitor | 40mg/ kg | Once a Day | 6 | A7 Ointment | 0.1% | 20% |
| | | | | | | | 3.0% | 60% |
| 51 | Osimertin ib | Third-generation EGFR Small Molecule Inhibitor | 60mg/ kg | Twice a Day | 7 | A7 Ointment | 3.0% | 10% |
| | | | | | | | 10.0% | 70% |
| 52 | EAI045 | Fourth-generation EGFR Small Molecule Inhibitor | 80mg/ kg | Once a Day | 8 | A7 Ointment | 1.0% | 50% |
| | | | | | | | 5.0% | 80% |
| 53 | Larotinib | Fourth-generation EGFR Small Molecule Inhibitor | 20mg/ kg | Once a Day | 6 | A7 Ointment | 3.0% | 6% |
| 54 | BPI-7711 | EGFR Small Molecule Inhibitor | 60mg/ kg | Once a Day | 6 | A7 Ointment | 3.0% | 70% |
| 55 | Osimertin ib | Third-generation EGFR Small Molecule Inhibitor | 60mg/ kg | Twice a Day | 7 | A27 Ointment | 0.2% | 40% |
| | | | | | | | 4.0% | 100% |
| 56 | EAI045 | Fourth-generation EGFR Small Molecule Inhibitor | 80mg/ kg | Once a Day | 8 | A27 Ointment | 1.0% | 60% |
| | | | | | | | 3.0% | 80% |
| 57 | Osimertin ib | Third-generation EGFR Small Molecule Inhibitor | 60mg/ kg | Twice a Day | 7 | A33 Ointment | 3.0% | 30% |
| | | | | | | | 10.0% | 70% |
| 58 | EAI045 | Fourth-generation EGFR Small Molecule Inhibitor | 80mg/ kg | Once a Day | 8 | A33 Ointment | 1.0% | 30% |
| | | | | | | | 5.0% | 60% |

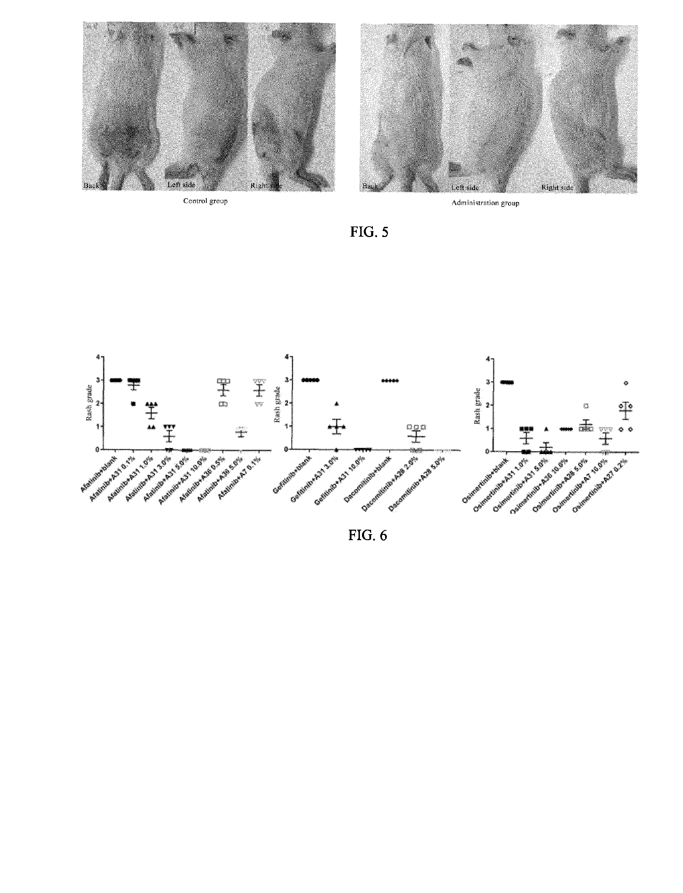

FIG. 5 shows the photographs of the left side, back and right side of typical rats in the control group and the administration group in Table 3. FIG. 6 shows the rash grades of the administration group and the control group at the end of the experiment.

The results in Table 3 and FIG. 6 show that the compound ointment of the present application is effective for treating rashes caused by small molecule EGFR inhibitors.

### An Experiment to Verify the Treatment of Rash Caused by Monoclonal EGFR Inhibitors with Topical Administration of Compounds of the Present Application on a Rat Animal Model

After the SD rats were fed and adapted for one week (about 200 g), the rats were divided into 10 rats per group. The hair on the back of rats was gently removed with an electric razor one day before the experiment, and then an administration test was carried out. The EGFR monoclonal antibody solution diluted with normal saline was injected via tail vein twice a week, and the injection rate and time were shown in Table 4; the treatment experiment was started at the time point when rats developed a rash after continuous administration for 1-2 weeks. The experiments were divided into the administration group with the compound of the present application and the control group. During the treatment experiment, monoclonal antibody EGFR inhibitors were injected twice a week, the administration group applied the compound ointment of the present application on the back (about 3 cm*3 cm) of rats every day, and the control group applied blank matrix ointment on the back (about 3 cm*3 cm); after application, the rats were fixed with a fixed cyclinder for about 4 hours, and then the rats were released, with the residual drug at the application site wiped off with clean water, and put back into the cage. After 10 days of drug application, the number of rats with normal skin or significantly milder rash in the administration group than that in the control group was counted as the number of rats with effective inhibition of rashes.

Table 4 lists the animal experiment combinations of monoclonal antibody EGFR inhibitors and the compound ointment of the present application, as well as the corresponding experiment results (wherein, the value in the control rate column = the number of rats whose rash is milder in the administration group than that in the control group/the total number of rats in the administration group × 100%).

**Table 4: Experiment Conditions and Results of Examples 59-68**

| Exampl es | EGFR Inhibitor | Dose | Frequency | Modeling Days | Drug Application | Concentration (wt%) | Days of Drug Application | Control Rate |
|---|---|---|---|---|---|---|---|---|
| 59 | Cetuximab | 100 mg/kg, at 1.3 mL/kg/min per injection | Twice a week via tail vein injection | 10 (3-4 times) | A33 Ointment | 0.1% | 10 | 20% |
| | | | | | | 1.0% | | 50% |
| | | | | | | 5.0% | | 80% |
| | | | | | | 10.0% | | 100% |
| 60 | Panitumum ab | 6mg/kg,90 min | Twice a week via tail vein injection | 14 (4 times) | A33 Ointment | 0.1% | 8 | 30% |
| | | | | | | 1.0% | | 70% |
| | | | | | | 5.0% | | 70% |
| | | | | | | 10.0% | | 100% |
| 61 | Cetuximab | 100 mg/kg, at 1.3 mL/kg/min per injection | Twice a week via tail vein injection | 10 (3-4 times) | A39 Ointment | 0.1% | 10 | 20% |
| | | | | | | 1.0% | | 40% |
| | | | | | | 5.0% | | 80% |
| | | | | | | 10.0% | | 100% |
| 62 | Panitumum ab | 6mg/kg,90 min | Twice a week via tail vein injection | 14 (4 times) | A39 Ointment | 0.1% | 8 | 20% |
| | | | | | | 1.0% | | 30% |
| | | | | | | 5.0% | | 60% |
| | | | | | | 10.0% | | 70% |
| 63 | Cetuximab | 102 mg/kg, at 1.3 mL/kg/min per injection | Twice a week via tail vein injection | 10 (3-4 times) | A28 Ointment | 0.3% | 10 | 40% |
| | | | | | | 5.0% | | 70% |
| 64 | Panitumum ab | 6mg/kg,90 min | Twice a week via tail vein injection | 14 (4 times) | A28 Ointment | 2.0% | 8 | 60% |
| | | | | | | 10.0% | | 100% |
| 65 | Cetuximab | 102 mg/kg, at 1.3 mL/kg/min per injection | Twice a week via tail vein injection | 10 (3-4 times) | A27 Ointment | 1.0% | 10 | 30% |
| | | | | | | 5.0% | | 80% |
| 66 | Panitumum ab | 6mg/kg,90 min | Twice a week via tail vein injection | 14 (4 times) | A27 Ointment | 0.1% | 8 | 60% |
| | | | | | | 3.0% | | 90% |
| 67 | Cetuximab | 102 mg/kg, at 1.3 mL/kg/min per injection | Twice a week via tail vein injection | 10 (3-4 times) | A14 Ointment | 1.0% | 10 | 20% |
| | | | | | | 5.0% | | 60% |
| 68 | Panitumum ab | 6mg/kg,90 min | Twice a week via tail vein injection | 14 (4 times) | A14 Ointment | 0.1% | 8 | 40% |
| | | | | | | 3.0% | | 70% |

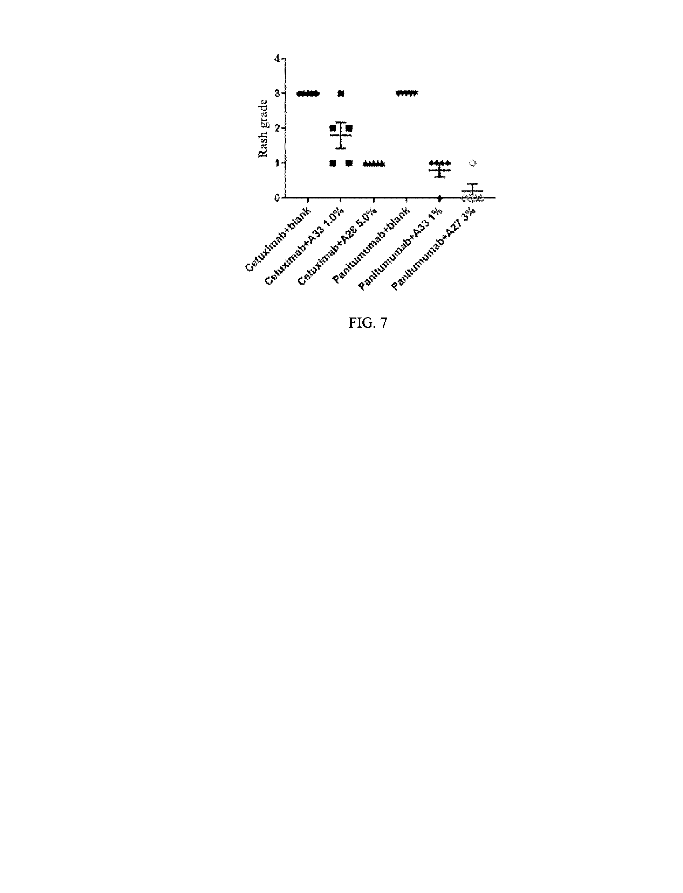

FIG. 7 shows the rash grades of the administration group and the control group at the end of the experiment (monoclonal EGFR inhibitor).

The results in Table 4 and FIG. 7 show that the compound ointment of the present application is effective for treating rashes caused by monoclonal EGFR inhibitors.

### Therapeutic Effect of Topical Administration of Compounds of the Present Application in Skin Contact Delayed-type Hypersensitivity Response Test in Mice

Delayed-type hypersensitivity (DTH) response in mouse skin is considered to be an effective model for clinical contact dermatitis and other T cell-mediated cutaneous immune diseases, such as atopic dermatitis and psoriasis. The DTH in mice shares the common features with these cutaneous immune diseases, including immunoinfiltration with an increase of inflammatory cytokines and hyperproliferation of keratinocytes.

Balb/c mice were sensitized by topical administration of the antigen 2,4-dinitrofluorobenzene (DNFB) to the shaved back of mice on Days 0 and 1. On Day 5, the thickness of the ears was measured using an engineering micrometer. The measurement result was recorded and used as a baseline. The animals were then challenged with a total of 20 µL of DNFB (10 µL for the internal pinna and 10 µL for the external pinna) topically dosed to both ears at a concentration of 0.2%. The ears were measured again 24-72 hours after challenge. The compound ointment of the present application was applied topically to the ears of the animals throughout the allergic and challenge periods (Days -1 ~ 7) or before or throughout the challenge period (generally Days 4 ~ 7). The therapeutic effect was expressed by a reduction in ear swelling compared to that of the blank ointment. The compound causing a reduction of 20% or more was considered effective.

Table 5 lists the skin contact delayed-type hypersensitivity experiment results in mice treated with the compound of the present application.

| Example No. | Compound Ointment of the Present Application | Concentration (wt%) | Reduction Rate of Ear Swelling |
|---|---|---|---|
| 69 | A7 Ointment | 0.05% | 18% |
| | | 0.1% | 22% |
| | | 1.0% | 27% |
| | | 5.0% | 35% |
| | | 10.0% | 42% |
| 70 | A16 Ointment | 0.1% | 25% |
| | | 1.0% | 35% |
| | | 5.0% | 39% |
| | | 10.0% | 42% |
| 71 | A18 Ointment | 0.1% | 23% |
| | | 1.0% | 29% |
| | | 5.0% | 37% |
| | | 10.0% | 45% |
| 72 | A31 Ointment | 1.0% | 16% |
| | | 5.0% | 28% |
| 73 | A36 Ointment | 1.0% | 29% |
| | | 5.0% | 34% |
| 74 | A33 Ointment | 1.0% | 22% |
| | | 5.0% | 32% |

The results in Table 5 that the compound ointment of the present application is effective for treating skin contact delayed-type hypersensitivity in mice.

Formalin-fixed and paraffin-embedded ear tissues of mice were prepared to obtain ear sections, and immunohistochemical analysis showed that the compound ointment of the present application reduced the number of infiltrating cells in the tissue.

## Claims

1. A compound of formula (I), or a pharmaceutically acceptable salt, bioactive metabolite, solvate, hydrate, prodrug, racemate, enantiomer or stereoisomer thereof, 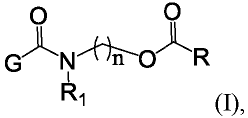 wherein:
G is a JAK inhibitor,
R₁ and R are independently selected from: hydrogen, deuterium, tritium, optionally substituted alkyl, optionally substituted deuterated alkyl, optionally substituted halogenated alkyl, optionally substituted alkoxy, optionally substituted halogenated alkoxy, halogen and optionally substituted cyclic group; and
n is 1, 2, 3, 4 or 5.

2. The compound of claim 1, wherein R₁ and R are independently selected from: -(CH₂)ₙ₁Ra, -(CH₂)ₙ₁ORa, -(CH₂)ₙ₁SRa, -(CH₂)ₙ₁C(O)Ra, -(CH₂)ₙ₁C(O)ORa, -(CH₂)ₙ₁S(O)ₘ₁Ra, -(CH₂)ₙ₁NRaRb, -(CH₂)ₙ₁C(O)NRaRb, -(CH₂)ₙ₁NRaC(O)Rb and -(CH₂)ₙ₁NRaS(O)ₘ₁Rb; wherein:
Ra and Rb can be the same or different, and each is independently selected from: hydrogen, deuterium, tritium, alkyl, deuterated alkyl, halogenated alkyl, alkoxy, hydroxyalkyl, halogenated alkoxy, halogen, cyano, nitro, hydroxy, amino, alkenyl, alkynyl, cycloalkyl, heterocyclyl, aryl, and heteroaryl; wherein alkyl, deuterated alkyl, halogenated alkyl, alkoxy, hydroxyalkyl, halogenated alkoxy, alkenyl, alkynyl, cycloalkyl, heterocyclyl, aryl, and heteroaryl are optionally further substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
or, the Ra is connected with Rb to form a cycloalkyl, heterocyclyl, aryl or heteroaryl group, wherein cycloalkyl, heterocyclyl, aryl and heteroaryl groups are optionally substituted with one or more groups selected from the group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
n1 is 0, 1, 2, 3, 4 or 5; and
m1 is 0, 1 or 2.

3. The compound of any one of claims 1 to 2, wherein the cyclic group is selected from: cycloalkyl, heterocyclyl, oxoheterocyclyl, thioheterocyclyl, aryl and heteroaryl; wherein cycloalkyl, heterocyclyl, oxoheterocyclyl, thioheterocyclyl, aryl and heteroaryl are optionally substituted with one or more groups selected from the following group: hydrogen, deuterium, substituted or unsubstituted alkyl, halogen, hydroxy, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl.

4. The compound of any one of claims 1 to 3, or a pharmaceutically acceptable salt, bioactive metabolite, solvate, hydrate, prodrug, racemate, enantiomer or stereoisomer thereof, wherein R₁ is hydrogen, optionally substituted alkyl or optionally substituted deuterated alkyl.

5. The compound of any one of claims 1 to 4, or a pharmaceutically acceptable salt, bioactive metabolite, solvate, hydrate, prodrug, racemate, enantiomer or stereoisomer thereof, wherein R₁ is hydrogen, optionally substituted C₁-C₆ alkyl or optionally substituted C₁-C₆ deuterated alkyl.

6. The compound of any one of claims 1 to 5, or a pharmaceutically acceptable salt, bioactive metabolite, solvate, hydrate, prodrug, racemate, enantiomer or stereoisomer thereof, wherein R₁ is hydrogen, optionally substituted methyl or optionally substituted deuterated methyl.

7. The compound of any one of claims 1 to 6, or a pharmaceutically acceptable salt, bioactive metabolite, solvate, hydrate, prodrug, racemate, enantiomer or stereoisomer thereof, wherein R is selected from the group: optionally substituted alkyl, optionally substituted deuterated alkyl, optionally substituted cycloalkyl, optionally substituted heterocycloalkyl, optionally substituted aryl and optionally substituted heteroaryl.

8. The compound of any one of claims 1 to 7, or a pharmaceutically acceptable salt, bioactive metabolite, solvate, hydrate, prodrug, racemate, enantiomer or stereoisomer thereof, wherein R is selected from the group: optionally substituted C₁-C₂₀ alkyl, optionally substituted C₁-C₂₀ deuterated alkyl, optionally substituted C₃-C₂₀ cycloalkyl, optionally substituted C₁-C₂₀ heterocycloalkyl, optionally substituted C₆-C₂₀ aryl and optionally substituted C₁-C₂₀ heteroaryl.

9. The compound of any one of claims 1 to 8, or a pharmaceutically acceptable salt, bioactive metabolite, solvate, hydrate, prodrug, racemate, enantiomer or stereoisomer thereof, wherein n is 2.

10. The compound of any one of claims 1 to 9, or a pharmaceutically acceptable salt, bioactive metabolite, solvate, hydrate, prodrug, racemate, enantiomer or stereoisomer thereof, wherein:
R₁ is hydrogen, optionally substituted methyl or optionally substituted deuterated methyl,
n is 2, and
R is optionally substituted C₃-C₂₀ cycloalkyl or optionally substituted C₁-C₂₀ heterocycloalkyl.

11. The compound of any one of claims 1 to 10, or a pharmaceutically acceptable salt, bioactive metabolite, solvate, hydrate, prodrug, racemate, enantiomer or stereoisomer thereof, wherein G is a JAK inhibitor selected from the group: Ruxolitinib, Tofacitinib, Oclacitinib, Fedratinib, Peficitinib, Upadacitinib, Barictinib, Fligotinib, Decernotinib, Cerdulatinib, Lestaurtinib, Pacritinib, Momelotinib, Gandotinib, Abrocitinib, Solcitinib, SHR-0203, Itacitinib, PF-06651600, BMS-986165, Abrocitinib, Cucurbitacin I, CHZ868, TD-1473, Zotiraciclib, Alkotinib, Jaktinib, AZD-4205, DTRMHS-07, KL130008, WXSH-0150, TQ05105, WXFL10203614, GLPG0634, CEP-33779, R-348, Itacitinib, Ritlecitinib, Brepocitini, Tasocitinib, Deucravacitinib, INCB-039110, Izencitinib, Entrectinib, Ivarmacitinib, Deuruxolitinib, Adelatinib, NDI-034858, Nezulcitinib, ATI-01777, TD-8236, INCB-054707, Ropsacitinib, AGA-201, ATI50001, Gusacitinib, Cerdulatinib, Roniciclib, AT-9283, FMX-114, OST-122, TT-00420, Repotrectinib, INCB-052793, CT-340, BMS-911543, Ilginatinib, BGB-23339, ICP-332, ESK-001, SYHX-1901, VTX-958, TLL-018, CEE-321, CJ-15314, TD-5202, ABBV-712, GLPG-3667, CPL-116, AZD-4604, TAS-8274, MAX-40279, TD-3504, KN-002, AZD-0449, R-548, AC-410, Spebrutinib, ONX-0805, AEG-41174, XL-019, CR-4, WP-1066, GDC-0214, INCB-047986, PF-06263276, R-333, AZD-1480, Tozasertib, CS-12192 and AC-1101.

12. The compound of any one of claims 1 to 11, or a pharmaceutically acceptable salt, bioactive metabolite, solvate, hydrate, prodrug, racemate, enantiomer or stereoisomer thereof, wherein G is a JAK inhibitor selected from the group: Tofacitinib, Ruxolitinib, Baricitinib, Peficitinib, Pacritinib, Delgocitinib, Pf-04965842, Upadacitinib, Filgotinib, Itacitinib, Fedratinib, Decernotinib, SHR-0302, ASN-002, Cerdulatinib, BMS-986165, PF-06700841, INCB-52793, ATI-502, PF-06651600, AZD-4205, Deuterium Modified Ruxolitinib Analog, ATI-501, R-348, NS-018, Jaktinib Hydrochloride and KL-130008.

13. A compound or a pharmaceutically acceptable salt, bioactive metabolite, solvate, hydrate, prodrug, racemate, enantiomer or stereoisomer thereof, and the compound is selected from the group:

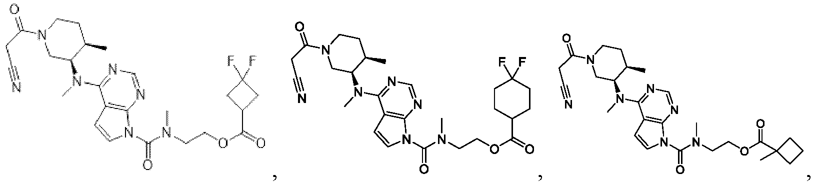

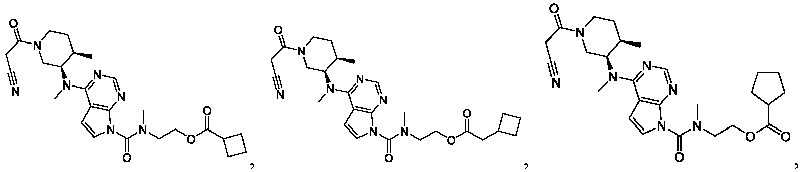

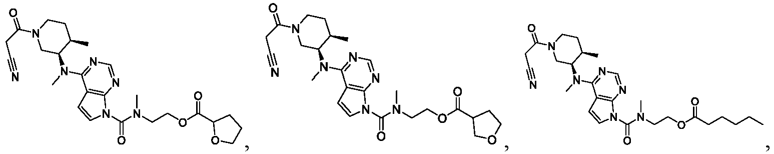

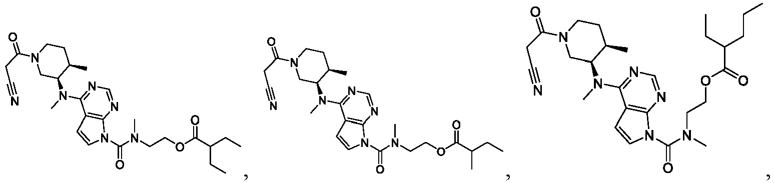

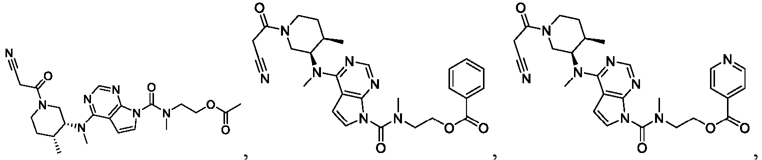

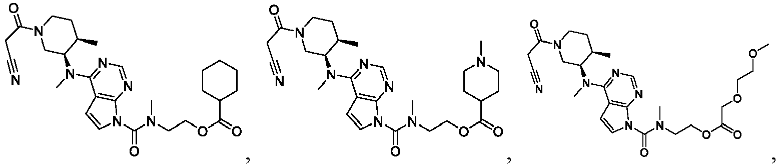

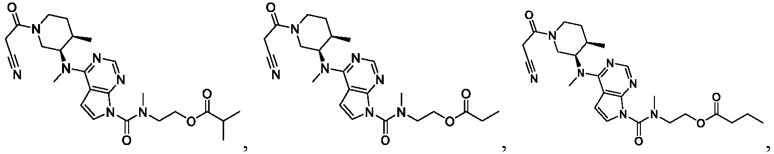

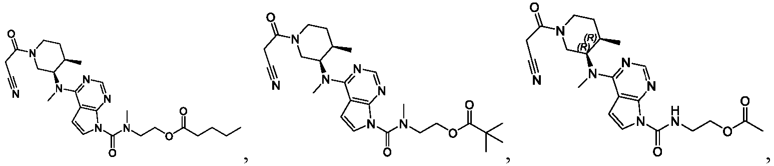

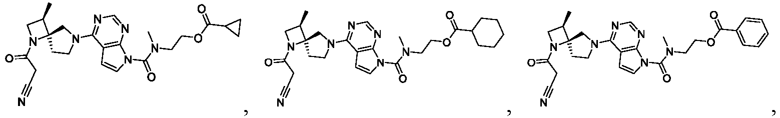

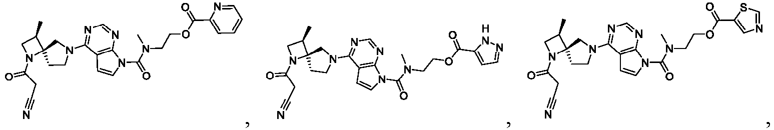

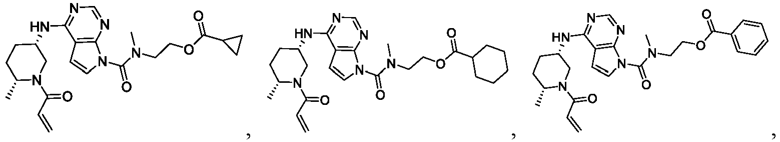

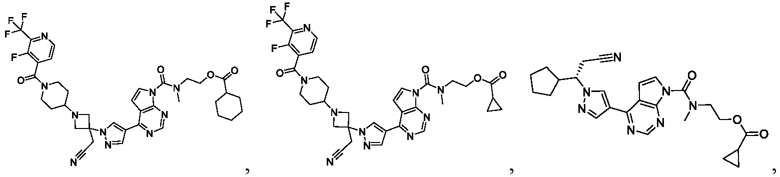

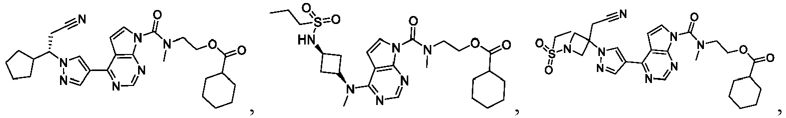

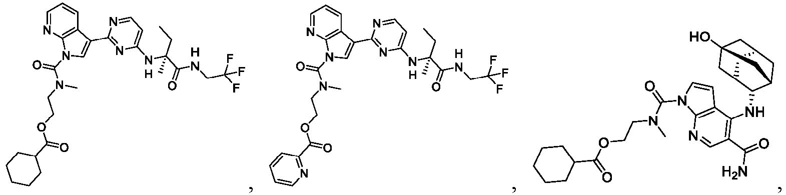

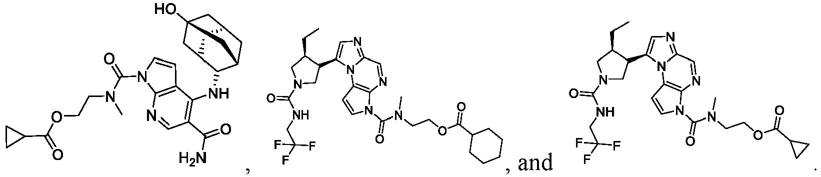

14. A preparation method of the compound of any one of claims 1 to 13, or a pharmaceutically acceptable salt, bioactive metabolite, solvate, hydrate, prodrug, racemate, enantiomer or stereoisomer thereof.

15. A composition comprising the compound of any one of claims 1 to 13, or a pharmaceutically acceptable salt, bioactive metabolite, solvate, hydrate, prodrug, racemate, enantiomer or stereoisomer.

16. The composition of claim 15 is a pharmaceutical composition and comprises a pharmaceutically acceptable carrier.

17. The composition of any one of claims 15 to 16, wherein the concentration of the compound, or a pharmaceutically acceptable salt, bioactive metabolite, solvate, hydrate, prodrug, racemate, enantiomer or stereoisomer of the compound is from about 0.001% w/w to about 40% w/w.

18. The composition of any one of claims 15 to 17, is a formulation suitable for topical application.

19. The composition of any one of claims 15 to 18, is an ointment.

20. The compound of any one of claims 1 to 13, or a pharmaceutically acceptable salt, bioactive metabolite, solvate, hydrate, prodrug, racemate, enantiomer or stereoisomer thereof; or the composition of any one of claims 15 to 19 for preventing, alleviating and/or treating a disease or disorder associated with the use of an antitumor agent in a subject.

21. The compound of any one of claims 1 to 13, or a pharmaceutically acceptable salt, bioactive metabolite, solvate, hydrate, prodrug, racemate, enantiomer or stereoisomer thereof; or the composition of any one of claims 15 to 19 for preparing a medicament for preventing, alleviating and/or treating a disease or disorder associated with the use of an antitumor agent in a subject.

22. The compound of any one of claims 1 to 13, or a pharmaceutically acceptable salt, bioactive metabolite, solvate, hydrate, prodrug, racemate, enantiomer or stereoisomer thereof; or the composition of any one of claims 15 to 19 for preparing a JAK inhibitor.

23. A method for preventing, alleviating and/or treating a disease or disorder associated with the use of an antitumor agent in a subject thereof, comprising administering the compound of any one of claims 1 to 13, or a pharmaceutically acceptable salt, bioactive metabolite, solvate, hydrate, prodrug, racemate, enantiomer or stereoisomer thereof; or the composition of any one of claims 15 to 19, to the subject in need.

24. A method for preventing, treating and/or alleviating a JAK-mediated disease or disorder in a subject in need thereof, comprising administering an effective amount of the compound of any one of claims 1 to 13, or a pharmaceutically acceptable salt, bioactive metabolite, solvate, hydrate, prodrug, racemate, enantiomer or stereoisomer thereof; or the composition of any one of claims 15 to 19.

25. The method of claim 24, the method comprising administering an additional therapeutic agent.

26. The method of any one of claims 24 to 25, wherein the JAK-mediated disease or disorders is selected from one or more of the group: autoimmune disorders or autoimmune responses, extensive activation of immune response, bacterial infections, viral infections, inflammation, chronic and/or acute inflammatory disorders or conditions, and/or autoinflammatory disorders, fibrotic disorders, metabolic disorders, neoplasm, or cardiovascular or cerebrovascular disorders, skin disorders, pruritus, hair loss disorders, cancer or malignancy, autoimmune connective tissue diseases and autoimmune conditions; Still's disease, adult-onset Still's disease, Th17-associated inflammation, polychondritis (for example, relapsed polychondritis); myositis, polymyositis, autoimmune myositis, dermatomyositis, juvenile dermatomyositis; myasthenia gravis; arthritis (for example, rheumatoid arthritis, juvenile rheumatoid arthritis, systemic-onset juvenile rheumatoid arthritis, osteoarthritis, infectious arthritis, inflammatory arthritis, inflammatory bowel disease-associated arthritis, idiopathic arthritis, juvenile idiopathic arthritis, systemic juvenile idiopathic arthritis, psoriatic arthritis), spondylitis/spondyloarthritis/spondyloarthropathy (ankylosing spondylitis), gout, scleroderma (systemic scleroderma, juvenile scleroderma), Reiter's syndrome/reactive arthritis, Lyme disease, lupus/systemic lupus erythematosus (SLE) (lupus erythematosus, juvenile systemic lupus erythematosus, cutaneous lupus (subacute cutaneous lupus, chronic cutaneous lupus/discoid lupus, chilblain lupus erythematosus)), polymyalgia rheumatica, enthesitis, mixed connective tissue disease, enthesopathy, carditis, myocarditis, angiogenesis disorder, myelodysplastic syndrome, atherosclerosis, atherosclerotic restenosis (atherosclerotic coronary restenosis), acute coronary syndrome, myocardial infarction, cardiac allograft vasculopathy, transplanted arterial disease; vasculitis (large vessel vasculitis, small vessel vasculitis, giant cell arteritis, polyarteritis nodosa, and vasculitic syndromes, which comprise aortitis, Wegener's granulomatosis and behcet disease), Stimulator of Interferon Genes (STING)-associated vasculopathy with onset in infancy (SAVI); gastrointestinal disorders, enterocolitis, colitis, inflammatory bowel disease (ulcerative colitis and Crohn's disease), irritable bowel syndrome, enteritis syndrome/spastic colon, celiac sprue; acute and chronic pancreatitis; primary biliary cirrhosis, primary sclerotic cholangitis, jaundice, cirrhosis (for example, primary biliary cirrhosis, or cirrhosis attributable to fatty liver diseases (for example, alcoholic fatty liver disease and non-alcoholic fatty liver disease); esophagitis, gastritis, gastric and duodenal ulcers, peritonitis; nephropathy, immune-mediated glomerulonephrosis, autoimmune nephropathy, membranous glomerulopathy, chronic progressive nephropathy, diabetic kidney disease/diabetic nephropathy, renal fibrosis, renal ischemia/reperfusion injury, HIV-associated nephropathy, ureteral obstructive nephropathy, glomerulosclerosis, proteinuria, nephrotic syndrome, polycystic kidney disease, autosomal dominant polycystic kidney disease, immune-mediated nephropathy, autoimmune nephropathy, chronic progressive nephropathy, diabetic nephropathy, renal fibrosis, ischemic/reperfusion injury-associated nephropathy, HIV-associated nephropathy, ureteral obstructive nephropathy, glomerulonephritis, chronic kidney disease (for example, diabetic nephropathy), hypertension-induced nephropathy, glomerulosclerosis, proteinuria, nephrotic syndrome, polycystic kidney disease, autosomal dominant polycystic kidney disease, diabetic kidney disease, lupus nephritis; interstitial cystitis; periodontitis, gingivitis; pulmonary inflammation, sinusitis, pneumonia, bronchitis, asthma, bronchial asthma, atopic asthma, non-atopic asthma, allergic bronchopulmonary mycosis, aspirin-induced asthma, adult-onset asthma, fixed airflow obstruction asthma, exercise-induced asthma, cough variant asthma, work related asthma, nocturnal asthma, asthma complicated with obesity, eosinophilic asthma, hormone resistant asthma/severe asthma, exogenous asthma, endogenous asthma/cryptogenic asthma, Churg-Strauss syndrome, bronchiolitis, obliterative bronchiolitis, chronic obstructive pulmonary disease (COPD), interstitial pulmonary disease (pulmonary fibrosis, idiopathic pulmonary fibrosis), acute lung injury, pulmonary fibrosis (for example, idiopathic pulmonary fibrosis or cystic fibrosis), chronic obstructive pulmonary disease, adult respiratory distress syndrome, acute lung injury, drug-induced lung injury; Meniere's disease; eye disorders (including ocular inflammation, uveitis, dry eye/keratoconjunctivitis sicca, scleritis, episcleritis, keratitis/keratopathy, choroiditis, retinal vasculitis, optic neuritis, retinopathy (diabetic retinopathy, immune-mediated retinopathy, macular degeneration, wet macular degeneration and dry (senile) macular degeneration)); mastocytosis, iron-deficiency anemia, uremia, hypereosinophilic syndrome (HES), systemic mastocyte disease (SMCD), myelodysplastic syndrome, idiopathic thrombocytopenic purpura; bone resorption diseases; neurodegenerative disorders, neurological/neuromuscular disorders (for example, multiple sclerosis, Parkinson's disease, Huntington's disease, amyotrophic lateral sclerosis (ALS) (including familial ALS and sporadic ALS), Alzheimer's disease, myasthenia gravis, Lambert-Eaton myasthenic syndrome (LEMS), Guillain-Barre syndrome, meningitis, encephalitis, traumatic brain injury; nervous system damage, delusional parasitosis, dysregulation of neuronal processes and sensory perception, stroke/neuronal ischemia, spinal cord injury, peripheral neuropathy, pseudaphia, spinal cord injury, mental disease; paresthesia pain (acute pain, chronic pain, neuropathic pain, or fibromyalgia), nerve stimulation, peripheral neuropathy; pruritus/scrapie (atopic pruritus, dry pruritus, psoriasis-associated pruritus/psoriatic scrapie/psoriasis-associated scrapie), acute pruritus, chronic pruritus, idiopathic pruritus, chronic idiopathic pruritus, biliary scrapie, hepatobiliary-associated scrapie, renal-associated scrapie/renal scrapie, uremic scrapie, cholestasis, intrahepatic cholestasis of pregnancy, lichen simplex chronicus-associated pruritus, lymphoma-associated scrapie, leukemia-associated scrapie, prurigo nodularis, atopic dermatitis-associated scrapie, atopic scrapie/atopic pruritus, bullous scrapie, brachioradialis pruritus, neurogenic scrapie, neurotic scrapie, notalgia paresthetica, HIV-associated pruritic papules, psychogenic scrapie, swimmer scrapie, itch or uremic scrapie, urticaria scrapie; skin diseases (for example, dermatological drug reaction/drug eruption, dermatoxerasia/dry skin, rash, skin sensitization, skin irritation, sunburn, infection caused by shaving, body lice, head lice/phthiriasis, crab louse, cutaneous larva migrans, scabies, parasitic infections, insect infections, urticaria, papular urticaria, insect bites, insect stings, dandruff, foreign bodies or devices on the skin, fungal infections, herpes, varicella, eosinophilic folliculitis, dermatosis of pregnancy/pruritic urticaria papula and plaque (PUPP), inflammatory dermatosis, neutrophilic dermatosis, histiocytoid neutrophilic dermatosis, intestinal bypass syndrome dermatosis, psoriasis/psoriasis vulgaris, lichen planus, lichen sclerosus et atrophicus, acne (acne vulgaris, comedonal acne, inflammatory acne, nodular cystic acne, keloidalis acne, acne keloidalis nuchae), atopy (allergic contact sensitization, allergic dermatitis), dermatitis (atopic dermatitis/eczema, contact dermatitis, photodermatitis, seborrheic dermatitis, stasis dermatitis), acute febrile neutrophil dermatosis (Sweet syndrome), chronic atypical neutrophilic dermatosis with lipodystrophy and elevated temperature (CANDLE syndrome), hidradenitis suppurativa, urticaria, pyoderma gangraenosum, alopecia (eyebrow loss, hair loss intranasal, alopecia cicatrisata (for example, alopecia scarring, alopecia scarring centralis, lichen planopilaris, frontal fibrosing alopecia, folliculitis decalvans), non-cicatricial alopecia (alopecia areata (AA) (patchy AA, alopecia totalis (AT), alopecia universalis (AU), alopecia serpentina, alopecia horseshoe)), androgenic/androgenic alopecia (AGA)/AGA in males and females ), telogen effluvium, tinea capitis, hypotrichosis (simple hereditary hypotrichosis), lichen planopilaris (frontal fibrosing alopecia), punctate palmoplantar keratodermasis, erythema elevatum diutinum (EED), neutrophilic eccrine hidradenitis, palisaded neutrophilic granulomatous dermatitis, neutrophilic urticaria dermatosis, vitiligo (for example, segmental vitiligo (including single segmental vitiligo, double segmental vitiligo and multi segmental vitiligo), non-segmental vitiligo (for example, acrotype vitiligo, facial vitiligo, acrofacial vitiligo, central facial vitiligo, mucosal vitiligo, confetti macules, trichrome vitiligo, borderline inflammatory vitiligo, quadrichrome vitiligo, blue vitiligo, Koebner phenomenon, vitiligo vulgaris, generalized vitiligo, common vitiligo), mixed vitiligo/ non-segmental vitiligo accompanied by segmental vitiligo, localized vitiligo, solitary mucous vitiligo and vitiligo with or without gray hair (body hair involved); bullous diseases, immune bullous disease (for example, bullous pemphigus, scarring pemphigus, pemphigus vulgaris, linear IgA disease), pemphigoid gestationis, xeroderma pigmentosum; fibrosis and scarring disorders; fibroma, liver fibrosis, pulmonary fibrosis, idiopathic pulmonary fibrosis (for example, scleroderma, increased fibrosis, keloid, low-grade scarring of postoperative scars); wound healing, surgical scarring and radiation-induced fibrosis (for example, head and neck, gastrointestinal tract, or lungs), central nervous system scarring, digestive or gastrointestinal tract fibrosis, renal fibrosis, liver or bile duct fibrosis, liver fibrosis (for example, non-alcoholic steatohepatitis, hepatitis C, or liver cell carcinoma), cardiac fibrosis (for example, endomyocardial fibrosis or atrial fibrosis), ocular scarring, fibrosclerosis, scar growth, wound or scab healing, keloid, mediastinal fibrosis, myelofibrosis, retroperitoneal fibrosis/Ormond disease, progressive mass fibrosis, renal systemic fibrosis; Sjogren syndrome, sarcoidosis, familial Mediterranean fever, cryopyrin-associated periodic syndrome (Muckle-Wells syndrome, familial cold autoinflammatory syndrome/familial cold urticaria /TNF receptor-associated periodic syndrome, neonatal-onset multisystem inflammatory disease), oxygen-induced inflammation, reperfusion injury, postoperative trauma, tissue injury, hyperthermia syndrome; diabetes (Type I and II diabetes)/diabetes, Hashimoto thyroiditis, Graves' disease, Addison's disease, Castleman's disease, hyperparathyroidism, menopause, obesity, steroid resistance, glucose intolerance, metabolic syndrome, thyroid disease, pituitaritis; systemic immunosenescence; autoimmune atrophic gastritis, autoimmune atrophic gastritis with pernicious anemia, autoimmune encephalomyelitis, autoimmune orchitis, Goodpasture syndrome, Sjogren syndrome, autoimmune thrombocytopenia, sympathetic ophthalmia; secondary hematological manifestations of autoimmune diseases (for example, anemia), autoimmune hemolytic syndrome (autoimmune hemolytic anemia), autoimmune hepatitis and inflammatory hepatitis, autoimmune ovarian failure, autoimmune thrombocytopenia, silicone implantation-associated autoimmune diseases, drug-induced autoimmune, HIV-associated autoimmune syndrome, metal-induced autoimmune, autoimmune deafness, and autoimmune thyroid disorders; anaphylaxis and allergy (including hypersensitivity, for example, Type I hypersensitivity (for example, allergies), Type II hypersensitivity (for example, Goodpasture syndrome and autoimmune hemolytic anemia), Type III hypersensitivity (for example, Arthus reaction, serum disease) and Type IV hypersensitivity (for example, contact dermatitis and allograft rejection); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); acute and chronic infections, sepsis syndromes (sepsis, septic shock, endotoxic shock, exotoxin-induced toxic shock, Gram-negative sepsis, Gram-positive sepsis, fungal sepsis, toxic shock syndrome); rejection (for example, graft-versus-host reaction/graft-versus-host disease, allograft rejection (for example, acute/chronic allograft rejection), early allograft rejection; malignancies, cancers, lymphoma, leukemia, multiple myeloma, solid tumors, teratoma, metastatic disorders and bone disorders, internal cancer, bone cancer, oral/pharyngeal cancer, esophageal cancer, larynx cancer, gastric cancer, intestinal cancer, colon cancer, rectum cancer, lung cancer (for example, non-small cell lung cancer or small cell lung cancer), hepatic cancer (liver cancer), pancreatic cancer, nerve cancer, brain cancer (for example, glioma, spongioblastoma multiforme, astrocytoma, neuroblastoma, and neurinoma), head and neck cancer, throat cancer, ovarian cancer, uterine cancer, prostate cancer, testicular cancer, bladder cancer, renal cancer (kidney cancer), breast cancer, gallbladder cancer, cervical cancer, thyroid cancer, prostate cancer, eye cancer (eye malignancies), and skin cancer (melanoma and keratoacanthoma); and fibrotic cancers, fibromas, fibroadenomas, fibrosarcomas, myeloproliferative disorders, neoplasms (hematopoietic tissue neoplasms, spinal cord neoplasms, lymphoid tissue neoplasms (myelofibrosis, primary myelofibrosis, polycythemia vera, essential thrombocytosis)), leukemias (acute lymphoblastic leukemia, acute myelocytic leukemia, and chronic myelocytic leukaemia, chronic lymphoblastic leukemia, acute lymphoblastic leukemia, chronic myelomonocytic leukemia (CMML), or promyelocytic leukemia), multiple myeloma and other myeloid malignancies (myelofibrosis with myeloid metaplasia (MMM), primary myelofibrosis (PMF), idiopathic myelofibrosis (IMF)), lymphoma (Hodgkin's disease, cutaneous lymphoma (cutaneous T-cell lymphoma, mycosis fungoides), lymphoma (for example, B-cell lymphoma, T-cell lymphoma, mantle cell lymphoma, hairy cell lymphoma, Burkitt's lymphoma, mastocytoma, Hodgkin's disease, or non-Hodgkin's disease); Kaposi's sarcoma, rhabdomyosarcoma, seminoma, teratoma, osteosarcoma, follicular thyroid carcinoma; increased accumulation of exogenous or synthetic opioids, notalgia paresthetica, obsessive-compulsive disorder, obsessive-compulsive disorder-associated nostopathy and combinations thereof.
